# EUROPEAN PATENT APPLICATION

(11) **EP 4 725 959 A1**
(43) Date of publication of application: **15.04.2026**
(21) Application number: 24818807.0
(22) Date of filing: 07.06.2024
(51) Int. Cl.: C07K 14/005, C07K 14/08, C07K 14/115, C07K 14/135, A61K 39/155, C12N 15/45, A61P 31/14

(54) **RSV VACCINE COMPOSITION, METHOD AND USE THEREOF**

(30) Priority: 07.06.2023 CN 202310705477
(71) Applicant: SICHUAN CLOVER BIOPHARMACEUTICALS, INC., Chengdu, Sichuan 610041 (CN)
(72) Inventor: LIANG, Joshua, Chengdu, Sichuan 610041 (CN); SU, Danmei, Chengdu, Sichuan 610041 (CN); LIANG, Peng, Chengdu, Sichuan 610041 (CN)
(74) Representative: Kuttenkeuler, David
(86) International application number: PCT/CN2024/098246
(87) International publication number: WO 2024/251281

(57) **Abstract**

An immunogenic composition comprising a recombinant peptide and a protein, wherein the recombinant peptide and the protein comprise a respiratory syncytial virus (RSV) viral antigen and an immunogen such as an RSV F protein peptide. The immunogenic composition comprises a secreted fusion protein, the secreted fusion protein comprises a soluble RSV virus antigen, and the soluble RSV virus antigen is linked with the C-terminal part of collagen by means of in-frame fusion to form a disulfide bond-linked trimer fusion protein. The immunogenic composition can be used for producing an immune response, for example for treating or preventing RSV infection. The immunogenic composition can be used in a vaccine composition, for example as part of a prophylactic and/or therapeutic vaccine. Further provided are a method for producing the recombinant peptide and protein, prevention, treatment and/or diagnosis methods and related kits.

## Description

### TECHNICAL FIELD

The present disclosure relates in some aspects to an immunogenic composition comprising a recombinant peptide and a protein, and the recombinant peptide and protein comprising a respiratory syncytial virus (RSV) antigen and an immunogen, such as an RSV F protein peptide, for use in the treatment and/or prevention of RSV infection.

### BACKGROUND OF THE INVENTION

Respiratory syncytial virus (RSV) results in respiratory infections in adults and children and is the main reason for lower respiratory infection and hospitalization in infants and young children. Although the RSV infection rate is high, therapeutic means including prophylactic agents, therapeutic agents and vaccines are limited or unavailable. There is a need for improved methods to treat RSV. Provided herein are compositions, methods, uses, and articles of manufacture to meet these and other needs.

### SUMMARY OF THE INVENTION

In one aspect, provided herein is a protein comprising a plurality of recombinant polypeptides, each recombinant polypeptide comprising a respiratory syncytial virus (RSV) F protein peptide or a fragment or epitope thereof linked to a C-terminal propeptide of collagen, wherein the C-terminal propeptide of the recombinant polypeptide forms an inter-polypeptide disulfide bond. In some embodiments, the RSV belongs to subtype A or subtype B. In some embodiments, the epitope is a linear epitope or a conformational epitope.

In some embodiments, disclosed herein is a recombinant subunit vaccine comprising an extracellular domain of RSV F protein, or a fragment thereof (e.g. lacking a transmembrane domain and a cytoplasmic domain), within which the extracellular domain is fused in-frame with a collagen C propeptide capable of forming a homotrimer via disulfide linkages. The resulting recombinant subunit vaccine, such as an F trimer, can be expressed and purified from transfected cells and is expected to be in a trimeric native-like conformation. This solves the problem of misfolding often encountered when a viral antigen is expressed as a recombinant peptide or protein in a soluble form lacking a transmembrane and/or cytoplasmic domain. Such misfolded viral antigens do not accurately retain native viral antigen conformation and are often unable to elicit neutralizing antibodies.

In some embodiments, the F protein peptide comprises an F1 subunit peptide, an F2 subunit peptide, or any combination thereof, and the protein comprises three recombinant polypeptides. In some embodiments, the F protein peptide comprises a signal peptide, a heptad repeat sequence C (HRC) peptide, a pep27 peptide, a fusion peptide (FP), a heptad repeat sequence A (HRA) peptide, a domain I peptide, a domain II peptide, or a heptad repeat sequence B (HRB) peptide, or any combination thereof. In some embodiments, the F protein peptide comprises F1 subunit of F protein but does not include F2 subunit, or vice versa. In some embodiments, the F protein peptide comprises an F1 subunit of F protein and an F2 subunit, optionally pep27 is absent, and optionally, wherein the F1 subunit and the F2 subunit are linked by a disulfide bond or an artificially introduced linker. In some embodiments, the F protein peptide does not comprise a transmembrane (TM) domain peptide and/or a cytoplasmic (CP) domain peptide. In some embodiments, the F protein peptide comprises a protease cleavage site, wherein the protease is optionally furin, trypsin, factor Xa, thrombin, or cathepsin L. In some embodiments, the F protein peptide does not comprise a protease cleavage site, wherein the protease is optionally furin, trypsin, factor Xa, thrombin, or cathepsin L.

In some embodiments, the F protein peptide is soluble or is not directly associated with a lipid bilayer, such as a membrane or viral envelope. In some embodiments, the F protein peptide is the same or different among the recombinant polypeptides of the protein. In some embodiments, the F protein peptide is directly fused to a C-terminal propeptide, or to a C-terminal propeptide via a linker such as a linker comprising a Gly-X-Y repeat sequence, wherein X and Y are independently any amino acid, optionally proline or hydroxyproline.

In some embodiments, the protein is soluble or is not directly associated with a lipid bilayer, such as a membrane or viral envelope. In some embodiments, the protein can form a rosette-like oligomer comprising an F protein peptide trimer. In some embodiments, the protein can bind to a cell-surface attachment factor or receptor of a subject, optionally, the subject is a mammal, such as a primate, such as a human.

In some embodiments, the C-terminal propeptide is derived from human collagen. In some embodiments, the C-terminal propeptide comprises a C-terminal polypeptide or a fragment thereof from proα1 (I), proα1 (II), proα1 (III), proα1 (V), proα1 (XI), proα2 (I), proα2 (V), proα2 (XI), or proα3 (XI). In some embodiments, the C-terminal propeptide is the same or different among the recombinant polypeptides. In some embodiments, the C-terminal propeptide comprises any one of SEQ ID NOs: 48-63, or an amino acid sequence having at least 90% identity to any one of SEQ ID NOs: 48-63, capable of forming an inter-polypeptide disulfide bond and trimerizing the recombinant polypeptides.

In some embodiments, the F protein peptide in each recombinant polypeptide is a prefusion conformation or a post-fusion conformation, and optionally, the protein comprises a rosette-like oligomer, and the rosette-like oligomer comprises a crutch-shaped rod-like F protein peptide trimer. In any of the above embodiments, the F protein peptide in each recombinant polypeptide may comprise or have at least 80% identity to any one of SEQ ID NOs: 17-47.

In any of the above embodiments, the recombinant polypeptide may comprise any one of SEQ ID NOs: 1-16 and 64-71, or an amino acid sequence having at least 80% identity to any one of SEQ ID NOs: 1-16 and 64-71. In any of the above embodiments, the recombinant polypeptide may comprise any one of SEQ ID NOs: 17-47 and 72-79, or an amino acid sequence having at least 80% identity to any one of SEQ ID NOs: 17-47 and 72-79, and may be directly or indirectly fused or operably linked to any one of SEQ ID NOs: 48-63 or an amino acid sequence having at least 90% sequence identity to any one of SEQ ID NOs: 48-63.

Also provided herein is an immunogen comprising the protein provided herein. Provided herein is a protein nanoparticle comprising the protein provided herein, the protein being directly or indirectly attached to the nanoparticle. Provided herein is a virus-like particle (VLP) comprising the protein provided herein.

Also provided herein is an isolated nucleic acid encoding one, two, three, or more recombinant polypeptides of the protein provided herein. In some embodiments, the nucleic acid encoding the F protein peptide is fused in-frame with a nucleic acid encoding the C-terminal propeptide of collagen. In some embodiments, the isolated nucleic acid provided herein is operably linked to a promoter.

In some embodiments, the isolated nucleic acid provided herein is a DNA molecule. In some embodiments, the isolated nucleic acid provided herein is an RNA molecule, optionally an mRNA molecule, such as a nucleoside modified mRNA, a non-amplified mRNA, a self-amplifying mRNA, or a trans-amplifying mRNA.

Also provided herein is a vector comprising the isolated nucleic acid provided herein. In some embodiments, the vector is a viral vector.

In some aspects, provided herein is a virus, pseudovirus, or cell comprising a vector as provided herein, optionally, the virus or cell has a recombinant genome. In some aspects, provided herein is an immunogenic composition comprising a protein, an immunogen, a protein nanoparticle, a VLP, an isolated nucleic acid, a vector, a virus, a pseudovirus, or a cell provided herein, and a pharmaceutically acceptable carrier.

Also provided herein is a vaccine comprising the immunogenic composition provided herein and optionally an adjuvant, wherein the vaccine is optionally a subunit vaccine. In some embodiments, the vaccine is a prophylactic and/or therapeutic vaccine.

In some aspects, there is provided a method of producing a protein, the method comprising: expressing an isolated nucleic acid or a vector as provided herein in a host cell to produce a protein as provided herein; and purifying the protein. Provided herein is a protein produced by a method provided herein.

Provided herein is a method of producing an immune response to an F protein peptide of RSV or a fragment or epitope thereof in a subject, the method comprising administering to the subject an effective amount of a protein, immunogen, protein nanoparticle, VLP, isolated nucleic acid, vector, virus, pseudovirus, cell, immunogenic composition, or a vaccine as provided herein to produce the immune response. In some embodiments, the method provided herein is used to treat or prevent RSV infection. In some embodiments, an immune response is generated to inhibit or reduce the replication of RSV in a subject. In some embodiments, the immune response includes a cell-mediated response and/or a humoral response, optionally including generating one or more neutralizing antibodies, such as polyclonal antibodies or monoclonal antibodies. In some embodiments, the immune response is directed to an F protein peptide of RSV or a fragment or epitope thereof, but not to a C-terminal propeptide. In some embodiments, administration to a subject does not result in antibody-dependent enhancement (ADE) due to prior exposure of the subject to one or more RSV. In some embodiments, when the subject is subsequently exposed to one or more RSV, the administration does not result in antibody-dependent enhancement (ADE). In some embodiments, the method further comprises a prime and/or a boosting step. In some embodiments, the administration step is performed by topical, transdermal, subcutaneous, intradermal, oral, intranasal (e.g. intranasal spray), endotracheal, sublingual, buccal, rectal, vaginal, inhalation, intravenous (e.g. intravenous injection), intra-arterial, intramuscular (e.g. intramuscular injection), intracardiac, intraosseous, intraperitoneal, transmucosal, intravitreal, subretinal, intra-articular, periarticular, topical or epidermal administration. In some embodiments, the effective amount is administered as a single dose or at one or more intervals. In some embodiments, the effective amount is administered without an adjuvant. In some embodiments, the effective amount is administered with an adjuvant.

Provided herein is a method comprising administering to a subject an effective amount of a protein provided herein to produce a neutralizing antibody or neutralizing antiserum against RSV in the subject. In some embodiments, the subject is a mammal, optionally a human or non-human primate. In some embodiments, the method further comprises separating the neutralizing antibody or neutralizing antiserum from the subject. In some embodiments, the method further comprises administering to the human subject an effective amount of the isolated neutralizing antibody or neutralizing antiserum by passive immunization to prevent or treat RSV infection. In some embodiments, the neutralizing antibody or neutralizing antiserum for RSV comprises a polyclonal antibody against RSV F protein peptide or fragment or epitope thereof, optionally, the neutralizing antibody or neutralizing antiserum is free or substantially free of antibodies against the C-terminal propeptide of collagen. In some embodiments, the neutralizing antibody comprises a monoclonal antibody against RSV F protein peptide or fragment or epitope thereof, optionally, the neutralizing antibody is free or substantially free of antibodies against the C-terminal propeptide of collagen.

In some aspects, the proteins, immunogens, protein nanoparticles, VLPs, isolated nucleic acids, vectors, viruses, pseudoviruses, cells, immunogenic compositions or vaccines provided herein are used to induce an immune response to RSV in a subject and/or to treat or prevent RSV infection.

In some aspects, provided herein is the use of proteins, immunogens, protein nanoparticles, VLPs, isolated nucleic acids, vectors, viruses, pseudoviruses, cells, immunogenic compositions or vaccines provided herein for inducing an immune response to RSV in a subject and/or treating or preventing RSV infection. In some aspects, provided herein is the use of proteins, immunogens, protein nanoparticles, VLPs, isolated nucleic acids, vectors, viruses, pseudoviruses, cells, immunogenic compositions or vaccines provided herein for the preparation of a medicament or prophylactic agent for inducing an immune response against RSV in a subject and/or for treating or preventing RSV infection.

Also provided herein is a method of analyzing a sample, the method comprising: contacting a sample with a protein provided herein and detecting binding between the protein and an analyte capable of specifically binding to RSV F protein peptide, or a fragment or epitope thereof. In some embodiments, the analyte is an antibody, receptor, or cell that binds to an F protein peptide or fragment or epitope thereof. In some embodiments, the binding indicates the presence of the analyte in the sample and/or RSV infection in the subject from whom the sample is derived.

Provided herein is a kit comprising a protein provided herein and a substrate, a pad, or a vial containing or immobilizing the protein, optionally, the kit is an ELISA or lateral flow assay kit.

### BRIEF DESCRIPTION OF THE DRAWINGS

**FIG. 1** shows a schematic diagram of an exemplary fusion peptide comprising an extracellular F-domain that is fused with a trimerized peptide.
**FIG. 2** shows the OD value in the supernatant of 293T cells on day 3. Day 3, the total protein in the supernatant and the amount of prefusion F protein were determined by ELISA.
**FIG. 3** shows the OD value in the supernatant of CHO cells on day 3. On day 3, the total protein in the supernatant and the amount of prefusion F protein were determined by ELISA.
**FIG. 4** shows the OD value in the supernatant of CHO cells on day 7. On day 7, determining the total protein in the supernatant and the amount of F protein before fusion by ELISA.
**FIG. 5** shows the expression levels of peptides expressed using exemplary fusion peptides from serum-free fed batch cell cultures, as analyzed by 8% SDS-PAGE. SCB-N25C: the cell-free conditioned medium from day 1 to day 13 is separated under non-reducing and reducing conditions, then Coomassie blue staining is carried out, and the loading amount is 26 µl; SCB-N25: the cell-free conditioned medium from day 1 to day 13 is separated under non-reducing and reducing conditions, then Coomassie blue staining is performed, and the loading amount is 26 µl.
**FIG. 6** shows the purity assessment of an exemplary purification fusion peptide by SEC-HPLC, the main peak area of an exemplary SCB-N25 fusion protein is 92.8%, and the main peak area of the protein of SCB-N25C is 81.7%.

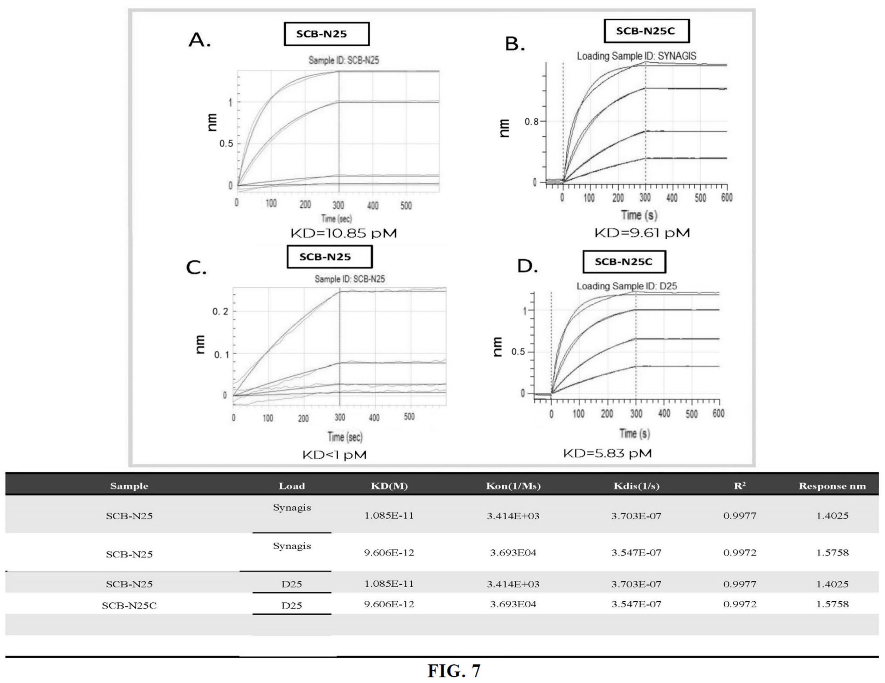

**FIG. 7** is an affinity dynamics test. Graphs A and B are studied by binding to palivizumab and a Bio-Layer Interferometry comprising an exemplary fusion peptide of RSV F protein peptides. First, 5 µg/mL Palivizumab is immobilized on a protein A sensor, and then the sensor is immersed in an exemplary fusion peptide at different concentrations to measure the binding kinetics. The resulting curve is fitted to a 1:1 binding model by subtracting the buffer reference value to obtain K associations and K dissociation, values as shown in the table below. C and D show a binding study by binding between antibody D25 and an exemplary fusion peptide comprising RSV F protein peptide. Corresponding KD, Kon, Kdis from antibody D25 binding experiments are shown in the following tables. SCB-N25C exhibits a high affinity for both Palivizumab and antibody D25.

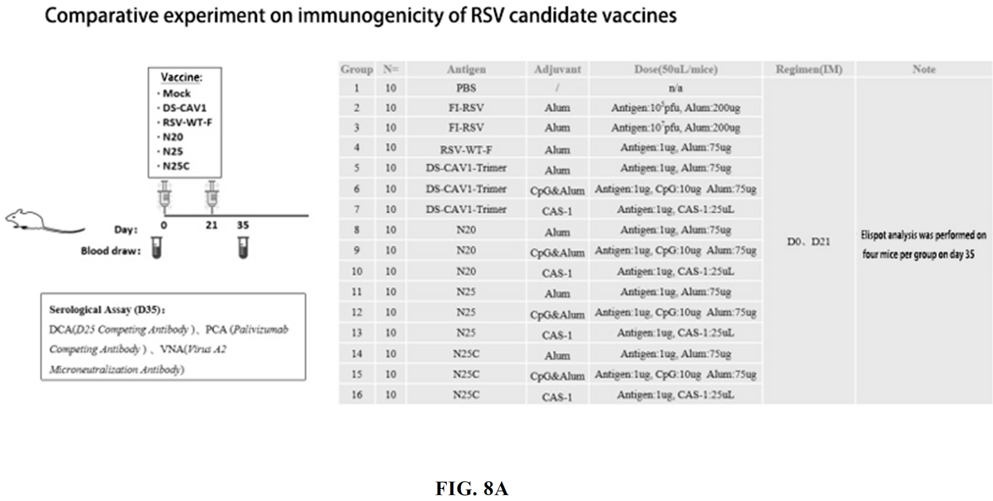

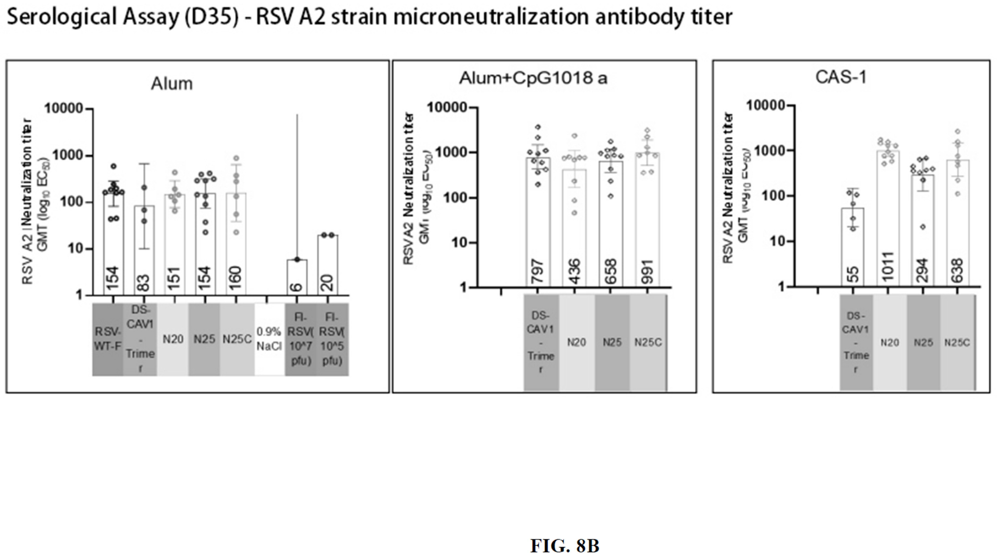

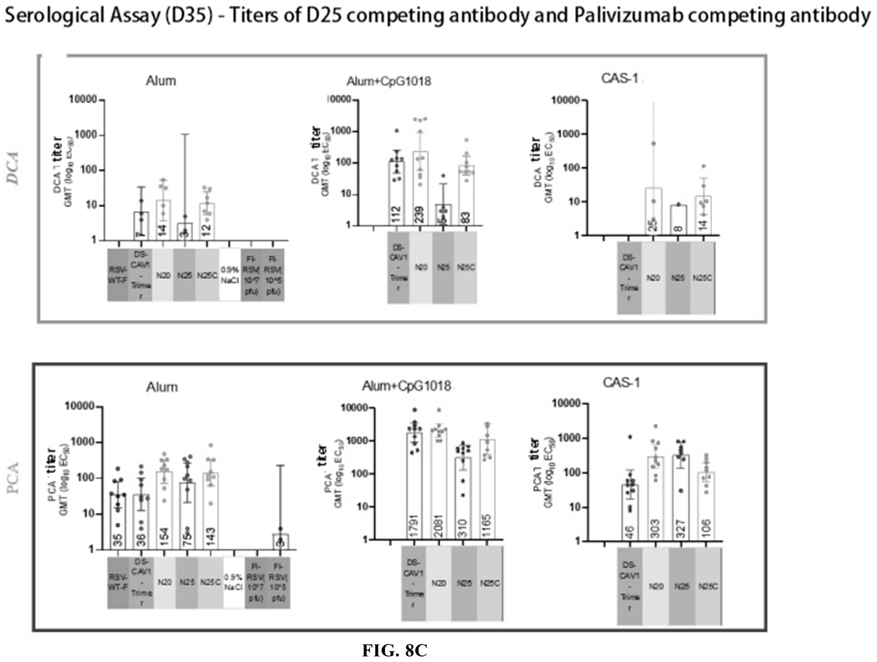

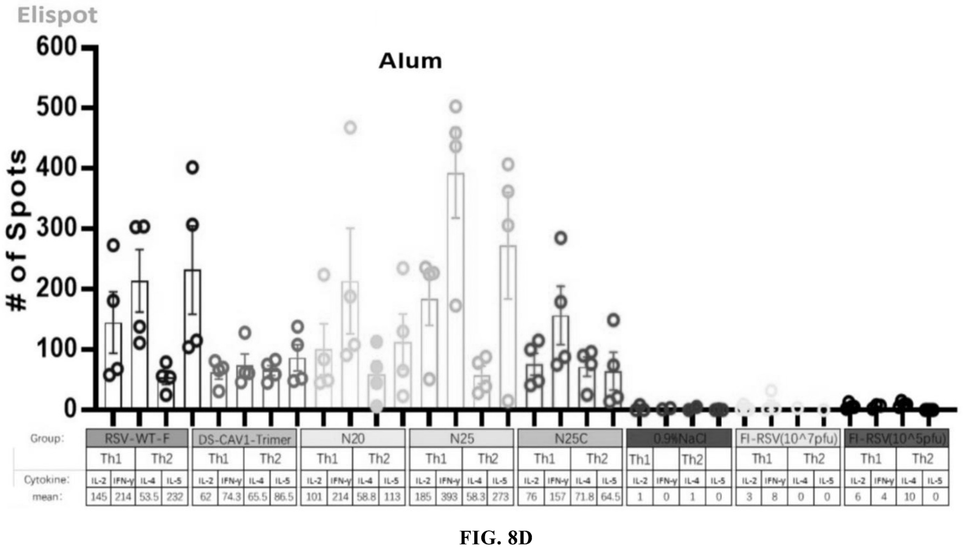

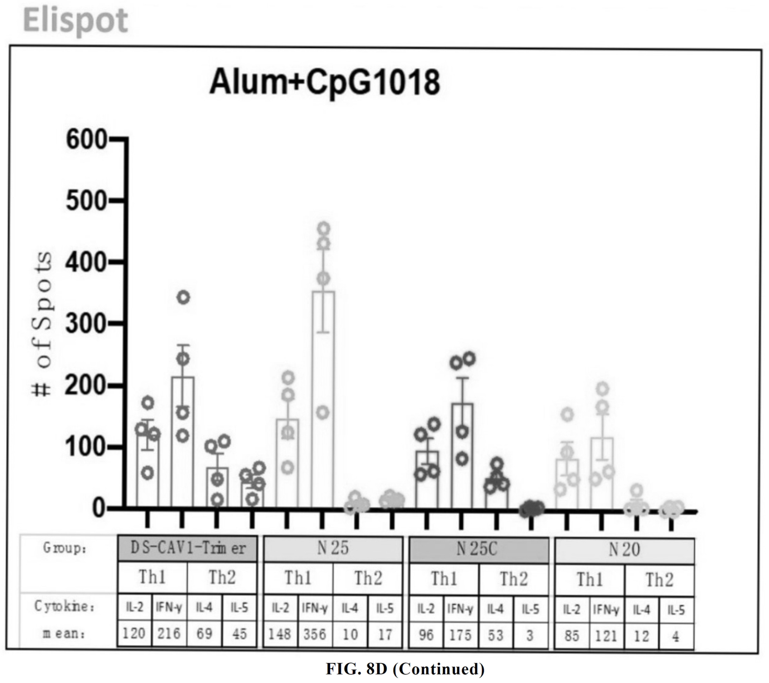

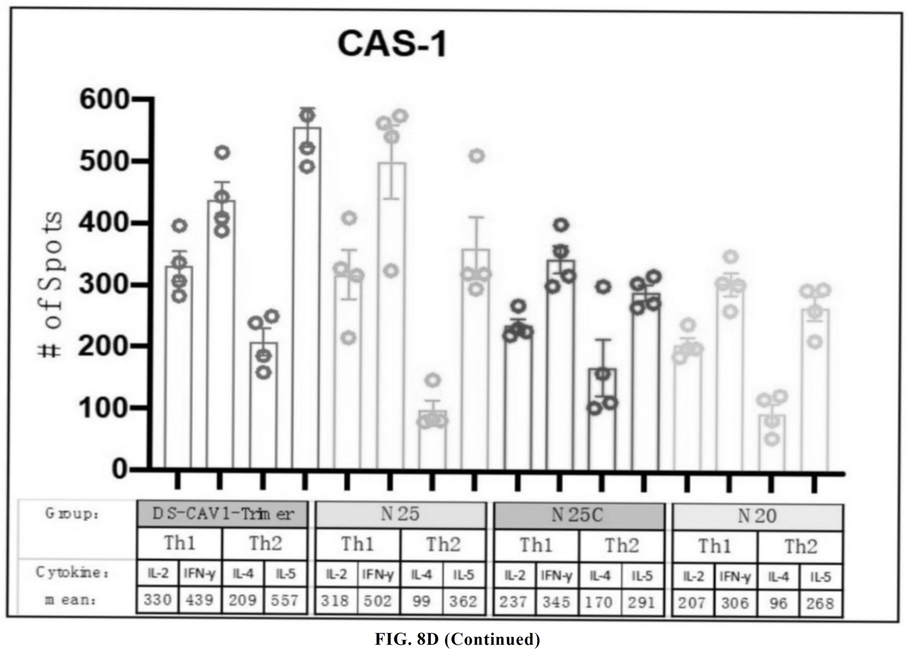

**FIGs. 8A-8D** illustrates test results for immunization with an exemplary fusion peptide comprising RSV F protein peptides. **FIG. 8A** shows a schematic diagram of an experimental method: vaccinating mice at day 0 and day 21, and collecting serum at day 0 and day 35, respectively. **FIG. 8B** shows a serologic experiment (D35)-RSV A2 strain-type micro-neutralizing antibody titer for a purified exemplary fusion peptide comprising a fusion protein of RSV F protein peptide and adjuvant Alum, Alum + CpG 1018 or CAS-1. The fusion protein using Alum + CpG 1018 as an adjuvant exhibits a higher titer than a corresponding fusion protein of Alum or CAS-1 as an adjuvant. SCB-N25C showed a higher titer value than other antigens. **FIG. 8C** illustrates the combination of D25 and Palivizumab with heat-inactivated RSV (HI-RSV) particles, as determined by serum sample dilution, to provide 50% inhibition of D25 and Palivizumab-competing IgG titers. Values are expressed as log₂ and mean ± SEM. All antigens bound to Alum showed a lower antibody than the other two adjuvants (DCA: D25 competitive antibody and PCA: Palivizumab anti-competitive antibody). When used in combination with an Alum + CpG 1018 adjuvant, both the DCA titer and the PCA titer of the DS-CAV1-Trimer and N20 are both high, and there is no significant difference between the two. The worst performance is N25. None of the four candidate antigens used in combination with the CAS-1 adjuvant has no effective DCA titer. The PCA titer of N20 and N25 is high, and DS-CAV1-Trimer is the lowest. **FIG. 8D** shows the results of the Elispot test. The N25C antigen-combined Alum adjuvant can produce a stronger Thl-like cell immune response. The antigen-combined Alum + CpG 1018 adjuvant generates a low Th2-type cellular immune response. The antigen-combined CAS-1 adjuvant generates a higher Th1 class and Th2 cell immune response. CAS-1 comprises squalene, α-tocopherol, and Tween-80.

### DETAILED DESCRIPTION OF THE PREFERRED EMBODIMENTS

In some embodiments, compositions comprising recombinant soluble surface antigens from RNA viruses in a covalently linked trimeric form and methods of using the same are disclosed. In some embodiments, the resulting fusion protein is secreted as a disulfide bond-linked homotrimer, which is structurally more stable, while retaining the conformation of the native-like trimeric viral antigen, and thus can be used as a more effective vaccine against these hazardous pathogens.

In some embodiments, disclosed herein are methods of preventing viral infections using a viral antigen trimer as a vaccine or as part of a multivalent vaccine, using an adjuvant, without an adjuvant, or using more than one adjuvant, optionally administered via intramuscular injection or intranasal administration.

In some embodiments, disclosed herein are methods of diagnosing viral infections by detecting antibodies that recognize the viral antigen, such as IgM, IgG, or neutralizing antibodies, using a viral antigen trimer as an antigen.

In some embodiments, disclosed herein are methods for using viral antigen trimers as immunogens to induce polyclonal or monoclonal antibodies that can be used for passive immunization, such as neutralizing mAbs for the treatment of RSV infections in infants.

In some embodiments, disclosed herein is a viral antigen trimer as a vaccine or as part of a multivalent vaccine, wherein the vaccine comprises a plurality of trimeric subunit comprising viral antigens derived from the same viral protein or viral antigens derived from two or more different proteins, derived from one or more viral species or from one or more strains of the same viral species.

In some embodiments, disclosed herein is a monovalent vaccine comprising the viral antigen trimer disclosed herein. In some embodiments, disclosed herein is a bivalent vaccine comprising the viral antigen trimer disclosed herein. In some embodiments, disclosed herein is a trivalent vaccine comprising the viral antigen trimer disclosed herein. In some embodiments, disclosed herein is a tetravalent vaccine comprising the viral antigen trimer disclosed herein.

In some embodiments, disclosed herein is a monovalent vaccine comprising the F trimer disclosed herein. In some embodiments, disclosed herein is a bivalent vaccine comprising the F trimer disclosed herein. In some embodiments, disclosed herein is a bivalent vaccine comprising at least one F trimer comprising a first F protein antigen and at least one F trimer comprising a second F protein antigen. In some embodiments, the first and second F protein antigens are derived from the same F protein of viruses belongs to one or more species or strains/subtypes, or from two or more different F proteins derived from different viral species or from different strains/subtypes of the same species. In some embodiments, disclosed herein is a trivalent vaccine comprising the F trimer disclosed herein. In some embodiments, disclosed herein is a trivalent vaccine comprising at least one F trimer comprising a first F protein antigen, at least one F trimer comprising a second F protein antigen, and at least one F trimer comprising a third F protein antigen. In some embodiments, the first, second and third F protein antigens are derived from the same F protein of viruses belongs to one or more viral species or strains/subtypes, or from two, three or more different F proteins derived from different viral species or from different strains/subtypes of the same viral species. In some embodiments, disclosed herein is a tetravalent vaccine comprising the F trimer disclosed herein. In some embodiments, disclosed herein is a tetravalent vaccine comprising at least one F trimer comprising a first F protein antigen, at least one F trimer comprising a second F protein antigen, at least one F trimer comprising a third F protein antigen, and at least one F trimer comprising a fourth F protein antigen. In some embodiments, the first, second, third and fourth F protein antigens are derived from the same F protein of viruses belongs to one or more viral species or strains/subtypes, or from two, three, four or more different F proteins derived from different viral species or from different strains/subtypes of the same viral species.

Provided herein are immunogenic compositions, methods, and uses of fusion peptides and proteins that comprise RSV antigens or immunogens for use in, for example, prophylactic or therapeutic treatment of RSV infections. Respiratory syncytial virus (RSV) is regarded as a major cause of infant and baby acute lower respiratory tract infections (ALRTI), causing approximately 7,000 to 20,000 child deaths worldwide each year. RSV infection is the second leading cause of infant mortality in developing countries. In addition, RSV can cause serious diseases in elderly and immunocompromised populations. Effective prophylactic humanized mAb Palivizumab (SYNAGIS^{®}) can only be used as a passive immunization measure for infants at high risk of RSV infection.

Despite decades of research, RSV vaccine development has not been successful for a variety of reasons. For example, the production, stability, and effectiveness problems of RSV candidate vaccines have been difficult to overcome, in particularly, safety is a major concern as it is now well recognized that formalin-inactivated RSV (FI-RSV) vaccine can mediate vaccine-induced disease enhancement (VED).

Provided herein are proteins comprising RSV antigens and immunogens, including recombinant polypeptides and fusion proteins, useful for effective and safe treatment (e.g. therapeutic or prophylactic) of RSV infection. For example, proteins comprising RSV viral antigens and immunogens provided herein treat RSV infection without inducing VED and/or antibody-dependent enhancement (ADE). In addition, proteins comprising RSV antigens and immunogens provided herein are readily produced and exhibit stability under high stress conditions such as high temperature, extreme pH, and hyperosmotic or hypoosmotic conditions. Therefore, the proteins and immunogenic compositions provided herein circumvent and address the production, stability, safety, and efficacy issues that hinder the development of RSV vaccines.

In some aspects, the RSV antigen and immunogen provided herein comprise RSV glycoprotein (F), also referred to herein as RSV F protein peptide or peptide. The RSV F protein peptide is a homotrimeric type I transmembrane protein that mediates membrane and virus penetration into a host cell. The RSV F protein peptide is synthesized as an F0 proprotein precursor which is cleaved at two sites by the furin to generate the disulfide bond-linked mature F1 and F2 subunits. The RSV F protein peptide is highly conserved between RSV A and B strains. Neutralizing antibodies, such as Palivizumab, which can target the antigen site of F and provide protection against respiratory diseases caused by RSV infection.

In some embodiments, proteins comprising RSV viral antigens or immunogens, such as RSV F protein peptides, can produce an immune response, such as an immune response to RSV F peptide proteins. In some embodiments, the immune response inhibits or reduces replication of RSV in a subject, such as a patient. In some embodiments, the immune response includes generating one or more neutralizing antibodies, such as polyclonal and/or monoclonal antibodies. In some embodiments, neutralizing antibodies inhibit or reduce replication of RSV in a subject, such as a patient. In some embodiments, administering a protein (e.g. in the form of an immunogenic composition) to a subject does not result in antibody-dependent enhancement (ADE), even in subjects previously exposed to RSV. In some aspects, proteins comprising RSV viral antigens and immunogens, such as RSV F protein peptides, are used as vaccines.

In some embodiments, RSV viral antigen and immunogen, such as RSV F protein peptide, are linked to a protein or peptide to form a fusion protein or recombinant polypeptide. In some embodiments, the protein or peptide to which the RSV antigen or immunogen is linked can further associate covalently or non-covalently with another protein or peptide, including those present in a fusion protein or recombinant polypeptide. Thus, in some cases, the protein or peptide to which the RSV antigen or immunogen is attached is a domain capable of multimerization.

In some embodiments, RSV antigens and immunogens, such as RSV F protein peptides, are linked to collagen propeptides, such as C-terminal propeptide of collagen, to form fusion peptides or recombinant polypeptides. Accordingly, in some embodiments, the proteins provided herein comprise recombinant polypeptides containing RSV viral antigens and immunogens, such as RSV F protein peptides or fragments or epitopes thereof, which are linked to the C-terminal propeptide of collagen. In some embodiments, the collagen propeptide is derived from the C-terminal propeptide of human α1 collagen and is capable of self-trimerization.

In some embodiments, linking RSV antigens and immunogens, such as RSV F protein peptides, to collagen propeptides, such as C-terminal propeptide of collagen, aids the ability of proteins to generate an immune response. For example, the production of recombinant proteins can preserve the tertiary and quaternary structure of the RSV F protein peptide, which may be important for the stability of the native conformation of the RSV F protein peptide, thereby making the accessibility of antigen sites on the surface of proteins (e.g. neutralizing antibodies) capable of inducing an immune response. In addition, linking the RSV F protein peptide to a protein or peptide capable of self-trimerization allows the recombinant protein to aggregate, thereby simulating the native homotrimeric structure of the RSV F protein peptide on the viral envelope.

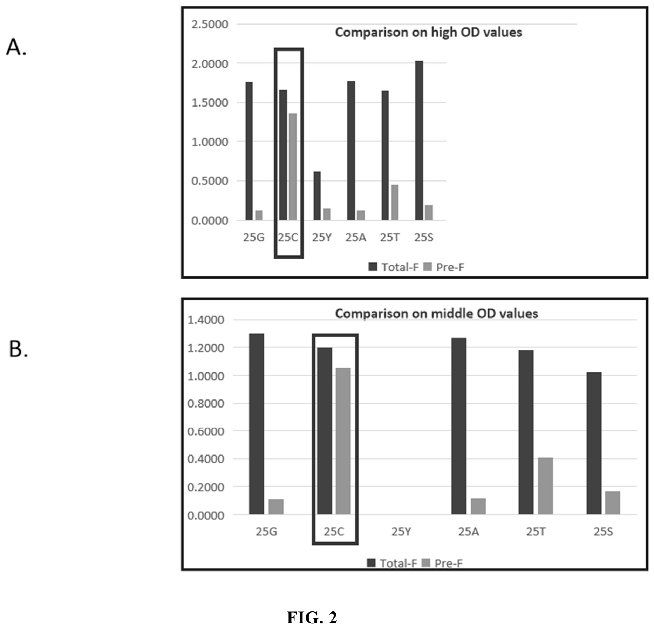

In some embodiments, linking the RSV F protein peptide to the C-terminal propeptide of collagen produces a self-trimerized recombinant polypeptide. In some embodiments, the protein provided herein comprises a plurality of self-trimerized RSV F protein peptides and a propeptide of a collagen recombinant polypeptide, optionally, the plurality of recombinant proteins form a rosette-like structure (see, e.g. **FIG. 2B** PCT/CN2021/099286, which is incorporated herein by reference in its entirety for all purposes). In some embodiments, the trimeric property of the recombinant protein helps to maintain the stability of the protein. In some embodiments, the macroscopic structure of the plurality of self-trimerized recombinant proteins (e.g., a rosette-like structure) helps to maintain the stability of the protein. In some embodiments, the trimeric property of the recombinant protein and the macroscopic structure of the plurality of self-trimerized recombinant proteins (e.g. a rosette-like structure) helps to maintain the stability of the protein. In some embodiments, the trimeric property of the recombinant protein facilitates the ability of the protein to produce an immune response. In some embodiments, the macroscopic structure of the plurality of self-trimerized recombinant proteins (e.g. a rosette-like structure) facilitates the ability of the protein to produce an immune response. In some embodiments, the trimeric property of the recombinant protein and the macroscopic structure of the plurality of self-trimerized recombinant proteins facilitate the ability of the protein to produce an immune response.

Also provided herein are immunogenic compositions that comprise the proteins provided herein, methods for producing the proteins provided herein, methods for treating a subject using the proteins and the compositions provided herein, and kits.

All publications mentioned in this application, including patent documents, scientific papers, and databases, are incorporated herein by reference in their entirety for all purposes, to the same extent as if each individual publication were individually incorporated by reference. To the extent that the definitions set forth herein are contrary or inconsistent with the definitions set forth in the patents, applications, published applications, and other publications, which are incorporated herein by reference, the definitions set forth herein shall prevail.

The section headings used herein are for organizational purposes only and are not to be considered limiting of the subject matter.

### I. Viral Antigen and Immunogen

Respiratory syncytial virus (RSV) is the most common cause of acute lower respiratory tract infections in infants and is also a major cause of morbidity in the elderly. Although RSV was identified more than half a century ago, there is currently only very limited vaccines against RSV, and the vaccine-induced disease enhancement observed in children vaccinated with formalin-inactivated RSV in the 1960s has hindered development efforts. Challenges in the production, purity, stability, and efficacy of the RSV candidate vaccine have also consistently posed obstacles to development.

In some embodiments, the proteins provided herein comprise RSV antigens and/or immunogens. In some embodiments, the RSV antigen and/or immunogen can promote or stimulate a cell-mediated response and/or a humoral response. In some embodiments, the response (e.g. a cell-mediated response or a humoral response) includes the generation of antibodies, such as neutralizing antibodies. In some embodiments, neutralizing antibodies (NAb) against viral antigens and/or immunogens provide adaptive immune defense against RSV exposure by blocking infection of susceptible cells. In some embodiments, the efficacy of a vaccine against several viruses is attributed to and/or associated with their ability to induce NAb. In some embodiments, the RSV antigen or immunogen is the RSV F protein peptide disclosed herein.

The RSV F protein peptide is an envelope glycoprotein of respiratory syncytial virus (RSV). The RSV F protein peptide is translated into a single precursor polypeptide (named F0). The RSV F protein mediates viral entry into cells and cell-cell fusion, is a target for neutralizing antibodies, and is highly conserved between RSV A and B strains. F0 can be cleaved by furin protease at Arg 109 and Arg 136, the shorter F2 polypeptide is covalently linked to a longer F1 polypeptide at the N-terminus by two disulfide bonds, the latter having a fusion domain of 18 amino acids at the N-terminus and a hydrophobic transmembrane region near the C-terminus; and releasing the fragments of the intermediate 27 amino acids. Neutralizing monoclonal antibodies Palivizumab and motavizumab bind to RSV F antigen site II (Asn 258-Val 278) and has proven to have a protective effect on the lower respiratory tract and upper respiratory RSV diseases high-risk and full-term infants. The structure of the RSV F epitope polypeptide that binds to these neutralizing antibodies is larger than that of a linear peptide, Palivizumab binds to RSV F with nanomolar affinity, whereas motavizumab binds with picomolar affinity. Modeling predicts that full engagement of palivizumab or motavizumab requires amino acids derived from one or two RSV F protomers. Thus, retaining the RSV F tertiary and quaternary structure can be very important in developing RSV F vaccines that retain native conformation of the important neutralizing region.

In some embodiments, the F0 precursor polypeptide has a length of 574 amino acids, as shown in SEQ ID NO: 31.

10 20 30 40 50 60
MELLILKANAITTILTAVTFCFASGQNITEEFYQSTCSAVSKGYLSALRTGWYTSVITIE
70 80 90 100 110 120
LSNIKENKCNGTDAKVKLIKQELDKYKNAVTELQLLMQSTPPTNNRARRELPRFMNYTLN
130 140 150 160 170 180
NAKKTNVTLSKKRKRRFLGFLLGVGSAIASGVAVSKVLHLEGEVNKIKSALLSTNKAVVS
190 200 210 220 230 240
LSNGVSVLTSKVLDLKNYIDKQLLPIVNKQSCSISNIETVIEFQQKNNRLLEITREFSVN
250 260 270 280 290 300
AGVTTPVSTYMLTNSELLSLINDMPITNDQKKLMSNNVQIVRQQSYSIMSIIKEEVLAYV
310 320 330 340 350 360
VQLPLYGVIDTPCWKLHTSPLCTTNTKEGSNICLTRTDRGWYCDNAGSVSFFPQAETCKV
370 380 390 400 410 420
QSNRVFCDTMNSLTLPSEINLCNVDIFNPKYDCKIMTSKTDVSSSVITSLGAIVSCYGKT
430 440 450 460 470 480
KCTASNKNRGIIKTFSNGCDYVSNKGMDTVSVGNTLYYVNKQEGKSLYVKGEPIINFYDP
490 500 510 520 530 540
LVFPSDEFDASISQVNEKINQSLAFIRKSDELLHNVNAGKSTTNIMITTIIIVIIVILLS
550 560 570
LIAVGLLLYCKARSTPVTLSKDQLSGINNIAFSN

In some embodiments, the F0 precursor polypeptide has a length of 574 amino acids, as shown in SEQ ID NO: 32.

        10          20          30          40          50          60
MELLILKANAITTILTAVTFCFASGQNITEEFYQSTCSAVSKGYLSALRTGWYTSVITIE
        70          80          90         100         110         120
LSNIKENKCNGTDAKVKLIKQELDKYKNAVTELQLLMQSTPATNNRARRELPRFMNYTLN
       130         140         150         160         170         180
NAKKTNVTLSKKRKRRFLGFLLGVGSAIASGVAVSKVLHLEGEVNKIKSALLSTNKAVVS
       190         200         210         220         230         240
LSNGVSVLTSKVLDLKNYIDKQLLPIVNKQSCSISNIATVIEFQQKNNRLLEITREFSVN
       250         260         270         280         290         300
AGVTTPVSTYMLTNSELLSLINDMPITNDQKKLMSNNVQIVRQQSYSIMSIIKEEVLAYV
       310         320         330         340         350         360
VQLPLYGVIDTPCWKLHTSPLCTTNTKEGSNICLTRTDRGWYCDNAGSVSFFPQAETCKV
       370         380         390         400         410         420
QSNRVFCDTMNSLTLPSEVNLCNVDIFNPKYDCKIMTSKTDVSSSVITSLGAIVSCYGKT
       430         440         450         460         470         480
KCTASNKNRGIIKTFSNGCDYVSNKGVDTVSVGNTLYYVNKQEGKSLYVKGEPIINFYDP
       490         500         510         520         530         540
LVFPSDEFDASISQVNEKINQSLAFIRKSDELLHNVNAGKSTTNIMITTIIIVIIVILLS
       550         560         570
LIAVGLLLYCKARSTPVTLSKDQLSGINNIAFSN

In some embodiments, the RSV F protein peptide herein comprises proline or alanine at residue 102. In some embodiments, the RSV F protein peptide herein comprises substitutions, deletions, and/or insertions at and/or near residue 102 of SEQ ID NO: 31 or 32. In some embodiments, the RSV F protein peptide herein comprises residue 102 of SEQ ID NO: 31 replaced with A. In some embodiments, the RSV F protein peptide herein comprises glutamic acid or alanine at residue 218. In some embodiments, the RSV F protein peptide herein comprises substitutions, deletions, and/or insertions at and/or near residue 218 of SEQ ID NO: 31 or 32. In some embodiments, the RSV F protein peptide herein comprises residue 218 of SEQ ID NO: 32 where alanine is replaced with glutamic acid. In some embodiments, the RSV F protein peptide herein comprises residue 523 of SEQ ID NO: 32 where I is replaced with T. In some embodiments, the RSV F protein peptide herein comprises valine or isoleucine at residue 379. In some embodiments, the RSV F protein peptide herein comprises substitutions, deletions, and/or insertions at and/or near the residue 379 of SEQ ID NO: 31 or 32. In some embodiments, the RSV F protein peptide herein comprises valine or methionine at residue 447. In some embodiments, the RSV F protein peptide herein comprises substitutions, deletions, and/or insertions at and/or near the residue 447 of SEQ ID NO: 31 or 32. In some embodiments, the RSV F protein peptide herein comprises substitutions, deletions, and/or insertions at and/or near any one or more of the following: proline or alanine at residue 102, glutamic acid or alanine at residue 218, valine or isoleucine at residue 379, and valine or methionine at residue 447. In some embodiments, the RSV F protein peptide herein comprises substitutions, deletions and/or insertions at and/or near any one or more of residues 102, 218, 379 and 447 of SEQ ID NO: 31 or 32. In some embodiments, the RSV F protein peptide herein comprises substitutions, deletions, and/or insertions at and/or near any one or more of the other residues of SEQ ID NO: 31 or 32.

In some embodiments, the RSV F protein peptide herein comprises substitutions, deletions, and/or insertions at and/or near residues 106, 107, 108 and/or 109 of SEQ ID NO: 31 or 32. In some embodiments, the RSV F protein peptide herein comprises glutamine or asparagine at residue 108 and/or 109. In some embodiments, the RSV F protein peptide herein comprises glutamine at residues 108 and 109. In some embodiments, the RSV F protein peptide herein comprises asparagine at residues 108 and 109. In some embodiments, the RSV F protein peptide herein comprises substitutions, deletions, and/or insertions at and/or near residues 131, 132, 133, 134, 135, and/or 136 of SEQ ID NO: 31 or 32. In some embodiments, the RSV F protein peptide herein comprises glycine, arginine, glutamine, or asparagine at and/or near residues 131, 132, 133, 134, 135 and/or 136 of SEQ ID NO: 31 or 32. In some embodiments, the RSV F protein peptide herein comprises glutamine at residues 131, 132, 133, 134, 135 and/or 136 of SEQ ID NO: 31 or 32. In some embodiments, the RSV F protein peptide herein comprises glutamine at residues 133, 135, and 136 of SEQ ID NO: 31 or 32.

In some embodiments, the RSV F protein peptide herein comprises substitution of alanine with glutamic acid at residue 218 of SEQ ID NO: 32, while replacement at one or more of residues 106, 107, 108, 109, 133, 135, and 136 is independently selected from cysteine, alanine, threonine, tyrosine, glycine, or serine, or any combination thereof.

In some embodiments, the RSV F protein peptide herein comprises substitution of alanine with glutamic acid at residue 218 of SEQ ID NO: 32, while one or more of residues 106, 107, 108, 109, 133, 135, and 136 are substituted. In some embodiments, the RSV F protein peptide herein comprises the substitution of alanine with glutamic acid at the residue 218 of SEQ ID NO: 32, while one or more of residues 106, 108, 109, 133, 135, and 136 are substituted. In some embodiments, the RSV F protein peptide herein comprises substitution of alanine with glutamic acid at residue 218 of SEQ ID NO: 32, while one or more of residues 106, 108, 109, 133, 135, and 136 are substituted with cysteine. In some embodiments, the RSV F protein peptide herein comprises substitution of alanine with glutamic acid at residue 218 of SEQ ID NO: 32, while all of residues 106, 108, 109, 133, 135, and 136 are substituted with cysteine. The RSV F protein peptide herein comprises substitution of alanine with glutamic acid at residue 218 of SEQ ID NO: 32, while all of residues 108, 109, 133, 135, and 136 are replaced with cysteine. The RSV F protein peptide herein comprises substitution of alanine with glutamic acid at residue 218 of SEQ ID NO: 32, while all of residues 109, 133, 135, and 136 are substituted with cysteine. The RSV F protein peptide herein comprises substitution of alanine with glutamic acid at residue 218 of SEQ ID NO: 32, while all of residues 133, 135, and 136 are substituted with cysteine. The RSV F protein peptide herein comprises substitution of alanine with glutamic acid at residue 218 of SEQ ID NO: 32, while all of residues 135 and 136 are substituted with cysteine. The RSV F protein peptide herein comprises substitution of alanine with glutamic acid at residue 218 of SEQ ID NO: 32, while residue 136 is replaced with cysteine. The RSV F protein peptide herein comprises substitution of alanine with glutamic acid at residue 218 of SEQ ID NO: 32, while residue 135 is replaced with cysteine. The RSV F protein peptide herein comprises substitution of alanine with glutamic acid at residue 218 of SEQ ID NO: 32, while all of residues 106, 133, 135, and 136 are substituted with cysteine. The RSV F protein peptide herein comprises substitution of alanine with glutamic acid at residue 218 of SEQ ID NO: 32, while all of residues 106, 135, and 136 are substituted with cysteine. The RSV F protein peptide herein comprises substitution of alanine with glutamic acid at residue 218 of SEQ ID NO: 32, while all of residues 106, 133, and 136 are substituted with cysteine. The RSV F protein peptide herein comprises substitution of alanine with glutamic acid at residue 218 of SEQ ID NO: 32, while all of residues 106, 133, and 135 are substituted with cysteine. The RSV F protein peptide herein comprises substitution of alanine with glutamic acid at residue 218 of SEQ ID NO: 32, while all of residues 106, and 136 are substituted with cysteine. The RSV F protein peptide herein comprises substitution of alanine with glutamic acid at residue 218 of SEQ ID NO: 32, while all of residues 106, and 135 are substituted with cysteine. The RSV F protein peptide herein comprises substitution of alanine with glutamic acid at residue 218 of SEQ ID NO: 32, while all of residues 108 and 136 are substituted with cysteine. The RSV F protein peptide herein comprises substitution of alanine with glutamic acid at residue 218 of SEQ ID NO: 32, while all of residues 108, and 135 are substituted with cysteine. The RSV F protein peptide herein comprises substitution of alanine with glutamic acid at residue 218 of SEQ ID NO: 32, while all of residues 106 and 133 are substituted with cysteine. The RSV F protein peptide herein comprises substitution of alanine with glutamic acid at residue 218 of SEQ ID NO: 32, while all of residues 106 and 133 are substituted with cysteine. The RSV F protein peptide herein comprises substitution of alanine with glutamic acid at residue 218 of SEQ ID NO: 32, while all of residues 108, and 133 are substituted with cysteine. The RSV F protein peptide herein comprises substitution of alanine with glutamic acid at residue 218 of SEQ ID NO: 32, while all of residues 108, and 133 are substituted with cysteine.

In some embodiments, the RSV F protein peptide herein comprises substitutions, deletions, and/or insertions at and/or near residues 109, 136, 161 and/or 215 of SEQ ID NO: 31 or 32. In some embodiments, the RSV F protein peptide herein comprises alanine or proline at any one or more of residues 109, 136, 161 and/or 215 of SEQ ID NO: 31 or 32. In some embodiments, the RSV F protein peptide herein comprises alanine at residue 109 of SEQ ID NO: 31 or 32. In some embodiments, the RSV F protein peptide herein comprises alanine at residue 136 of SEQ ID NO: 31 or 32. In some embodiments, the RSV F protein peptide herein comprises alanine at residues 109 and 136 of SEQ ID NO: 31 or 32. In some embodiments, the RSV F protein peptide herein comprises proline at residue 161 of SEQ ID NO: 31 or 32. In some embodiments, the RSV F protein peptide herein comprises proline at residue 215 of SEQ ID NO: 31 or 32. In some embodiments, the RSV F protein peptide herein comprises proline at residues 161 and 215 of SEQ ID NO: 31 or 32. In some embodiments, the RSV F protein peptide herein comprises alanine at residues 109 and 136 and proline at residues 161 and 215 of SEQ ID NO: 31 or 32.

In some embodiments, the RSV F protein peptide herein comprises substitutions, deletions, and/or insertions at and/or near any one or more of the residues 131-154 of SEQ ID NO: 31 or 32. In some embodiments, the RSV F protein peptide herein comprises 2, 3, 4, 5, 6, 7, 8, 9, 10, 11, 12, 13, 14, 15, 16, 17, 18 or more deletions within residues 131-154 of SEQ ID NO: 31 or 32. In some embodiments, the RSV F protein peptide herein comprises deletion of any one or more of residues 137-154 of SEQ ID NO: 31 or 32. In some embodiments, the RSV F protein peptide herein comprises deletion of any one or more of residues 137-146 of SEQ ID NO: 31 or 32. In some embodiments, the RSV F protein peptide herein comprises glutamine at residues 133, 135, and 136 and deletion of residues 137-146 of SEQ ID NO: 31 or 32.

In some embodiments, the RSV F protein peptide herein comprises amino acid residues 1-25 of the F0 precursor, which is the signal peptide MELLILKANAITTILTAVTFCFASG (SEQ ID NO: 33). In some embodiments, the precursor polypeptide F0 forms a precursor trimer. In some embodiments, the RSV F protein peptide herein is hydrolyzed by one or more cellular proteases, such as at a conserved furin consensus cleavage site to produce a Pep27 polypeptide (also referred to as p27), an F1 polypeptide, and an F2 polypeptide. In some embodiments, the Pep27 polypeptide (e.g. the amino acid 110-136 of the F0 precursor) is cleaved off, in some aspects not becoming part of the mature RSV F trimer. In some embodiments, F2 polypeptides (which may alternatively be referred to herein as "F2" or "F2 subunit peptides") comprise amino acid residues 26-109 of F0 precursors. In some embodiments, F1 polypeptides (alternatively referred to herein as "F1" or "F1 subunit peptides") comprise amino acid residues 137-574 of F0 precursors, and may comprise extracellular regions (e.g. residues 137-524), transmembrane domains (e.g. residues 525-550) and cytoplasmic domains (e.g. residues 551-574).

In some embodiments, the RSV F protein peptide herein comprises F1 and F2 polypeptides, both linked by disulfide bonds to form heterodimers, referred to as RSV F "protomers". In some embodiments, the RSV F protein peptide herein comprises three protomer forming an RSV F trimer, so it is the homotrimer of the three protomer. In some embodiments, the RSV F protein peptide herein is a mature RSV F trimer. In some embodiments, the RSV F protein peptide herein is a membrane-bound type. In some embodiments, the RSV F protein peptide herein is not a membrane-bound type. In some embodiments, the RSV F protein peptide herein is soluble and lacks transmembrane and cytoplasmic regions or fragments thereof. The soluble form can be converted, for example, by truncating the RSV F protein at amino acid 513 (i.e., removing residues 514 onward), 514, 515, 516, 517, 518, 519, 520, 521, 522, 523 or 524. In nature, mature RSV F trimer mediates virus and cell membrane fusion. The prefusion conformation of the mature RSV F trimer (which may be referred to herein as "pre-F" or prefusion) is highly unstable (metastable). However, once the RSV is docked with the cell membrane, the RSV F protein trimer undergoes a series of conformational changes and transitions to a highly stable fused ("post-F") conformation.

In some embodiments, the RSV antigen or immunogen comprises a signal peptide (SP) (e.g. amino acid residues 1-22 of SEQ ID NO: 31 or 32) or fragments and/or mutation sequences thereof, heptad repeat sequences C (HRC) (e.g. F2, which may be amino acid residues 23-109 of SEQ ID NO: 31 or 32) or fragments and/or mutation sequences thereof, furin cleavage sites (FCS) (e.g. at the junction between amino acids residues 109/110 of SEQ ID NO: 31 or 32) or fragments and/or mutation sequences thereof, 27 mer fragments (pep27) (e.g. amino acid residues 110-136 of SEQ ID NO: 31 or 32) or fragments and/or mutation sequences thereof, and a putative fusion peptide (FP) (for example, amino acid residues 137-155 of SEQ ID NO: 31 or 32) or fragments and/or mutation sequences thereof, and heptad repeat sequences A (HRA) (for example, amino acid residues 156-214 of SEQ ID NO: 31 or 32) or fragments and/or mutation sequences thereof, domain I and II (e.g. amino acid residues 215-476 of SEQ ID NO: 31 or 32) or fragments and/or mutation sequences thereof, heptad repeat sequences B (HRB) (e.g. amino acid residues 477-524 of SEQ ID NO: 31 or 32) or a fragment and/or mutation sequence thereof, a transmembrane (TM) domain (e.g. amino acid residues 525-550 of SEQ ID NO: 31 or 32) or a fragment and/or mutation sequence thereof, and/or a cytoplasmic (CP) domain (e.g. amino acid residues 551-574 of SEQ ID NO: 31 or 32) or any suitable combination thereof.

In some embodiments, the RSV antigen or immunogen is an RSV F protein peptide of RSV subtype A. In some embodiments, the RSV antigen or immunogen is an RSV subtype A2 RSV F protein peptide. In some embodiments, the RSV antigen or immunogen is an RSV F protein peptide of RSV subtype B. In some cases, the RSV F protein peptide is conserved among RSV subtypes.

In some cases, the RSV antigen or immunogen is a fragment of an RSV F protein peptide. In some embodiments, the RSV antigen or immunogen is an epitope of an RSV F protein peptide. In some embodiments, the epitope is a linear epitope. In some embodiments, the epitope is a conformational epitope. In some embodiments, the epitope is a neutralizing epitope site, such as site I, II, or IV. In some embodiments, all neutralizing epitopes of the RSV F protein peptide or fragment thereof are present as RSV antigens or immunogens.

In some cases, such as when the RSV antigen or immunogen is a fragment of an RSV F protein peptide, there is only a single subunit of the RSV F protein peptide.

In some embodiments, the RSV antigen or immunogen is or comprises an F1 subunit peptide. In some embodiments, the F1 subunit peptide is or comprises an amino acid sequence of 137-574 of a wild-type F protein. In some embodiments, the RSV antigen or immunogen is or comprises an F2 subunit peptide. In some embodiments, the RSV antigen or immunogen comprises a RSV F protein peptide comprising a signal peptide, a heptad repeat sequence C (HRC) peptide, a pep27 peptide, a fusion peptide (FP), a heptad repeat sequence A (HRA) peptide, a domain I peptide, a domain II peptide, or a heptad repeat sequence B (HRB) peptide, or any combination thereof. In some embodiments, the RSV antigen or immunogen comprises an RSV F protein peptide containing a signal peptide. In some embodiments, the RSV antigen or immunogen comprises an RSV F protein peptide containing a pep27 peptide. In some embodiments, the RSV antigen or immunogen comprises an RSV F protein peptide containing a fusion peptide (FP) (also referred to as a fusion domain (FD)). In some embodiments, the RSV antigen or immunogen comprises an RSV F protein peptide comprising a signal peptide, a pep27 peptide, and a fusion peptide (FP).

In some embodiments, the RSV viral antigen or immunogen comprises an RSV F protein peptide comprising the F1 subunit and F2 subunit of the F protein. In some embodiments, the RSV viral antigen or immunogen comprises an RSV F protein peptide comprising the F1 subunit peptide and F2 subunit peptide of the F protein but not containing the pep 27 peptide. In some embodiments, the RSV viral antigen or immunogen comprises an RSV F protein peptide comprising the F1 subunit peptide, F2 subunit peptide, and pep 27 peptide of the F protein. In some embodiments, the RSV viral antigen or immunogen comprises an RSV F protein peptide comprising the F1 subunit peptide, F2 subunit peptide, pep 27 peptide, and FP of the F protein.

In some cases, for example when the viral antigen or immunogen comprises both the F1 subunit peptide of the RSV F protein peptide and the F2 subunit peptide, the F1 and F2 subunits are linked. In some embodiments, F1 and F2 subunits are linked by disulfide bonds. In some embodiments, F1 and F2 subunits are linked by artificially introduced linker. In some embodiments, F1 and F2 subunits are linked by pep27 peptide. For example, in some embodiments, the direction from the N-terminal to the C-terminal is or includes F2-pep27-F1 in some embodiments, the direction from the N-terminal to the C-terminal is or includes F2-pep27-FP-F1 (F2-pep27-FP-F1). In some embodiments, the FP is considered a structural feature of the F1 subunit peptide.

In some cases, the RSV antigen or immunogen comprises an RSV F protein peptide that does not comprise a transmembrane (TM) domain peptide. In some cases, the RSV F protein does not comprise a cytoplasmic (CP) domain peptide. In some cases, the RSV F protein does not comprise a TM domain peptide or a CP domain peptide.

In some embodiments, the RSV antigen or immunogen comprises an RSV F protein peptide containing a protease cleavage site. In some embodiments, the protease cleavage site is specific to the protease furin. In some embodiments, the protease cleavage site is specific to the protease trypsin. In some embodiments, the protease cleavage site is specific to the protease factor Xa. In some embodiments, the protease cleavage site is specific to the protease cathepsin L.

In some cases, the RSV antigen or immunogen comprises an RSV F protein peptide that does not comprise a protease cleavage site. In some cases, the RSV antigen or immunogen comprises an RSV F protein peptide that does not contain a protease cleavage site specific to protease Furin. In some cases, the RSV antigen or immunogen comprises an RSV F protein peptide that does not contain a protease cleavage site specific to protease trypsin. In some cases, the RSV antigen or immunogen comprises an RSV F protein peptide that does not contain a protease cleavage site specific to protease factor Xa. In some cases, the RSV antigen or immunogen comprises an RSV F protein peptide that does not contain a protease cleavage site specific to protease cathepsin L.

In some embodiments, the RSV antigen or immunogen comprises a soluble RSV F protein peptide. In some embodiments, the soluble RSV F protein peptide does not comprise a TM domain peptide and a CP domain peptide. In some embodiments, the soluble RSV F protein peptide does not bind to a lipid bilayer, such as a membrane or viral envelope.

In some embodiments, the RSV F protein peptide is produced by a codon optimized nucleic acid sequence. In some embodiments, the RSV F protein peptide is produced from a nucleic acid sequence that is not codon-optimized.

In some embodiments, the RSV F protein peptide may comprise any F protein sequence known in the art, such as those disclosed in US Pat. No. 10,017,543, which is incorporated herein by reference in its entirety for all purposes.

In some embodiments, the RSV antigen or immunogen is or comprises an RSV F protein peptide having the amino acid sequence from 1 to 520 of SEQ ID NO: 31 or 32. In some embodiments, the RSV antigen or immunogen is or comprises an RSV F protein peptide having the amino acid sequence from 26 to 520 of SEQ ID NO: 31 or 32.

In some embodiments, the RSV antigen or immunogen is or comprises a sequence of F2, a sequence of pep27 and a sequence of F1 (e.g. F2-pep27-F1). In some embodiments, the RSV antigen or immunogen comprises an exposed fusion peptide and has a post-fusion conformation. In some embodiments, the RSV antigen or immunogen comprises a furin cleavage site mutation. In some embodiments, the RSV antigen or immunogen comprises a furin site I mutation (e.g. R109A) and/or a furin site II mutation (e.g. R136A), in some of these examples, the RSV antigen or immunogen has a post-fusion conformation, while in other examples the RSV antigen or immunogen has a prefusion conformation. In some embodiments, the RSV antigen or immunogen comprises a furin site I mutation and a furin site II mutation (e.g. R109A/R136A), in some of these examples, the RSV antigen or immunogen comprising a full length F0, with no fusion peptide exposed and having a prefusion conformation. In some embodiments, the RSV antigen or immunogen comprises one or more mutations that prevent formation of a long helix and/or a stable α4-α5 hinge loop. In some embodiments, the RSV antigen or immunogen comprises one or more mutations that retain a prefusion conformation. In some embodiments, the RSV antigen or immunogen comprises one or more mutations that improve expression. In some embodiments, replacement of positions 161, 182 and 215 (e.g. with proline) results in higher expression levels, E161P and S215P also increase protein stability. In some embodiments, the RSV antigen or immunogen comprises E161P and/or S215P and has a prefusion conformation. In some embodiments, the RSV antigen or immunogen comprises R109A, R136A, E161P, and/or S215P and has a prefusion conformation.

In some embodiments, the viral antigen or immunogen comprises the sequence shown in SEQ ID NO: 17. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 17, including sequences containing substitutions, deletions, and/or insertions at one or more amino acid positions.

In some embodiments, the viral antigen or immunogen comprises a sequence as set forth in SEQ ID NO: 18. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 18, including sequences containing substitutions, deletions, and/or insertions at one or more amino acid positions.

In some embodiments, the viral antigen or immunogen comprises a sequence as set forth in SEQ ID NO: 19. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 19, including sequences containing substitutions, deletions, and/or insertions at one or more amino acid positions.

In some embodiments, the viral antigen or immunogen comprises a sequence as set forth in SEQ ID NO: 20. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 20, including sequences containing substitutions, deletions, and/or insertions at one or more amino acid positions.

In some embodiments, the viral antigen or immunogen comprises a sequence as set forth in SEQ ID NO: 21. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 21, including sequences containing substitutions, deletions, and/or insertions at one or more amino acid positions.

In some embodiments, the viral antigen or immunogen comprises the sequence shown in SEQ ID NO: 22. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 22, including sequences containing substitutions, deletions, and/or insertions at one or more amino acid positions.

In some embodiments, the viral antigen or immunogen comprises the sequence shown in SEQ ID NO: 23. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 23, including sequences containing substitutions, deletions, and/or insertions at one or more amino acid positions.

In some embodiments, the viral antigen or immunogen comprises the sequence shown in SEQ ID NO: 24. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 24, including sequences containing substitutions, deletions, and/or insertions at one or more amino acid positions.

In some embodiments, the viral antigen or immunogen comprises a sequence as set forth in SEQ ID NO: 25. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 25, including sequences containing substitutions, deletions, and/or insertions at one or more amino acid positions.

In some embodiments, the viral antigen or immunogen comprises a sequence as set forth in SEQ ID NO: 26. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 26, including sequences containing substitutions, deletions, and/or insertions at one or more amino acid positions.

In some embodiments, the viral antigen or immunogen comprises a sequence as set forth in SEQ ID NO: 27. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 27, including sequences containing substitutions, deletions, and/or insertions at one or more amino acid positions.

In some embodiments, the viral antigen or immunogen comprises the sequence shown in SEQ ID NO: 28. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 28, including sequences containing substitutions, deletions, and/or insertions at one or more amino acid positions.

In some embodiments, the viral antigen or immunogen comprises a sequence as set forth in SEQ ID NO: 29. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 29, including sequences containing substitutions, deletions, and/or insertions at one or more amino acid positions.

In some embodiments, the viral antigen or immunogen comprises the sequence shown in SEQ ID NO: 30. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 30, including sequences containing substitutions, deletions, and/or insertions at one or more amino acid positions.

In some embodiments, the viral antigen or immunogen comprises a sequence as set forth in SEQ ID NO: 31. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 31, including sequences containing substitutions, deletions, and/or insertions at one or more amino acid positions.

In some embodiments, the viral antigen or immunogen comprises the sequence shown in SEQ ID NO: 32. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 32, including sequences containing substitutions, deletions, and/or insertions at one or more amino acid positions.

In some embodiments, the viral antigen or immunogen comprises the sequence shown in SEQ ID NO: 32. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 32, including sequences containing substitutions, deletions, and/or insertions at one or more amino acid positions. In some embodiments, the RSV F protein peptide herein comprises substitution of A with E at residue 218 of SEQ ID NO: 32, and also comprises substitutions at one or more of the residues 106, 107, 108, 109, 133, 135, and 136. In some embodiments, the RSV F protein peptide herein comprises substitution of A with E at residue 218 of SEQ ID NO: 32, and further comprises substitutions at one or more of residues 106, 108, 109, 133, 135, or 136. In some embodiments, the RSV F protein peptide herein comprises substitution of alanine with glutamic acid at residue 218 of SEQ ID NO: 32, while one or more of residues 106, 108, 109, 133, 135, 136 are substituted with cysteine. In some embodiments, the RSV F protein peptide herein comprises substitution of alanine with glutamic acid at residue 218 of SEQ ID NO: 32, while all of residues 106, 108, 109, 133, 135, and 136 are substituted with cysteine. The RSV F protein peptide herein comprises substitution of alanine with glutamic acid at residue 218 of SEQ ID NO: 32, while all of residues 108, 109, 133, 135, and 136 are substituted with cysteine. The RSV F protein peptide herein comprises substitution of alanine with glutamic acid at residue 218 of SEQ ID NO: 32, while all of residues 109, 133, 135, and 136 are substituted with cysteine. The RSV F protein peptide herein comprises substitution of alanine with glutamic acid at residue 218 of SEQ ID NO: 32, while all of residues 133, 135, and 136 are substituted with cysteine. The RSV F protein peptide herein comprises substitution of alanine with glutamic acid at residue 218 of SEQ ID NO: 32, while all of residues 135 and 136 are substituted with cysteine. The RSV F protein peptide herein comprises substitution of alanine with glutamic acid at residue 218 of SEQ ID NO: 32, while the residue 136 is substituted with cysteine. The RSV F protein peptide herein comprises substitution of alanine with glutamic acid at residue 218 of SEQ ID NO: 32, while the residue 135 is replaced with cysteine. The RSV F protein peptide herein comprises substitution of alanine with glutamic acid at residue 218 of SEQ ID NO: 32, while all of residues 106, 133, 135, and 136 are substituted with cysteine. The RSV F protein peptide herein comprises substitution of alanine with glutamic acid at residue 218 of SEQ ID NO: 32, while all of residues 106, 135, and 136 are substituted with cysteine. The RSV F protein peptide herein comprises substitution of alanine with glutamic acid at residue 218 of SEQ ID NO: 32, while all of residues 106, 133, and 136 are substituted with cysteine. The RSV F protein peptide herein comprises substitution of alanine with glutamic acid at residue 218 of SEQ ID NO: 32, while all of residues 106, 133, and 135 are substituted with cysteine. The RSV F protein peptide herein comprises substitution of alanine with glutamic acid at residue 218 of SEQ ID NO: 32, while all of residues 106, and 136 are substituted with cysteine. The RSV F protein peptide herein comprises substitution of alanine with glutamic acid at residue 218 of SEQ ID NO: 32, while all of residues 106, and 135 are substituted with cysteine. The RSV F protein peptide herein comprises substitution of alanine with glutamic acid at residue 218 of SEQ ID NO: 32, while all of residues 108, and 136 are substituted with cysteine. The RSV F protein peptide herein comprises substitution of alanine with glutamic acid at residue 218 of SEQ ID NO: 32, while all of residues 108, and 135 are substituted with cysteine. The RSV F protein peptide herein comprises substitution of alanine with glutamic acid at residue 218 of SEQ ID NO: 32, while all of residues 106, and 133 are substituted with cysteine. The RSV F protein peptide herein comprises substitution of alanine with glutamic acid at residue 218 of SEQ ID NO: 32, while all of residues 106, and 133 are substituted with cysteine. The RSV F protein peptide herein comprises substitution of alanine with glutamic acid at residue 218 of SEQ ID NO: 32, while all of residues 108, and 133 are substituted with cysteine. The RSV F protein peptide herein comprises substitution of alanine with glutamic acid at residue 218 of SEQ ID NO: 32, while all of residues 108, and 133 are substituted with cysteine.

In some embodiments, the viral antigen or immunogen comprises a sequence as set forth in SEQ ID NO: 33. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 33, including sequences containing substitutions, deletions, and/or insertions at one or more amino acid positions.

In some embodiments, the viral antigen or immunogen comprises the sequence shown in SEQ ID NO: 34. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 34, including sequences containing substitutions, deletions, and/or insertions at one or more amino acid positions.

In some embodiments, the viral antigen or immunogen comprises the sequence shown in SEQ ID NO: 35. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 35, including sequences containing substitutions, deletions, and/or insertions at one or more amino acid positions.

In some embodiments, the viral antigen or immunogen comprises a sequence as set forth in SEQ ID NO: 36. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 36, including sequences containing substitutions, deletions, and/or insertions at one or more amino acid positions.

In some embodiments, the viral antigen or immunogen comprises a sequence as set forth in SEQ ID NO: 37. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 37, including sequences containing substitutions, deletions, and/or insertions at one or more amino acid positions.

In some embodiments, the viral antigen or immunogen comprises a sequence as set forth in SEQ ID NO: 38. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 38, including sequences containing substitutions, deletions, and/or insertions at one or more amino acid positions.

In some embodiments, the viral antigen or immunogen comprises a sequence as set forth in SEQ ID NO: 39. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 39, including sequences containing substitutions, deletions, and/or insertions at one or more amino acid positions.

In some embodiments, the viral antigen or immunogen comprises the sequence shown in SEQ ID NO: 40. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 40, including sequences containing substitutions, deletions, and/or insertions at one or more amino acid positions.

In some embodiments, the viral antigen or immunogen comprises the sequence shown in SEQ ID NO: 41. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 41, including sequences containing substitutions, deletions, and/or insertions at one or more amino acid positions.

In some embodiments, the viral antigen or immunogen comprises a sequence as set forth in SEQ ID NO: 42. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 42, including sequences containing substitutions, deletions, and/or insertions at one or more amino acid positions.

In some embodiments, the viral antigen or immunogen comprises a sequence as set forth in SEQ ID NO: 43. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 43, including sequences containing substitutions, deletions, and/or insertions at one or more amino acid positions.

In some embodiments, the viral antigen or immunogen comprises a sequence as set forth in SEQ ID NO: 44. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 44, including sequences containing substitutions, deletions, and/or insertions at one or more amino acid positions.

In some embodiments, the viral antigen or immunogen comprises a sequence as set forth in SEQ ID NO: 45. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 45, including sequences containing substitutions, deletions, and/or insertions at one or more amino acid positions.

In some embodiments, the viral antigen or immunogen comprises a sequence as set forth in SEQ ID NO: 46. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 46, including sequences containing substitutions, deletions, and/or insertions at one or more amino acid positions.

In some embodiments, the viral antigen or immunogen comprises a sequence as set forth in SEQ ID NO: 47. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity of amino acid sequences, including sequences containing substitutions, deletions, and/or insertions at one or more amino acid positions.

In some embodiments, the viral antigen or immunogen comprises a sequence selected from the group consisting of SEQ ID NO: 72-79. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to a sequence independently selected from SEQ ID NO: 64-79, including sequences containing substitutions, deletions, and/or insertions at one or more amino acid positions.

In some embodiments, the viral antigen or immunogen comprises a sequence selected from the group consisting of SEQ ID NO: 76. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 76, including sequences containing substitutions, deletions, and/or insertions at one or more amino acid positions.

In some embodiments, the viral antigen or immunogen herein may comprise RSV glycoprotein (G) or a fragment, variant or mutant thereof; RSV small hydrophobic protein (SH) or a fragment, variant or mutant thereof; RSV fusion protein (F) or a fragment, variant or mutant thereof; RSV matrix protein (M) or a fragment, variant or mutant thereof; RSV nucleoprotein (N) or a fragment, variant or mutant thereof; RSV phosphoprotein (P) or a fragment, variant or mutant thereof; RSV "large" protein (L) or a fragment, variant or mutant thereof; M2-1 protein or a fragment, variant or mutant thereof; RSV M2-2 protein or a fragment, variant or mutant thereof; RSV NS-1 protein or a fragment, variant or mutant thereof; or RSV NS-2 protein or a fragment, variant or mutant thereof; or any combination thereof.

In some embodiments, the viral antigen or immunogen is generated from a codon optimized nucleic acid sequence. In some embodiments, the viral antigen or immunogen is produced from a nucleic acid sequence that is not codon-optimized.

In some embodiments, the RSV antigen or immunogen mentioned herein may comprise a recombinant polypeptide or fusion polypeptide comprising the viral antigen or immunogen. The term viral antigen or immunogen may be used to refer to a protein comprising the RSV antigen or immunogen. In some cases, the RSV antigen or immunogen is an RSV protein peptide as provided herein.

### II. RECOMBINANT PEPTIDE AND PROTEIN

The RSV antigens and immunogens provided herein, such as RSV F protein peptides (see Section I), may be combined with other proteins or peptides, e.g. linked to form recombinant polypeptides, including fusion peptides. In some embodiments, the individual recombinant polypeptides (e.g. monomers) provided herein associate to form multimers of recombinant polypeptides, such as trimers. In some embodiments, the association of individual recombinant polypeptide monomers occurs through a covalent interaction. In some embodiments, the association of individual recombinant polypeptide monomers occurs through a non-covalent interaction. In some embodiments, the interaction (e.g. covalent or non-covalent) is influenced by a protein or peptide linked to an RSV antigen or immunogen (e.g. RSV F protein peptide). In some embodiments, for example when the RSV antigen or immunogen is an RSV F protein peptide as described herein, a protein or peptide to which it is to be linked may be selected to retain the native homotrimer structure of the glycoprotein. This may be beneficial for eliciting strong and effective immunogenic responses to RSV F protein peptides. For example, retaining and/or maintaining the native conformation of RSV antigens or immunogens (e.g. RSV F protein peptides) may improve or allow access to antigen sites capable of producing an immune response. In some cases, a recombinant polypeptide comprising the RSV F protein peptide (see section I) described herein is alternatively referred to herein as a recombinant RSV F antigen, a recombinant RSV F immunogen, or a recombinant RSV F protein.

It is also contemplated that, in some cases, the recombinant polypeptide or the multimeric recombinant polypeptide thereof is aggregated or may be aggregated to form a protein comprising a plurality of recombinant polypeptides of RSV antigens and/or immunogens. The formation of such proteins may elicit a strong and effective immunogenic response to RSV viral antigens and/or immunogens. For example, the formation of proteins comprising a plurality of recombinant polypeptides, and thus the formation of a plurality of RSV viral antigens (e.g. RSV F protein peptides), can preserve the tertiary and/or quaternary structure of viral antigens, allowing for enhanced immune responses against natural structures. In some cases, aggregation may impart stability to the RSV antigen or immunogenic structure, which in turn may allow access to potential antigen sites capable of promoting an immune response.

### 1. Fusion peptide and recombinant polypeptide

In some embodiments, the RSV antigen or immunogen may be linked at its C-terminus to a trimerization domain to facilitate monomer trimerization. In some embodiments, trimerization stabilizes the membrane proximal conformation of RSV viral antigens or immunogens (e.g. RSV F protein peptides) in a trimer conformation.

Non-limiting examples of exogenous multimerization domains that facilitate the stable trimer of soluble recombinant proteins include: GCN 4 leucine zipper (Harbury et al. 1993 Science 262: 1401-1407), a trimerization motif from pulmonary surfactant protein (Hoppe et al. 1994 FEBS Lett 344: 191-195), collagen (McAlinden, et al. 2003 J Biol Chem 278: 42200-42207) and the phage T4 fibritin foldon (Miroshnikov et al. 1998 Protein Eng 11: 329-414), either of which can be linked to the recombinant RSV antigen or immunogen described herein (e.g. by being linked to the C-terminus of RSV F peptide) to promote trimerization of recombinant viral antigens or immunogens. See further US Pat. Nos. 7,268,116, 7,666,837, 7,691,815, 10,618,949, 10,906,944 and 10,960,070, and US 2020/0009244, which are incorporated herein by reference in their entirety for all purposes.

In some embodiments, recombinant viral antigens or immunogens and multimerization domains may be linked using one or more peptide linkers, such as gly-ser linkers, e.g. 10 amino acids glycine-serine peptide linkers. The trimer may include any stabilizing mutation (or combination thereof) as described herein, as long as the recombinant viral antigen or immunogen trimer retains desired characteristics (e.g. a prefusion conformation).

For therapeutic feasibility, the desired trimerization protein portion for the biological drug design should meet the following criteria. Ideally, it should be part of a naturally secreted protein, such as immunoglobulin Fc, that is abundant in circulation (non-toxic), derived from humans (lacking immunogenicity), relatively stable (half-life), and capable of being efficiently trimerized (stabilized by interchain covalent disulfide bonds), thus stabilizing the trimeric RSV antigen or immunogen structure.

Collagen belongs to the fibrous protein family and is the main component of the extracellular matrix. It is the most abundant protein in mammals and occupies approximately 25% of the total protein in the body. Collagen plays a major structural role in bone, tendon, skin, cornea, cartilage, blood vessels, and tooth formation. The fiber types of collagen I, II, III, IV, V, and XI are synthesized into a larger trimer precursor, referred to as procollagen, wherein a central uninterrupted triple-helical domain consisting of hundreds of "G-X-Y" repeat sequences (or glycine repeating sequences) is flanked by a non-collagen domain (NC), an N-propeptide, and a C-propeptide. Both the C-terminal and N-terminal extensions are subjected to proteolytic processes after procollagen secretion. This event triggers the assembly of mature proteins into collagen fibrils, thereby forming an insoluble cell matrix. BMP-1 is a protease that recognizes the specific peptide sequence of the procollagen in the vicinity of the boundary between the glycine repeat sequence and the collagen C propeptide and is responsible for removing the propeptide. It is found that the concentration of the exfoliated trimer C propeptide of type I collagen in normal adult human serum is in the range of 50-300 ng/mL, and the level of children is much higher, indicating that bone formation is active. In a person having a family high serum type I collagen C propeptide concentration, the level can be as high as 1-6 µg/mL with no significant anomaly, indicating that the C propeptide is non-toxic. A structural study of the collagen trimer C propeptide indicates that it is a trilobal structure in which all three subunits are brought together in a junction region near its N-terminus and attached to the remainder of the procollagen molecule. The geometry of the protein to be fused extends in one direction similar to the geometry of the Fc dimer.

Type I, type IV, type V and type XI collagen are mainly assembled in the form of heterotrimers consisting of two α-1 chains and one α-2 chain (for type I, type IV, type V) or three different but highly homologous chains (for type XI). Both type II and type III collagen are homotrimers composed of the α-1 chain. For the most abundant collagen form of type I collagen, stable α(I) homotrimers are also formed and present in different tissues at variable levels. Most of these collagen C propeptide chains can be self-assembled into homotrimers when overexpressed alone in cells. Although the N propeptide domains are synthesized first, the molecular assembly into trimeric collagen begins with mutual registration of the C-propeptide. C propeptide complexes are believed to be stabilized by forming interchain disulfide bonds, but the necessity for disulfide bonds to form suitable chain registration is unclear. The glycine-based triple helix is repeated and then propagated from the associated C-terminus to the N-terminus in a zipper-like manner. This recognition has produced a non-native type of collagen matrix by exchanging C propeptides of different collagen chains using recombinant DNA techniques. Non-collagen, such as cytokines and growth factors, are also fused to the N-terminus of the collagen or mature collagen to form a new collagen matrix, which is intended to allow slow release of non-collagen from the extracellular matrix. However, in both cases, it is necessary to cleave the C-propeptide before the recombinant collagen fibril is assembled into an insoluble cell matrix.

Although other protein trimerization domains have been previously described, such as GCN 4 from yeast, the fibritin foldon of phage T4 and the trimerization domain of aspartate transcarbamylase of *Escherichia coli,* which allow for trimerization of heterologous proteins, none of these trimerization proteins are native human proteins, nor naturally secreted proteins. Thus, any trimeric fusion protein must be produced within the cell, which may not only cause native secreted proteins (e.g. soluble receptors) to fold incorrectly, but also make it difficult to purify such fusion proteins from thousands of other intracellular proteins. Furthermore, a fatal drawback of using such non-human protein trimerization domains (e.g. from yeast, phage, and bacteria) for trimeric biopharmaceuticals design is their potential immunogenicity in the human body such that such fusion proteins become ineffective shortly after they are injected into the human body.

Therefore, the use of collagen in recombinant polypeptides as described herein has a number of advantages, including: (1) collagen is the most abundant protein secreted in mammals, accounting for approximately 25% of the total protein in the body; (2) the main forms of the collagen are naturally present as a triple helix, and the spherical C propeptide thereof is responsible for inducing trimerization; (3) it is found that the collagen trimeric C propeptide released by the proteolytic cleavage of mature collagen is naturally present in mammalian blood at sub-microgram/ml level and is known to be non-toxic to the body; (4) the linear triple-helical region of collagen can be included as a linker, the spacing of each residue is predicted to be 2.9 Å, or can be excluded from the portion of the fusion protein, so the distance between the protein to be trimerized and the collagen C propeptide can be precisely adjusted to achieve optimal biological activity; (5) the BMP1 recognition site of the pre-collagen cleavage C propeptide can be mutated or deleted to prevent the disruption of the trimeric fusion protein; and (6) the C propeptide domain trimerizes via a disulfide bond, which provides a universal affinity tag and can be used for purifying any secreted fusion protein produced. In some embodiments, the collagen C propeptide linked to the RSV antigen and immunogen (e.g. RSV F protein peptide) enables recombination to produce a soluble covalently linked homotrimeric fusion protein.

In some embodiments, the RSV antigen or immunogen is linked to a C-terminal propeptide of collagen to form a recombinant polypeptide. In some embodiments, the C-terminal propeptide of the recombinant polypeptide forms an inter-polypeptide disulfide bond. In some embodiments, the recombinant protein forms a trimer. In some embodiments, the RSV antigen or immunogen is an RSV F protein peptide as described in section I**.**

In some embodiments, the C-terminal propeptide is derived from human collagen. In some embodiments, the C-terminal propeptide comprises a C-terminal polypeptide or a fragment thereof from proα1 (I), proα1 (II), proα1 (III), proα1 (V), proα1 (XI), proα2 (I), proα2 (V), proα2 (XI), or proα3 (XI). In some embodiments, the C-terminal propeptide is or comprises a C-terminal polypeptide of proα1 (I).

In some embodiments, the C-terminal propeptide is or comprises an amino acid sequence shown in SEQ ID NO: 48. In some embodiments, the C-terminal propeptide is an amino acid sequence having at least or about 85%, 90%, 92%, 95%, or 97% sequence identity to the sequence of SEQ ID NO: 48. In some embodiments, the C-terminal propeptide is or comprises the amino acid sequence shown in SEQ ID NO: 49. In some embodiments, the C-terminal propeptide is an amino acid sequence having at least or about 85%, 90%, 92%, 95%, or 97% sequence identity to the sequence of SEQ ID NO: 49. In some embodiments, the C-terminal propeptide is or comprises the amino acid sequence shown in SEQ ID NO: 50. In some embodiments, the C-terminal propeptide is an amino acid sequence having at least or about 85%, 90%, 92%, 95%, or 97% sequence identity to the sequence of SEQ ID NO: 50. In some embodiments, the C-terminal propeptide is or comprises the amino acid sequence shown in SEQ ID NO: 51. In some embodiments, the C-terminal propeptide is an amino acid sequence having at least or about 85%, 90%, 92%, 95%, or 97% sequence identity to the sequence of SEQ ID NO: 51. In some embodiments, the C-terminal propeptide is or comprises the amino acid sequence shown in SEQ ID NO: 52. In some embodiments, the C-terminal propeptide is an amino acid sequence having at least or about 85%, 90%, 92%, 95%, or 97% sequence identity to the sequence of SEQ ID NO: 52. In some embodiments, the C-terminal propeptide is or comprises an amino acid sequence shown in SEQ ID NO: 53. In some embodiments, the C-terminal propeptide is an amino acid sequence having at least about 85%, 90%, 92%, 95%, or 97% sequence identity to the sequence of SEQ ID NO: 53.

In some embodiments, the C-terminal propeptide is or comprises an amino acid sequence as shown in SEQ ID NO: 54. In some embodiments, the C-terminal propeptide is an amino acid sequence having at least or about 85%, 90%, 92%, 95%, or 97% sequence identity to the sequence of SEQ ID NO: 54. In some embodiments, the C-terminal propeptide is or comprises an amino acid sequence as shown in SEQ ID NO: 55. In some embodiments, the C-terminal propeptide is an amino acid sequence having at least or about 85%, 90%, 92%, 95%, or 97% sequence identity to the sequence of SEQ ID NO: 55. In some embodiments, the C-terminal propeptide is or comprises the amino acid sequence shown in SEQ ID NO: 56. In some embodiments, the C-terminal propeptide is an amino acid sequence having at least or about 85%, 90%, 92%, 95%, or 97% sequence identity to the sequence of SEQ ID NO: 56. In some embodiments, the C-terminal propeptide is or comprises the amino acid sequence shown in SEQ ID NO: 57. In some embodiments, the C-terminal propeptide is an amino acid sequence having at least or about 85%, 90%, 92%, 95%, or 97% sequence identity to the sequence of SEQ ID NO: 57. In some embodiments, the C-terminal propeptide is or comprises an amino acid sequence shown in SEQ ID NO: 58. In some embodiments, the C-terminal propeptide is an amino acid sequence having at least or about 85%, 90%, 92%, 95%, or 97% sequence identity to the sequence of SEQ ID NO: 58. In some embodiments, the C-terminal propeptide is or comprises the amino acid sequence shown in SEQ ID NO: 59. In some embodiments, the C-terminal propeptide is an amino acid sequence having at least or about 85%, 90%, 92%, 95%, or 97% sequence identity to the sequence of SEQ ID NO: 59.

In some embodiments, the C-terminal propeptide is or comprises an amino acid sequence as shown in SEQ ID NO: 60. In some embodiments, the C-terminal propeptide is an amino acid sequence having at least or about 85%, 90%, 92%, 95%, or 97% sequence identity to the sequence of SEQ ID NO: 60. In some embodiments, the C-terminal propeptide is or comprises an amino acid sequence as shown in SEQ ID NO: 61. In some embodiments, the C-terminal propeptide is an amino acid sequence having at least or about 85%, 90%, 92%, 95%, or 97% sequence identity to the sequence of SEQ ID NO: 61. In some embodiments, the C-terminal propeptide is or comprises an amino acid sequence as shown in SEQ ID NO: 62. In some embodiments, the C-terminal propeptide is an amino acid sequence having at least or about 85%, 90%, 92%, 95%, or 97% sequence identity to the sequence of SEQ ID NO: 62. In some embodiments, the C-terminal propeptide is or comprises an amino acid sequence as shown in SEQ ID NO: 63. In some embodiments, the C-terminal propeptide is an amino acid sequence having at least or about 85%, 90%, 92%, 95%, or 97% sequence identity to the sequence of SEQ ID NO: 63.

In some embodiments, the C-terminal propeptide is or comprises an amino acid sequence of a collagen trimerization domain (e.g. a C propeptide of human α1 (I) collagen), wherein aspartic acid (D) in the BMP-1 site is replaced with asparagine (N), e.g. wherein the RAD is mutated to RAN. In some embodiments, the C-terminal propeptide is or comprises an amino acid sequence of a collagen trimerization domain (e.g. a C propeptide of human α1 (I) collagen), wherein alanine (A) in the BMP-1 site is replaced with asparagine (N), e.g. wherein RAD is mutated to RND. In some embodiments, the C-terminal propeptide herein may comprise a mutated BMP-1 site, such as RSAN replacing DDAN. In some embodiments, the C-terminal propeptide herein may comprise a BMP-1 site, such as a sequence comprising RAD (e.g. RADDAN) sequence rather than RAN (e.g. RANDAN) or RND (e.g. RNDDAN) can be used in the fusion polypeptide disclosed herein.

In some embodiments, the C-terminal propeptide is or comprises an amino acid sequence as a fragment of any one of SEQ ID NOs: 48-63.

In some embodiments, the C-terminal propeptide may comprise a sequence comprising a Gly-X-Y repeat sequence, wherein X and Y are independently any amino acids, or a sequence having at least 85%, 90%, 92%, 95%, or 97% identity thereto, enabling the formation of inter-polypeptide disulfide bonds and to enable the recombinant polypeptide to trimerize amino acid sequence. In some embodiments, X and Y are independently proline or hydroxyproline.

In some cases, in which the RSV F peptide protein (e.g. RSV antigen or immunogen, e.g. see section I) is linked to a C-terminal propeptide to form a recombinant polypeptide, the recombinant polypeptide forms a trimer, thereby producing the homotrimer of RSV F protein peptide. In some embodiments, the trimerized recombinant polypeptide comprises a crutch shaped rod-like F protein peptide trimer. In some embodiments, the RSV F protein peptide of the trimerized recombinant polypeptide is in a pre-fusion conformation. In some embodiments, the RSV F protein peptide of the trimerized recombinant polypeptide is in a post-fusion conformation. In some embodiments, the conformational state allows access to different antigen sites on the F protein peptide. In some embodiments, the antigenic site is an epitope, such as a linear epitope or a conformational epitope. One advantage of having the trimerized recombinant polypeptide is that an immune response against various potentially different antigen sites can be enhanced.

In some embodiments, the trimerized recombinant polypeptide comprises an individual recombinant polypeptide comprising the same viral antigen or immunogen. In some embodiments, the trimerized recombinant polypeptide comprises individual recombinant polypeptides that each comprise a different viral antigen or immunogen than the other recombinant polypeptides. In some embodiments, the trimerized recombinant polypeptide comprises an individual recombinant polypeptide, wherein one of the individual recombinant polypeptides comprises a viral antigen or immunogen different from other recombinant polypeptides. In some embodiments, the trimerized recombinant polypeptide comprises an individual recombinant polypeptide, wherein two of the individual recombinant polypeptides comprise the same viral antigen or immunogen, and the viral antigen or immunogen is different from the viral antigen or immunogen contained in the remaining recombinant polypeptide.

In some embodiments, the recombinant polypeptide comprises any RSV antigen or immunogen described in Section I. In some embodiments, the recombinant polypeptide comprises any RSV antigen or immunogen described in Section I, which is connected as described herein to a C-terminal propeptide of collagen as described herein.

In some embodiments, the recombinant polypeptide or the fusion protein comprises a first sequence of any one of SEQ ID NOs: 17-47 and 72-79 linked to a second sequence shown in any one of SEQ ID NOs: 48-63, wherein the C-terminal of the first sequence is directly linked to the N-terminal of the second sequence.

In some embodiments, the recombinant polypeptide or the fusion protein comprises a first sequence of any one of SEQ ID NOs: 17-47 and 72-79 linked to a second sequence shown in any one of SEQ ID NOs: 48-63, wherein the C-terminal of the first sequence is indirectly linked to the N-terminal of the second sequence, such as by a linker. In some embodiments, the linker comprises a sequence containing a Gly-X-Y repeat sequence.

In some embodiments, the recombinant polypeptide is or comprises a sequence as shown in SEQ ID NO: 1. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 1, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof. In some embodiments, the recombinant polypeptide is or comprises a variant of SEQ ID NO: 1, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 2. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 2, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 2, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 3. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 3, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 3, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 4. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 4, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 4, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 5. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 5, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 5, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 6. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 6, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 6, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 7. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 7, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 7, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence shown in SEQ ID NO: 8. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 8, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 8, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO: 9. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 9, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 9, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence shown in SEQ ID NO: 10. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 10, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 10, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO:11. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 11, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 11, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence shown in SEQ ID NO:12. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 12, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 12, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence shown in SEQ ID NO:13. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 13, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 13, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence shown in SEQ ID NO:14. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 14, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 14, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence shown in SEQ ID NO:15. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 15, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 15, the variant comprising any one, two, three, four, five or more mutations, or any combination thereof selected from the group consisting of P102A, R109A, R136A, E161P, E218A, S215P, I379A, and M447V.

In some embodiments, the recombinant polypeptide is or comprises a sequence shown in SEQ ID NO:16. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 16, including a sequence comprising substitutions, deletions, and/or insertions at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 16, the variant comprising any one, two, three, four, five or more mutations, or any combination thereof selected from the group consisting of P102A, R109A, R136A, E161P, E218A, S215P, I379A and M447V.

In some embodiments, the recombinant polypeptide is or comprises a sequence selected from the group consisting of SEQ ID NO: 64-71. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to a sequence independently selected from SEQ ID NO: 64-71.

In some embodiments, the recombinant polypeptide is or comprises a sequence shown in SEQ ID NO: 64. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98% or 99% sequence identity to the sequence of SEQ ID NO: 64, including a sequence comprising substitutions, deletions, and/or insertions at one or more amino acid positions such as 102, 107, 109, 131, 132, 134, 137, 138, 139, 140, 141, 142, 143, 144, 145, 146, 147, 148, 149, 150, 151, 152, 153, 154, 155, 161, 215, 218, 379 or 447 (relative to the amino acid position of SEQ ID NO: 31 or 32) or any combination thereof . In some embodiments, the recombinant polypeptide is or comprises a variant of SEQ ID NO: 64, the variant comprising any one, two, three, four, five or more mutations, or any combination thereof selected from the group consisting of P102A, R109A, R136A, E161P, E218A, S215P, I379A, and M447V.

As noted above, in some embodiments, the recombinant polypeptide provided herein not only associates to form a trimer, but may also aggregates or are aggregated to produce a protein comprising a plurality of recombinant polypeptides. In some embodiments, the formed protein has a macrostructure. In some cases, the macrostructure may impart structural stability to the RSV antigen or immunogen recombinant polypeptide, which in turn may allow access to potential antigen sites capable of promoting an immune response.

In some embodiments, the trimerized recombinant polypeptide is aggregated to form a protein containing a plurality of trimerized recombinant polypeptides. In some embodiments, the plurality of trimerized recombinant polypeptides form a protein having a macrostructure. In some embodiments, the protein comprises a rosette-like oligomer comprising a crutch-shaped rod-like F-protein peptide trimer.

In some embodiments, provided herein is a complex comprising any suitable combination of recombinant polypeptides or fragments, variants or mutants thereof selected from the group consisting of SEQ ID NOs: 1-16 and 64-71 is provided herein. In some embodiments, provided herein is a complex comprising a trimer of a recombinant polypeptide or fragment, variant or mutant thereof selected from the group consisting of SEQ ID NOs: 1-16 and 64-71, wherein the recombinant polypeptide is trimerized by disulfide bonds between polypeptides to form the trimer.

In some embodiments, the protein comprising the plurality of recombinant polypeptides described herein is an immunogen. In some embodiments, a protein comprising a plurality of recombinant polypeptides described herein is contained in nanoparticles. For example, in some embodiments, the protein is directly linked to nanoparticles, such as protein nanoparticles. In some embodiments, the protein is indirectly linked to the nanoparticles. In some embodiments, a protein comprising a plurality of recombinant polypeptides described herein is contained in a virus-like particle (VLP).

### 2. Polynucleotide and vector

Also provided herein are polynucleotides (nucleic acid molecules) encoding RSV antigens or immunogens and recombinant polypeptides provided herein, and vectors for genetically engineering cells to express such RSV antigens or immunogens and recombinant polypeptides.

In some embodiments, provided herein are polynucleotides encoding recombinant polypeptides provided herein. In some aspects, the polynucleotide comprises a single nucleic acid sequence, such as a nucleic acid sequence encoding a recombinant polypeptide. In other cases, the polynucleotide comprises a first nucleic acid sequence encoding a recombinant polypeptide comprising a specific RSV antigen or an immunogen and a second nucleic acid sequence encoding a recombinant polypeptide comprising different RSV antigens or immunogens.

In some embodiments, a polynucleotide encoding a recombinant polypeptide contains at least one promoter operably linked to control the expression of the recombinant polypeptide. In some embodiments, the polynucleotide contains two, three or more promoters, which are operably linked to control the expression of the recombinant polypeptide.

In some embodiments, for example, when the polynucleotide contains two or more nucleic acid coding sequences, such as a sequence encoding a recombinant polypeptide comprising different RSV antigens or immunogens, at least one promoter is operably linked to control the expression of the two or more nucleic acid sequences. In some embodiments, the polynucleotide contains two, three or more promoters, which are operably linked to control the expression of the recombinant polypeptide.

In some embodiments, the expression of the recombinant polypeptide is inducible or conditional. Thus, in some aspects, the polynucleotide encoding the recombinant polypeptide comprises a conditional promoter, an enhancer, or a trans-activator. In some such aspects, the conditional promoter, enhancer, or trans-activator is an inducible promoter, an enhancer or a trans-activator, or an inhibitory promoter, an enhancer, or a trans-activator. For example, in some embodiments, the expression of the recombinant polypeptide may be limited to a particular microenvironment using an inducible or conditional promoter. In some embodiments, the expression driven by an inducible or conditional promoter is regulated by exposure to an exogenous factor such as heat, radiation, or a drug.

Where the polynucleotide contains more than one nucleic acid sequence encoding a recombinant polypeptide, the polynucleotide may also include a nucleic acid sequence encoding the peptide between the one or more nucleic acid sequences. In some cases, the nucleic acid encoded peptide located between the nucleic acid sequences separates the translation product of the nucleic acid sequence during or after translation. In some embodiments, the peptide contains an internal ribosome entry site (IRES), a self-cleaving peptide, or a peptide that causes a ribosome jump, such as a T2A peptide.

In some embodiments, polynucleotides encoding recombinant polypeptides are introduced into compositions containing cultured cells (e.g. host cells), such as by retroviral transduction, transfection or transformation. In some embodiments, this may allow for expression (e.g. production) of recombinant polypeptides. In some embodiments, the expressed recombinant polypeptide is purified.

In some embodiments, the polynucleotides (nucleic acid molecules) provided herein encode RSV antigens or immunogens as described herein. In some embodiments, the polynucleotide (nucleic acid molecule) provided herein encodes a recombinant polypeptide comprising an RSV antigen or an immunogen, such as the RSV F peptide protein described herein.

A vector or construct containing a nucleic acid molecule as described herein is also provided. In some embodiments, the vector or construct contains one or more promoters that are operably linked to nucleic acid molecules encoding recombinant polypeptides to drive their expression. In some embodiments, the promoter is operably linked to one or more nucleic acid molecules, such as nucleic acid molecules encoding recombinant polypeptides containing different RSV viral antigens or immunogens.

In some embodiments, the vector is a viral vector. In some embodiments, the viral vector is a retroviral vector. In some embodiments, the retroviral vector is a lentiviral vector. In some embodiments, the retroviral vector is a γ retroviral vector.

In some embodiments, the vector or construct includes a single promoter that drives expression of one or more nucleic acid molecules of a polynucleotide. In some embodiments, such a promoter may be a polycistronic promoter (bicistronic or tricistronic), see, e.g. US Pat. No. 6,060,273. For example, in some embodiments, the transcription unit can be engineered as a bicistronic unit containing an IRES (internal ribosome entry site), thereby allowing gene products to be co-expressed by signals from a single promoter (e.g. encoding different recombinant polypeptides). In some embodiments, the vector provided herein is bicistronic, allowing the vector to contain and express two nucleic acid sequences. In some embodiments, the vector provided herein is tricistronic, allowing the vector to contain and express three nucleic acid sequences.

In some embodiments, a single promoter directs RNA expression that contains two or three genes in a single open reading frame (ORF) (e.g. encoding a chimeric signaling receptor and a recombinant receptor) that are separated from one another by a sequence encoding a self-cleaving peptide (e.g. 2A sequence) or a protease recognition site (e.g. furin). The ORF thus encodes a single polypeptide that is processed into individual proteins during translation (in the case of 2A) or after translation. In some cases, the peptide, such as T2A, may lead to ribosome skipping (ribosome jump) to synthesize peptide bonds at the C-terminus of 2A element, resulting in the 2A sequence end being separated from the next downstream peptide (see, e.g. de Felipe. Genetic Vaccines and Ther. 2: 13(2004); and de Felipe et al. Traffic 5: 616-626(2004)). Many 2A elements are known in the art. Examples of 2A sequences that may be used in the methods and nucleic acids disclosed herein include, but are not limited to, 2A sequences from foot-and-mouth disease virus (F2A), equine rhinitis A virus (E2A), Thosea asigna virus (T2A), and porcine teschovirus-1 (P2A) as described in US Patent Publication No. 20070116690.

In some embodiments, the vector is contained in a virus. In some embodiments, the virus is a pseudovirus. In some embodiments, the virus is a virus-like particle. In some embodiments, the carrier is contained in a cell. In some embodiments, the virus or cell containing the vector contains a recombinant genome.

### III. IMMUNOGENIC COMPOSITION AND FORMULATION

In some embodiments, provided herein is an immunogenic composition comprising a trimer of a recombinant polypeptide or a combination of any two or more of the trimers comprising a sequence selected from the group consisting of SEQ ID NOs: 1-16 and 64-71. In some embodiments, the unit dose of the immunogenic composition may comprise from about 10 µg to about 100 µg of the RSV F antigen, preferably from about 25 µg to about 75 µg of the RSV F antigen, preferably from about 40 µg to about 60 µg of the RSV F antigen or about 50 µg of the RSV F antigen. In some embodiments, the dose contains 3 µg of the RSV F antigen. In other embodiments, the dose contains 9 µg of the RSV F antigen. In other embodiments, the dose contains 30 µg of the RSV F antigen.

In some cases, it may be desirable to combine the disclosed immunogens with other drugs (e.g. vaccines) that induce protective responses to other factors. For example, a composition comprising the recombinant RSV F antigen (e.g. trimer or protein) described herein may be administered simultaneously (typically separately) or sequentially with other vaccines recommended by the Advisory Committee on Immunization Practices (ACIP; cdc.gov/vaccines/acip/index.html) for the target age group (e.g., infants about 1 to 6 months old), such as influenza vaccines or varicella zoster vaccines. Thus, the disclosed immunogens comprising the recombinant RSV F antigen described herein can be administered simultaneously or sequentially with vaccines, such as those for hepatitis B (HepB), diphtheria, tetanus and pertussis (DTaP), pneumococcal (PCV), Haemophilus influenzae type b (Hib), poliovirus, influenza, and rotavirus.

Multivalent or combined vaccines provide protection against a variety of pathogens. In some cases, multivalent vaccines may provide protection against multiple serotypes or strains of the same pathogen. In some instances, multivalent vaccines provide protection against a variety of pathogens, such as the combined Tdap vaccine, which provides protection against strains of tetanus, pertussis, and diphtheria. Multivalent vaccines are highly desirable for minimizing the number of vaccinations required to provide protection against a variety of pathogens or pathogenic types or strains, reducing the cost of administration and increasing coverage. This may be particularly useful when vaccinating an infant or child, for example.

In some embodiments, a vaccine comprising an immunogenic composition described herein, for example, is a multivalent vaccine. In some embodiments, the antigenic material for incorporation into the multivalent vaccine composition of the present invention is derived from RSV subtype A or subtype B, or a combination thereof. Antigens for incorporation into the multivalent vaccine compositions of the present invention can be derived from one RSV strained or a plurality of strains, e.g. from two to five strains, in order to provide a broader scope of protection. In one embodiment, the antigen for incorporation into the multivalent vaccine composition of the present invention is derived from multiple RSV strains. Other useful antigens include live viruses, attenuated viruses, and inactivated viruses, such as inactivated poliovirus (Jiang et al. J Biol. Stand. (1986) 14: 103-9), Attenuated hepatitis A virus strain (Bradley et al. J Med. Virol. (1984) 14: 373-86), attenuated measles virus (James et al. N Engl. J Med. (1995) 332: 1262-6) and pertussis virus epitopes (e.g. ACEL-IMUNErM acellular DTP, Wyeth-Lederle vaccine, and children's medication).

In some aspects, the vaccines provided herein are universal vaccines. In some embodiments, the universal vaccine is a vaccine against multiple strains of the same virus, such as multiple RSV strains. Developing an efficient generic RSV vaccine will reduce the cost and labor of, for example, seasonal vaccine formulation and allow more robust pandemic prevention.

In some aspects, the universal vaccine is a vaccine comprising a plurality of epitopes derived from different viral strains. In some aspects, the universal vaccine contains a single epitope that is conserved among different viral strains. For example, a universal vaccine may be based on a relatively conserved domain of RSV F protein.

Also provided are immunogenic compositions comprising the disclosed immunogens (e.g. the disclosed recombinant RSV F antigen or a nucleic acid molecule encoding the disclosed recombinant RSV F antigen) and a pharmaceutically acceptable carrier. In some embodiments, the immunogenic composition comprises the trimerized recombinant polypeptide provided herein and optionally a pharmaceutically acceptable carrier. In some embodiments, the immunogenic composition comprises a protein comprising a plurality of trimerized recombinant polypeptides provided herein and optionally a pharmaceutically acceptable carrier. In some embodiments, the immunogenic composition comprises the protein nanoparticles provided herein and optionally a pharmaceutically acceptable carrier. In some embodiments, the immunogenic composition comprises a VLP provided herein and optionally a pharmaceutically acceptable carrier. In some embodiments, the immunogenic composition comprises the isolated nucleic acid provided herein and optionally a pharmaceutically acceptable carrier. In some embodiments, the immunogenic composition comprises a vector provided herein and optionally a pharmaceutically acceptable carrier. In some embodiments, the immunogenic composition comprises a virus provided herein and optionally a pharmaceutically acceptable carrier. In some embodiments, the immunogenic composition comprises the pseudovirus provided herein and optionally a pharmaceutically acceptable carrier. In some embodiments, the immunogenic composition comprises the cells provided herein and optionally a pharmaceutically acceptable carrier. In some embodiments, the immunogenic composition is a vaccine as described herein. In some embodiments, the vaccine is a prophylactic vaccine. In some embodiments, the vaccine is a therapeutic vaccine. In some embodiments, the vaccine is a prophylactic vaccine and a therapeutic vaccine. Such pharmaceutical compositions can be administered to a subject by various modes of administration known to one of persons skilled in the art, such as intramuscular, intradermal, subcutaneous, intravenous, intra-arterial, intra-articular, intraperitoneal, intranasal, sublingual, tonsillar, oropharyngeal, or other parenteral and mucosal routes. In several embodiments, the pharmaceutical composition comprising one or more of the disclosed immunogens is an immunogenic composition. Actual methods for preparing a pharmaceutical composition will be known or apparent to those skilled in the art and are described in more detail in Remington: The Science and Practice of Pharmacy, 19th ed., Mack Publishing Company, Easton, Pa. (1995).

Thus, immunogens, such as recombinant RSV F antigens, such as trimers and proteins described herein, may be formulated with pharmaceutically acceptable carriers to help preserve biological activity while also helping to increase stability during storage over an acceptable temperature range. Possible carriers include, but are not limited to, physiologically balanced media, phosphate buffered saline solutions, water, emulsions (e.g. oil/water or water/oil emulsions), various types of wetting agents, cryoprotective additives or stabilizers such as proteins, peptides or hydrolysates (e.g. albumin, gelatin), sugars (e.g. sucrose, lactose, sorbitol), amino acids (e.g. sodium glutamate), or other protective agents. The resulting aqueous solution may be used as such or lyophilized. The lyophilized formulation is combined with a sterile solution prior to administration for single or multiple administration.

Formulated compositions, especially liquid formulations, may contain bacteriostatic agents to prevent or minimize degradation during storage, including, but not limited to an effective concentration (typically 1% w/v), phenol, m-cresol, chlorobutanol, methyl p-hydroxybenzoate and/or propyl p-hydroxybenzoate. Some patients may be sensitive to a bacteriostatic agent; thus, the lyophilized formulation may be reconstituted in a solution that contains or does not contain such ingredients.

The immunogenic compositions of the present disclosure can contain a pharmaceutically acceptable excipient to approximate physiological conditions, such as pH adjustment and buffering agents, tonicity adjusting agents, wetting agents, and the like, as desired, such as sodium acetate, sodium lactate, sodium chloride, potassium chloride, calcium chloride, sorbitan monolaurate and triethanolamine oleate. The immunogenic composition may optionally include an adjuvant to enhance the immune response of the host. Suitable adjuvants are, for example, toll-like receptor agonists, aluminum agents, AlPO₄, aluminum hydrogels, lipid A and derivatives or variants thereof, oil emulsions, saponins, neutral liposomes, liposomes containing vaccine antigens and cytokines, non-ionic block copolymers, and chemokines. Non-ionic block polymers containing polyoxyethylene (POE) and polyoxypropylene (POP) such as POE-POP-POE block copolymers, MPL ^{™} (3-O- deacylated monophosphoryl lipid A; Corixa, Hamilton, Ind. and IL-12 (Genetics Institute, Cambridge, Mass). And many other suitable adjuvants known in the art can be used as adjuvants (Newman et al. 1998, Critical Reviews in Therapeutic Drug Carrier Systems 15: 89-142). The advantage of these adjuvants is that they help stimulate the immune system in a non-specific manner, thereby enhancing the immune response to the drug. In some embodiments, the immunogenic composition of the present disclosure may include more than one adjuvant or be administered with more than one adjuvant. In some embodiments, the immunogenic composition of the present disclosure may include or be administered with two adjuvants. In some embodiments, the immunogenic compositions of the present disclosure may include or be administered with a variety of adjuvants. For example, in some instances, a vaccine including an immunogenic composition provided herein may include, or be administered in combination with, a variety of adjuvants.

Examples of suitable adjuvants for vaccine compositions include, for example, aluminum hydroxide, lecithin, Freund's adjuvant, MPL ^{™}, and IL-12. In some embodiments, the vaccine compositions or nanoparticle immunogens disclosed herein (e.g. RSV vaccine compositions) may be formulated controlled-release or timed-release formulations. This may be accomplished in a composition containing a sustained release polymer or by a micro-encapsulated delivery system or a bioadhesive gel. Various pharmaceutical compositions can be prepared according to standard procedures well known in the art.

In some embodiments, the immunogenic composition of the present invention may contain an adjuvant formulation, a metabolizable oil in the form of an oil-in-water emulsion (e.g. squalene) and α-tocopherol, and polyoxyethylene sorbitan monooleate (Tween -80). In some embodiments, the adjuvant formulation may comprise from about 2% to about 10% squalene, from about 2 to about 10% α-tocopherol (e.g. D-α-tocopherol) and from about 0.3 to about 3% polyoxyethylene sorbitan monooleate. In some embodiments, the adjuvant formulation may comprise about 5% squalene, about 5% tocopherol, and about 0.4% polyoxyethylene sorbitan monooleate. In some embodiments, the immunogenic composition of the present disclosure may contain an adjuvant in the form of a 3-O-deacylated monophosphoryl lipid A (3D-MPL) and an oil-in-water emulsion containing metabolizable oil, α-tocopherol and polyoxyethylene sorbitan monooleate. In some embodiments, the immunogenic composition of the present disclosure may contain QS21 (Quillaja saponaria Molina extract, fraction 21), 3D-MPL, and water-in-oil emulsion, wherein the oil-in-water emulsion comprises metabolizable oil, α-tocopherol and polyoxyethylene sorbitan monooleate. In some embodiments, an immunogenic composition of the present disclosure may contain QS21, 3D-MPL, and an oil-in-water emulsion, wherein the oil-in-water emulsion has the following composition: metabolizable oil such as squalene, α-tocopherol, and Tween-80 in some embodiments, the immunogenic composition of the present disclosure may contain an adjuvant in the form of a liposome composition.

In some embodiments, the immunogenic compositions of the present invention may contain an adjuvant formulation comprising a metabolizable oil (e.g., squalene), polyoxyethylene sorbitan monooleate (Tween-80), and Span 85. In some embodiments, the adjuvant formulation may comprise about 5% (w/v) squalene, about 0.5% (w/v) polyoxyethylene sorbitan monooleate, and about 0.5% (w/v) Span 85.

In some embodiments, an immunogenic composition of the present disclosure may contain, for example, an adjuvant formulation in the form of a nanoparticle composition comprising saponin, cholesterol, and phospholipids. In some embodiments, the immunogenic compositions of the present disclosure may contain a mixture of separately purified Quillaja fractions, which are then formulated with cholesterol and phospholipids.

In some embodiments, the immunogenic composition of the present disclosure may contain an adjuvant selected from the group consisting of MF59^{™}, Matrix-A^{™}, Matrix-C^{™}, Matrix-M^{™}, AS01, AS02, AS03, and AS04.

In some embodiments, the immunogenic composition of the present disclosure may contain a toll-like receptor 9 (TLR9) agonist, wherein the TLR9 agonist is an oligonucleotide having a length of 8 to 35 nucleotides and comprises unmethylated cytidine-phosphate-guanosine (also referred to as CpG or cytosine-phosphate-guanosine) motif, the RSV antigen and the oligonucleotide being present in the immunogenic composition in an amount effective to stimulate an immune response of a mammalian subject, such as a human subject, to an RSV antigen. TLR9 (CD289) recognizes the unmethylated cytidine-phosphate-guanosine (CpG) motif found in microbial DNA, which can be simulated using a synthesized CpG-ODN-containing oligonucleotide (CpG-ODN). CpG-ODN is known to enhance antibody production and stimulate T helper 1 (Th1) cell responses (Coffman et al. Immunity, 33: 492-503, 2010). The optimal oligonucleotide TLR9 agonist typically contains a palindromic sequence following the general formula: 5'-purine-purine-CG-pyrimidine-pyrimidine-3', or 5'-purine-purine-CG-pyrimidine-CG-3'. US Pat. No. 6,589,940, which is incorporated herein by reference in its entirety. In some embodiments, the CpG oligonucleotide is linear. In other embodiments, the CpG oligonucleotide is circular or comprises a hairpin loop. The CpG oligonucleotide may be single-stranded or double-stranded. In some embodiments, the CpG oligonucleotide may contain a modification. Modifications include, but are not limited to, modification of 3'OH or 5'OH groups, modification of nucleotide bases, modification of sugar components, and modification of phosphate groups. The modified base may be included in the palindromic sequence of the CpG oligonucleotide so long as the modified base remains the same specificity for its natural complement by Watson-Crick base pairing (e.g. the palindromic portion is still itself complementary). In some embodiments, the CpG oligonucleotide comprises a non-typical base. In some embodiments, the CpG oligonucleotide comprises a modified nucleoside. In some embodiments, the modified nucleoside is selected from the group consisting of 2'-deoxy-7-deazaguanosine, 2'-deoxy-6-thioguanosine, arabinosyl guanosine, 2'-deoxy-2'-substituted arabinosyl guanosine and 2'-O-substituted arabinosyl guanosine. The CpG oligonucleotide may contain a modification of a phosphate group. For example, in addition to phosphodiester bonds, phosphate modifications include, but are not limited to, methyl phosphonate, phosphorothioate, phosphoramide (bridged or non-bridged), phosphotriester, and dithiophosphate, and may be used in any combination. Other non-phosphate linkages may also be used. In some embodiments, the oligonucleotide comprises only a phosphorothioate backbone. In some embodiments, the oligonucleotide comprises only a phosphodiester backbone. In some embodiments, the oligonucleotide comprises a combination of phosphate linkages in a phosphate backbone, such as a combination of a phosphodiester bond and a phosphorothioate linkage. Oligonucleotides having a phosphorothioate backbone may be more immunogenic than oligonucleotides having a phosphodiester backbone, appear more resistant to degradation after injection into a host body (Braun et al. J Immunol, 141: 2084-2089, 1988; and Latimer et al. Mol Immunol, 32: 1057-1064, 1995). The CpG oligonucleotides of the present disclosure comprise at least one, two or three phosphorothioate linkages. In some embodiments, both stereoisomers of the phosphorothioate linkage are present in the plurality of CpG oligonucleotide molecules when the plurality of CpG oligonucleotide molecules are present in the pharmaceutical composition comprising at least one excipient. In some embodiments, all nucleotides of the CpG oligonucleotide are phosphorothioate linkages, or in other words, the CpG oligonucleotide has a phosphorothioate backbone.

Any suitable CpG oligodeoxynucleotide (ODN) or a combination thereof can be used as an adjuvant in the present disclosure. For example, a K-type ODN (also referred to as B-type) contains a plurality of CpG motifs on a phosphorothioate backbone. The K-type ODN may be based on the following sequence: TCC**ATGGA**CG**TTCCT**GAGCGTT. The use of phosphorothioate nucleotides enhances resistance to nuclease digestion compared to natural phosphodiester nucleotides, resulting in a substantially longer in vivo half-life. The K-type ODN initiates pDC differentiation and produces TNF-α, and initiates B cell proliferation and secretion of IgM. The D-type ODN (also known as A-type) is constructed from a mixed phosphodiester/phosphorothioate backbone, contains a single CpG motif flanked by palindromic sequences, and has a poly (G) tail at the 3' and 5' ends (a structural motif that facilitates the formation of multimer formation). The D-type ODN may be based on the following sequence: GGTGCATCGATGCAGGGGG. The D-type ODN initiates pDC maturation and secretion of IFN-α but has no effect on B cells. The C-type ODN is similar to the K-type in that it is entirely composed of phosphorothioate nucleotides but are similar to the D-type in that it contains a CpG motif within a palindromic context. The C-type ODN may be based on the following sequence: TCG**T**CG**TT**CG**AA**CG**A**CGTTGAT. The C-type ODN stimulates B cells to secrete IL-6 and stimulates pDC to produce IFN-α. The P-type ODN contains two palindromic sequences so that they can form a more highly ordered structure. The P-type ODN may be based on the following sequence: **T**CG**T**CG**A**CG**AT**CG**G**CGCGCG**C**CG. The P-type ODN activates B cells and pDC and induces substantially higher IFN-α production compared to the C-type ODN. In this paragraph, the bold letters in the ODN sequence indicate self-complementary palindromic sequences, and the CpG motif is underlined.

Exemplary CpG ODN such as CpG 7909(5'-TCGTCGTTTTGTCGTTTTGTCGTT-3') and CpG 1018(5'-TGACTGTGAACGTTCGAGATGA-3') are known and are disclosed in US Pat. Nos. 7,255,868; 7,491,706; 7,479,285; 7,745,598; 7,785,610; 8,003,115; 8,133,874,8,114,418; 8,222,398; 8,333,980; 8,597,665; 8,669,237; 9,028,845; and 10,052,378; Application Publication US 2020/0002704; and Bode et al. "CpG DNA as a vaccine adjuvant," Expert Rev. Vaccines (2011), 10(4): 499-511, all of which are incorporated herein by reference in their entirety for all purposes.

One or more adjuvants may be used in combination and may include, but are not limited to, aluminum hydroxide (aluminum salt), oil-in-water emulsions, water-in-oil emulsions, liposomes, and microparticles, such as poly (lactide-co-glycolide) particles (Shah et al. Methods Mol, 1494: 1-14, 2017). In some embodiments, the immunogenic composition further comprises an aluminum salt adjuvant that adsorbs the RSV antigen. In some embodiments, the aluminum salt adjuvant comprises one or more of amorphous aluminum hydroxyphosphate, aluminum hydroxide, aluminum phosphate, and potassium aluminum sulfate. In some embodiments, the aluminum salt adjuvant comprises one or both aluminum hydroxide and aluminum phosphate. In some embodiments, the aluminum salt adjuvant comprises aluminum hydroxide. In some embodiments, the unit dose of the immunogenic composition comprises from about 0.25 to about 0.50 mg Al³⁺ or about 0.35 mg Al³⁺. In some embodiments, the immunogenic composition further comprises other adjuvants. Other suitable adjuvants include, but are not limited to, squalene-in-water emulsions (e.g. MF59 or AS03), TLR3 agonists (e.g. poly IC or poly ICLC), TLR4 agonists (e.g. bacterial lipopolysaccharide derivatives such as monophosphoryl lipid A (MPL) and/or saponins such as Quil A or QS -21, such as AS01 or AS02), TLR5 agonists (bacterial flagellin), and TLR7, TLR8 and/or TLR9 agonists (imidazoquinoline derivatives such as imiquimod and resiquimod) (Coffman et al. Immunity, 33: 492-503, 2010). In some embodiments, other adjuvants include MPL and an aluminum agent (e.g.AS04). For veterinary uses and for the production of antibodies in non-human animals, Freund's adjuvant (full and incomplete) can be used.

In some embodiments, the immunogenic composition comprises a pharmaceutically acceptable excipient, including, for example, a solvent, an extender, a buffering agent, a tonicity adjusting agent, and a preservative (Pramanick et al. Pharma Times, 45: 65-77, 2013). In some embodiments, the immunogenic composition may comprise an excipient that functions as one or more of a solvent, an extender, a buffering agent, and a tonicity adjusting agent (e.g. sodium chloride in salt water may be simultaneously used as an aqueous vehicle and a tonicity adjusting agent).

In some embodiments, the immunogenic composition comprises an aqueous vehicle as a solvent. Suitable media include, for example, sterile water, saline solution, phosphate buffered saline, and Ringer's solution. In some embodiments, the composition is isotonic.

The immunogenic composition may include a buffer. The buffer controls the pH to inhibit degradation of the active agent during processing, storage, and optional reconstitution. Suitable buffers include, for example, salts, including acetate, citrate, phosphate, or sulfate. Other suitable buffers include, for example, amino acids such as arginine, glycine, histidine, and lysine. The buffer may also include hydrochloric acid or sodium hydroxide. In some embodiments, the buffer maintains a pH of the composition in a range of 6 to 9. In some embodiments, the pH is greater than (lower limit) 6, 7, or 8 in some embodiments, the pH is less than (upper limit) 9, 8, or 7, i.e. the pH is in the range of about 6 to 9, with the lower limit being less than the upper limit.

The immunogenic composition may include a tonicity adjusting agent. Suitable tonicity adjusting agents include, for example, dextrose, glycerol, sodium chloride, glycerin, and mannitol.

The immunogenic composition may include a bulking agent. a bulking agent is particularly useful when the pharmaceutical composition is lyophilized prior to administration. In some embodiments, bulking agent functions as a protectant that helps stabilize and prevent degradation of the active agent during freezing or spray drying and/or during storage. Suitable bulking agents are sugars (monosaccharides, disaccharides, and polysaccharides), such as sucrose, lactose, trehalose, mannitol, sorbitol, glucose, and raffinose.

The immunogenic composition may include a preservative. Suitable preservatives include, for example, antioxidants and antimicrobial agents. In a preferred embodiment, however, the immunogenic composition is prepared under sterile conditions and in a single-dose container and thus need not include preservatives.

In some embodiments, the composition may be provided as a sterile composition. The pharmaceutical composition typically contains an effective amount of the disclosed immunogen and can be prepared by conventional methods. Typically, the amount of immunogen in each immunogenic composition is selected to induce an immune response without significant adverse side effects. In some embodiments, the composition may be provided in a unit dosage form for inducing an immune response in a subject. The unit dosage form contains a suitable single preselected dose to be administered to a subject, or multiple portions of two or more preselected unit doses that are appropriately labeled or measured, and/or a dosing mechanism for administering the unit dose or multiple unit doses. In other embodiments, the composition further includes an adjuvant.

### IV. METHOD FOR INDUCING IMMUNE RESPONSE

In some embodiments, provided herein is a method of producing an immune response to an RSV surface antigen in a subject, the method comprising administering to the subject an effective amount of a complex comprising a recombinant polypeptide selected from the group consisting of SEQ ID NOs: 1-16, 64-71. In some embodiments, provided herein is a method of producing an immune response to an RSV surface antigen in a subject, wherein the surface antigen comprises an F protein or an antigen fragment thereof, the method comprising administering to the subject an effective amount of a complex comprising a recombinant polypeptide selected from the group consisting of SEQ ID NOs: 1-16, 64-71. In some embodiments, provided herein is a method of producing an immune response to an RSV surface antigen in a subject, wherein the surface antigen comprises a sequence selected from SEQ ID NOs: 17-47 and 72-79, the method comprising administering to the subject an effective amount of a complex comprising a recombinant polypeptide selected from the group consisting of SEQ ID NOs: 1-16 and 64-71. In some embodiments, provided herein is a method of producing an immune response to an RSV surface antigen in a subject, wherein the surface antigen comprises an F protein of RSV or an antigen fragment thereof, optionally, the surface antigen comprises a sequence of any one or more of 17-47 and 72-79 or an antigen fragment thereof, the method comprising administering to the subject an effective amount of a complex comprising a recombinant polypeptide comprising the sequence of any one of SEQ ID NOs: 1-16 and 64-71.

In some embodiments, provided herein is a method of producing an immune response to an RSV surface antigen in a subject, wherein the surface antigen comprises an F protein or an antigen fragment thereof, the method comprising administering to the subject an effective amount of a complex comprising a recombinant polypeptide comprising a sequence selected from SEQ ID NOs: 1-16 or a combination of any two or more of such complexes.

The disclosed immunogens (e.g. recombinant RSV F antigens, such as trimers, proteins, nucleic acid molecules (e.g. RNA molecules) or vectors encoding a protomer of the disclosed recombinant RSV F antigen, or protein nanoparticles or virus-like particles comprising the disclosed recombinant RSV F antigen) can be administered to a subject to induce an immune response to the respective RSV F antigen in the subject. In one particular example, the subject is a human subject. The immune response may be a protective immune response, such as a response to inhibit a subsequent infection of a corresponding RSV. The induced immune response may also be used to treat or inhibit infections and diseases associated with the respective RSV.

In some embodiments, a subject who has an RSV infection or is at risk of RSV infection (e.g., due to exposure or potential exposure to RSV) may be selected for treatment. Upon administration of the disclosed immunogen, the subject's infection status and/or symptoms associated with RSV may be monitored.

Typical subjects intended to be treated using the therapeutic agents and methods disclosed herein include humans, as well as non-human primates and other animals. To identify subjects to be prevented or treated according to the methods of the present disclosure, accepted screening methods are employed to determine risk factors associated with a target or suspected disease or disorder, or to determine the status of an existing disease or disorder in a subject. These screening methods include, for example, assessing environmental, familial, occupational, and other such risk factors that may be associated with a target or suspected disease or disorder, and methods of diagnosing and/or characterizing RSV infection, such as various ELISA and other immunoassay methods. These and other conventional methods allow clinicians to select patients requiring treatment using the methods and pharmaceutical compositions of the present disclosure. According to these methods and principles, the composition may be administered in accordance with the teachings herein or other conventional methods as a stand-alone prevention or treatment regimen, or as a follow-on, adjunct, or coordinated treatment regimen for other treatments.

The disclosed immunogens, such as RSV F antigens, (e.g., trimers or proteins), can be administered for prophylactic or therapeutic purposes. When administered prophylactically, the disclosed therapeutic agent is provided prior to any symptom, such as prior to infection. The prophylactic administration of the disclosed therapeutic agent is used to prevent or ameliorate any secondary infection. When administered therapeutically, the disclosed therapeutic agent is provided upon or after the onset of a disease or infection symptom, such as after an RSV infection symptom corresponding to the RSV F antigen or after a diagnosis is RSV infection. The therapeutic agent may thus be provided prior to being expected to be exposed to RSV so as to reduce the anticipated severity, duration, or extent of infection and/or associated disease symptoms after exposure or suspected exposure to the virus or upon actual onset of infection.

The immunogens described herein and their immunogenic compositions are provided to the subject in an amount effective to induce or enhance the immune response in a subject, preferably a human, to the RSV F antigen. The actual dose of the disclosed immunogens will vary according to a number of factors, such as disease indications and specific conditions of the subject (e.g. age, body shape, health condition, symptom severity, susceptibility factors, etc. of the subject), administration timing and route, other drugs or treatments that are administered in parallel, and specific pharmacology of the composition for eliciting a desired activity or biological response in a subject. The dosage regimen may be adjusted to provide an optimal prophylactic or therapeutic response.

Immunogenic compositions comprising one or more of the disclosed immunogens may be used to coordinate (or prime-boost) a vaccination protocol or a combination formulation. In certain embodiments, the novel combined immunogenic composition and a coordinated immunization protocol employ separate immunogens or formulations, each directed to eliciting an antiviral immune response, such as an immune response to RSV F antigen. The individual immunogenic compositions that elicit an antiviral immune response may be combined in a multivalent immunogenic composition administered to a subject in a single immunization step, or they can be administered separately (in a monovalent immunogenic composition) in a coordinated (or prime-boost) immunization regimen.

Several boosts may be made, and each boost may be a different disclosed immunogen. In certain examples, the boost may be the same immunogen as another boost or prime immunization. The prime and booster doses may be administered as a single or multi-agent, for example two, three, four, five, six or more agents may be administered to the subject over days, weeks, or months. Multiple boosts may also be made, such as 1 to 5 times (e.g. 1, 2, 3, 4, or 5 times) or more. Different doses may be used in a series of sequential immunizations. For example, a relatively large dose is used in the first immunization, and a relatively small dose is then used in the boost.

In certain embodiments, the boost may be administered at about 2 weeks post-prime, about 3 to 8 weeks, or about 4 weeks post-prime, or about several months post-prime. In certain embodiments, the boost may be administered at a time of about 5, about 6, about 7, about 8, about 10, about 12, about 18, about 24 months, or more or less post-prime. Regular extra boost may also be used at a suitable point in time to enhance the "immune memory" of the subject. Selected vaccination parameters such as formulation, dose, schedule, etc. can be determined by acquiring aliquots of serum from the subject during an immunization schedule and determining antibody titers. In addition, clinical conditions of the subject may be monitored to find a desired effect, such as an improvement in infection prevention or disease status (e.g. a decrease in viral load). If such monitoring indicates that vaccination is suboptimal, the subject may be boosted with additional immunogenic composition doses, and the vaccination parameters may be modified in a manner that is expected to enhance the immune response.

In certain embodiments, the prime-boost method may include providing the subject with a DNA prime immunization and a protein booster vaccination regimen. The method may include two or more administrations of nucleic acid molecules or proteins.

For protein therapeutics, in general, each human dose includes 1-1000 µg of protein, such as about 1 µg to about 100 µg, such as about 1 µg to about 50 µg, such as about 1 µg, about 2 µg, about 5 µg, about 10 µg, about 15 µg, about 20 µg, about 25 µg, about 30 µg, about 40 µg, or about 50 µg.

The amount utilized in the immunogenic composition is selected based on a population of subjects, such as an infant or elderly person. By standard studies, including observing the antibody titer and other responses of the subject, an optimal dose of the particular composition can be determined. It is understood that the therapeutically effective amount of the disclosed immunogen in the immunogenic composition, such as the disclosed recombinant RSV F antigen (e.g., trimer, protein), viral vector, or nucleic acid molecule, may include an amount that is ineffective in evoking an immune response with a single dose but effective with multiple doses, for example in a prime-boost regimen.

After administration of the immunogen disclosed herein, the immune system of a subject typically responds to the immunogenic composition by producing antibodies specific to the RSV F protein peptide included in the immunogen. Such responses indicate hat an immunologically effective dose has been delivered to the subject.

In some embodiments, the antibody response of the subject is determined in the context of evaluating an effective dosage and/or a vaccination regimen. In most cases, evaluating the antibody titer in serum or plasma obtained from the subject is sufficient. The decision as to whether to administer a booster immunization and/or to adjust the amount of therapeutic agent administered to an individual may be based at least in part on the antibody titer levels. Antibody titer levels may be determined using, for example, immunobinding assays that measure antibody concentrations in serum that bind to antigens, including, for example, recombinant RSV F antigens, such as trimers, or proteins.

There is no need to completely eliminate or reduce or prevent RSV infection for the method to be considered effective. For example, compared to RSV infection in the absence of an immunogen, the amount required to reduce or suppress RSV infection by inducing an immune response to RSV with one or more disclosed immunogens is, for example, at least 10%, at least 20%, at least 50%, at least 60%, at least 70%, at least 80%, at least 90%, at least 95%, at least 98%, or even at least 100% (eliminating or preventing detectable infected cells). In other embodiments, RSV replication may be reduced or inhibited by the disclosed methods. There is no need to completely eliminate RSV replication for the method to be considered effective. For example, compared to RSV replication in the absence of an immunogen, an immune response induced by one or more disclosed immunogens can reduce the replication of the corresponding RSV by the required amount, such as at least 10%, at least 20%, at least 50%, at least 60%, at least 70%, at least 80%, at least 90%, at least 95%, at least 98%, or even at least 100% (eliminating or preventing detectable RSV replication).

In some embodiments, the disclosed immunogens are administered to a subject concurrently with administration of an adjuvant. In other embodiments, the disclosed immunogen is administered to a subject within a period of time after administration of the adjuvant that is sufficient to induce an immune response.

A method for administering nucleic acids is the use of plasmid DNA, such as by directly immunizing with a mammalian expression plasmid DNA. Immunization by nucleic acid constructs is well known in the art and taught, for example, in US Pat. No. 5,643,578 (describing methods for eliciting a cell-mediated or humoral immune response to immunize a vertebrate by introducing DNA encoding a desired antigen) and US Pat. Nos. 5,593,972 and 5,817,637 (describing a regulatory sequence operably linked to a nucleic acid sequence encoding an antigen and enabling it to be expressed). US Pat. No. 5,880,103 describes several methods of delivering nucleic acids encoding immunogenic peptides or other antigens to an organism. The method comprises liposomal delivery of nucleic acids (or of the synthetic peptide itself) and an immunostimulatory construct or ISCOMS^{™}, i.e. a negatively charged cage-like structure of 30-40 nanometer size spontaneously formed spontaneously upon mixing cholesterol and Quil A^{™} (a saponin). In various infection experimental models, including toxoplasmosis and Epstein-Barr virus-induced tumors, a protective immunity (Mowat and Donachie, Immunol) has been produced using the ISCOMS^{™} as an antigen delivery vehicle. Today 12: 383, 1991). As little as 1 µg of antigen doses encapsulated in ISCOMS^{™} have been found to produce Class I-mediated CTL responses (Takahashi et al. Nature 344: 873, 1990).

In some embodiments, plasmid DNA vaccines are used to express the disclosed immunogens in a subject. For example, nucleic acid molecules encoding the disclosed immunogens may be administered to a subject to induce an immune response to the RSV F antigen. In some embodiments, the nucleic acid molecule is incorporated into a plasmid vector such as pVRC8400 vector for DNA immunization (described in Barouch et al. J Virol, 79, 8828-8834, 2005, which is incorporated herein by reference).

In another method of immunization using nucleic acids, the disclosed recombinant RSV F antigen, such as trimers, proteins, can be expressed using attenuated viral hosts or vectors, or bacterial vectors. Recombinant vaccinia virus, adeno-associated virus (AAV), herpes virus, retrovirus, cytomegalovirus or other viral vectors can be used to express the peptides or proteins, thereby inducing CTL responses. For example, US Pat. No. 4,722,848 describes vaccinia virus vectors and methods that can be used in immunization schemes. BCG (Bacille Calmette-Guérin) provides another vector for expressing the peptides (see Stover, Nature 351: 456-460, 1991).

In one embodiment, a nucleic acid encoding the disclosed recombinant RSV F antigen is introduced directly into cells. For example, nucleic acids can be loaded onto gold microspheres by standard methods and introduced into the skin through a device such as a Bio-Rad HELIOS^{™} gene gun. The nucleic acid can be "bare" and comprises plasmids under the control of strong promoters. Typically, DNA is injected into the muscle, but it can also be injected directly into other sites. The injection dose is typically from about 0.5 µg/kg to about 50 mg/kg, and more typically from about 0.005 mg/kg to about 5 mg/kg (see, e.g. US Pat. No. 5,589,466).

For example, nucleic acids can be loaded onto gold microspheres by standard methods and introduced into the skin through a device such as a Bio-Rad HELIOS^{™} gene gun. The nucleic acid can be "bare" and comprises plasmids under the control of strong promoters. Typically, DNA is injected into the muscle, but it can also be injected directly into other sites. The injection dose is typically from about 0.5 µg/kg to about 50 mg/kg, and more typically from about 0.005 mg/kg to about 5 mg/kg (see, e.g. US Pat. No. 5,589,466).

In another embodiment, mRNA-based immunization protocols can be used to deliver nucleic acids encoding the disclosed recombinant RSV F antigen directly into cells. In some embodiments, an mRNA-based nucleic acid vaccine may provide an effective alternative to the foregoing method. The mRNA vaccine avoids safety issues with DNA integration into the host genome and can be translated directly in the host cytoplasm. In addition, simple cell-free synthesis of RNA avoids manufacturing complexity associated with viral vectors. Two exemplary forms of RNA-based vaccination that can be used to deliver nucleic acids encoding the disclosed recombinant RSV F antigen include conventional non-replicating mRNA immunization (see, e.g. Petsch et al. "Protective efficacy of specific mRNA vaccines synthesized in vitro against HIV virus infection" (Protective efficacy of in vivo synthetic, specific mRNA vaccines, Nature biotechnology, 30(12): 1210-6, 2012) and self-amplifying mRNA immunization (see, e.g. Geall et al. Nonviral delivery of self-amplified RNA vaccines, PNAS, 109(36): 14604-14609, 2012; Magini et al. "Self-Amplifying mRNA Vaccine Expression Multiple Condensed mRNA Vaccines", PLoS One, 11(8): e0161193, 2016; and Brito et al. "Self-amplified mRNA vaccine", Adv Genet., 89: 179-233, 2015).

In some embodiments, administering a therapeutically effective dose of one or more disclosed immunogens to a subject induces neutralizing immune responses in the subject. To assess the neutralization activity, serum can be collected from the subject at an appropriate point in time after vaccination and then frozen and stored for neutralization testing. Methods for determining neutralization activity are known to those of ordinary skill in the art and further described herein, including, but not limited to, plaque reduction neutralization (PRNT) assays, microneutralization assays, flow cytometry-based assays, single-cycle infection assays. In some embodiments, a set of RSV pseudoviruses may be used to determine serum neutralization activity.

In some embodiments, administering a therapeutically effective dose of one or more disclosed immunogens to a subject induces neutralizing immune responses in the subject. To assess the neutralization activity, serum can be collected from the subject at an appropriate point in time after vaccination and then frozen and stored for neutralization testing. Methods for determining neutralization activity are known to those of ordinary skill in the art and further described herein, including, but not limited to, plaque reduction neutralization (PRNT) assays, microneutralization assays, flow cytometry-based assays, single-cycle infection assays. In some embodiments, a set of RSV pseudoviruses may be used to determine serum neutralization activity.

In some embodiments, neutralizing antibodies directed against RSV are produced by immunogen-induced neutralizing immune responses disclosed herein. In some embodiments, the neutralizing antibody herein binds to a cell receptor or an auxiliary receptor of RSV or a component thereof. The nucleolin is an entry co-receptor of RSV, and also mediates cellular entry of influenza virus, parainfluenza virus, some enterovirus, and bacteria that cause tularemia. The combination of the prefusion RSV-F glycoprotein with an insulin-like growth factor 1 receptor (IGF1R) may also trigger activation of protein kinase C ζ (PKC ζ) to recruit nucleolin from the nucleus to the cytoplasmic membrane to bind RSV-F on the virus body. In some embodiments, the viral receptor or auxiliary receptor is a paramyxovirus receptor or an auxiliary receptor, preferably a pneumovirus receptor or an auxiliary receptor, more preferably a human RSV receptor or an auxiliary receptor. For example, CCR1, CCR2, CCR3, CCR4, CCR5, and/or CCR8 receptors may be involved in human RSV infection. RhoA is another example of a host cell RSV receptor or an auxiliary receptor. In some embodiments, neutralizing antibodies herein regulate, reduce, antagonize, alleviate, block, inhibit, eliminate, and/or interfere with at least one RSV activity or binding or RSV receptor activity or binding, such as RSV release, RSV receptor signaling, membrane RSV cleavage, RSV activity, RSV production, and/or synthesis, *in vitro, in situ,* and/or *in vivo.* In some embodiments, the immunogens disclosed herein induce neutralizing antibodies against RSV that regulate, reduce, antagonize, mitigate, block, inhibit, eliminate, and/or interfere with the binding of RSV to RSV receptor or auxiliary receptor such as nucleolin, IGF1R, CCR1, CCR2, CCR3, CCR4, CCR5, CCR8, and/or RhoA.

### V. Product or Kit

Articles or kits containing the provided recombinant polypeptides, proteins, and immunogenic compositions are also provided. The article may include a container and a label or package insert on or associated with the container. Suitable containers include, for example, bottles, vials, syringes, test tubes, IV infusion bags, and the like. The container may be formed of a variety of materials such as glass or plastic. In some embodiments, the container has a sterile access port. Exemplary containers include intravenous infusion bags, vials, including those having plugs that can be pierced by an injection needle. The article or kit may further include a package insert indicating that the composition may be used to treat a particular condition, such as a disorder (e.g. RSV infection) as described herein. Alternatively, or additionally, the article or kit may further comprise another or the same container comprising a pharmaceutically acceptable buffer. It may also include other materials such as other buffers, diluents, filters, needles, and/or syringes.

The label or package insert may indicate that the composition is used to treat RSV infection of an individual. A label or package insert on the container or with the container may indicate guidance regarding formulation reconstitution and/or use. The tag or package insert may also indicate that the formulation may be used or intended for subcutaneous, intravenous, or other modes of administration for treating or preventing RSV infection in an individual.

In some embodiments, the container contains the composition alone or in combination with another composition that can effectively treat, prevent, and/or diagnose the condition. The article or kit may include (a) a first container containing a composition (i.e., a first drug), wherein the composition comprises the immunogenic composition or its protein or recombinant polypeptide; and (b) a second container containing a composition (i.e., a second drug), wherein the composition comprises other agents, such as adjuvants or other therapeutic agents. The article or kit may also include instructions on the label or packaging insert regarding the treatment of the subject with the second drug in an effective amount.

### Terminology

Unless otherwise defined, all special terms, symbols, and other technical and scientific terms used herein have the same meaning as commonly understood by one of ordinary skill in the art to which the claimed subject matter belongs. In some instances, terms having commonly understood meanings are defined herein for clarity and/or ease of reference, and the inclusion of such definitions herein is not necessarily to be construed as a substantial difference from what is generally understood in the art.

The terms "polypeptide" and "protein" are interchangeable to refer to amino acid residue polymers and do not limit the minimum length. Polypeptides, including the receptors provided herein and other polypeptides, such as linkers or peptides, may include amino acid residues, including natural and/or non-natural amino acid residues. The term also includes post-translational modification of the polypeptide, such as glycosylation, sialylation, acetylation, and phosphorylation. In some aspects, the polypeptide may contain modifications to the parent or native sequence as long as the protein retains the desired activity. These modifications may be intentional, such as by site-directed mutagenesis, or incidental, such as mutations arising in the host producing the protein or errors due to PCR amplification.

As used herein, a "subject" is a mammal, such as a human or other animal, typically a human. In some embodiments, a subject, such as a patient, administered one or more medicants, cells, cell populations, or compositions is a mammal, typically a primate, such as a human. In some embodiments, the primate is a monkey or an ape. The subject may be a male or female and may be any suitable age, including infants, children, adolescents, adults and elderly subjects. In some embodiments, the subject is a non-primate mammal, such as a rodent.

As used herein, "treating" (and grammatical variations thereof) refers to a complete or partial improvement or alleviation of a disease or disorder or condition, or symptoms, adverse reactions, or outcomes associated therewith, or a phenotype. The desired effects of treatment include, but are not limited to, preventing disease occurrence or recurrence, relieving symptoms, reducing any direct or indirect pathological consequences of diseases, preventing metastasis, reducing the rate of disease progression, improving or alleviating disease states, and alleviating or improving prognosis. The term does not mean to completely cure the disease or completely eliminate any symptoms or to be effective for all symptoms or consequences.

As used herein, "delaying the development of a disease" means delaying, obstructing, slowing, retarding, stabilizing, inhibiting, and/or postponing the development of a disease, such as cancer. The duration of such delay may be different depending on the disease history and/or the individual being treated. In some embodiments, a sufficient or significant delay may actually constitute prevention because the individual does not develop the disease. For example, advanced cancer, such as the metastasis, may be delayed.

As used herein, "preventing" includes preventing the occurrence or recurrence of a subject that may be susceptible to a disease but has not yet been diagnosed with a disease. In some embodiments, the provided cells and compositions are used to delay the development of a disease or to slow the progression of a disease.

As used herein, "inhibiting" a function or activity means reducing a function or activity relative to an otherwise identical condition lacking the target condition or parameter. For example, cells that inhibit tumor growth cause reduced tumor growth rates compared to tumor growth rates in the absence of the cells.

In the context of administration, an "effective amount" of a medicament, such as a pharmaceutical formulation, cell or composition, refers to a dose and/or quantity, and regimen necessary to effectively achieve a desired result, such as a therapeutic or prophylactic result.

A "therapeutically effective amount" of a medicament, such as a pharmaceutical formulation, cell or composition, refers to a dose and regimen necessary to effectively achieve the desired therapeutic outcome, such as treating a disease, disorder, or condition and/or producing a desired pharmacokinetic or pharmacodynamic effect. The therapeutically effective amount may vary depending on factors such as disease status, age, gender and weight of the subject, and population of cells administered. In some embodiments, the methods provided include administering the cells and/or compositions in an effective amount (e.g. a therapeutically effective amount).

"Prophylactically effective amount" refers to a dose and duration necessary to effectively achieve a desired prevention result. Typically, though not required, the prophylactically effective amount is less than the therapeutically effective amount because the prophylactic dose is used in the subject before or early stages of the disease. In the event that the tumor burden is low, the prophylactically effective amount of some aspects will be higher than the therapeutically effective amount.

As used herein, the term "about" refers to the range of variation that would be understood by one of ordinary skill in the art. Reference herein to "about" a value or parameter includes (and describes) an implementation for that value or parameter itself.

As used herein, the singular forms "a", "an", and "the" include plural referents unless the context clearly dictates otherwise. For example, "a" or "an" means "at least one" or " one or more".

Various aspects of the claimed subject matter are presented in the form of ranges throughout this disclosure. It is to be understood that the description of such ranges is provided merely for convenience and brevity and should not be regarded as a rigid limitation on the scope of the claimed subject matter. Accordingly, the description of ranges should be taken as having explicitly disclosed all possible subranges as well as individual numerical values within that range. For example, where a range of values is provided, it should be understood that each intervening value between the upper and lower limits of the range, and any other stated or intervening value within that range, are encompassed within the claimed subject matter. The upper and lower limits of these smaller ranges may be independently included within a smaller range, and are likewise encompassed within the claimed subject matter, subject only to any explicit exclusions of the stated range. Where a range includes one or more endpoints, ranges excluding one or both of those endpoints are also encompassed within the claimed subject matter. This applies to any range width.

As used herein, a composition refers to any mixture of two or more products, substances, or compounds, including cells. It can be a solution, a suspension, a liquid, a powder, a paste, an aqueous solution, a non-aqueous solution, or any combination thereof.

As used herein, the term "vector" refers to a nucleic acid molecule capable of replicating a nucleic acid sequence joined thereto. The term comprises a vector as a vector for self-replicating a nucleic acid structure and a vector incorporated into the genome of a host cell into which it has been introduced. Certain vectors are capable of directing the expression of nucleic acids operably linked to them. Such vectors are referred to herein as "expression vectors".

### Exemplary Embodiments

Embodiment 1: A protein comprising a plurality of recombinant polypeptides, each recombinant polypeptide comprising a respiratory syncytial virus (RSV) F protein peptide or a fragment or epitope thereof linked to a C-terminal propeptide of collagen, wherein the C-terminal propeptide of the recombinant polypeptide forms an inter-polypeptide disulfide bond.

Embodiment 2: The protein according to Embodiment 1, wherein the RSV belongs to subtype A or subtype B.

Embodiment 3: The protein of Embodiment 1 or 2, wherein the epitope is a linear epitope or a conformational epitope.

Embodiment 4: The protein according to any one of embodiments 1 to 3, wherein the F protein peptide comprises an F1 subunit peptide, an F2 subunit peptide, or any combination thereof, and the protein comprises three recombinant polypeptides.

Embodiment 5: The protein according to any one of embodiments 1 to 4, wherein the F protein peptide comprises a signal peptide, a heptad repeat sequence C (HRC) peptide, a pep27 peptide, a fusion peptide (FP), a heptad repeat sequence A (HRA) peptide, a domain I peptide, a domain II peptide, or a heptad repeat sequence B (HRB) peptide, or any combination thereof.

Embodiment 6: The protein according to any one of embodiments 1 to 5, wherein the F protein peptide comprises an F1 subunit of the F protein but does not contain F2 subunit, or vice versa.

Embodiment 7: The protein of any one of Embodiments 1 to 6, wherein the F protein peptide comprises F1 subunit and F2 subunit of the F protein, optionally no pep27 is present, optionally, the F1 subunit and the F2 subunit are linked by a disulfide bond or an artificially introduced linker.

Embodiment 8: The protein according to any one of embodiments 1 to 7, wherein the F protein peptide does not comprise a transmembrane (TM) domain peptide and/or a cytoplasmic (CP) domain peptide.

Embodiment 9: The protein according to any one of embodiments 1 to 8, wherein the F protein peptide comprises a protease cleavage site, wherein the protease is optionally furin protease, trypsin, factor Xa, or cathepsin L.

Embodiment 10: The protein according to any one of embodiments 1 to 9, wherein the F protein peptide does not comprise a protease cleavage site, wherein the protease is optionally furin protease, trypsin, factor Xa, or cathepsin L.

Embodiment 11: The protein of any one of Embodiments 1 to 10, wherein the F protein peptide is soluble or not directly associated with a lipid bilayer, such as a membrane or viral envelope.

Embodiment 12: The protein according to any one of embodiments 1 to 11, wherein the F protein peptide is the same or different between the recombinant polypeptides of the protein.

Embodiment 13: The protein according to any one of embodiments 1 to 12, wherein the F protein peptide is directly fused to the C-terminal propeptide, or is linked to the C-terminal propeptide by a linker such as a linker comprising a Gly-X-Y repeat sequence, wherein X and Y are independently any amino acid, optionally proline or hydroxyproline.

Embodiment 14: The protein of any one of Embodiments 1 to 13, the protein being soluble or not directly associated with a lipid bilayer, such as a membrane or viral envelope.

Embodiment 15: The protein according to any one of embodiments 1 to 14, wherein the protein is capable of forming a rosette-like oligomer comprising an F protein peptide trimer.

Embodiment 16: The protein of any one of Embodiments 1 to 15, wherein the protein is capable of binding to a cell-surface attachment factor or receptor of a subject, optionally, the subject is a mammal, such as a primate, such as a human.

Embodiment 17: The protein according to any one of Embodiments 1 to 16, wherein the C-terminal propeptide is derived from human collagen.

Embodiment 18: The protein according to any one of embodiments 1 to 17, wherein the C-terminal propeptide comprises a C-terminal polypeptide or a fragment thereof from proα1 (I), proα1 (II), proα1 (III), proα1 (V), proα1 (XI), proα2 (I), proα2 (V), proα2 (XI), or proα3 (XI).

Embodiment 19: The protein of any one of Embodiments 1 to 18, wherein the C-terminal propeptide is the same or different between the recombinant polypeptides.

Embodiment 20: The protein according to any one of Embodiments 1 to 19, wherein the C-terminal propeptide comprises SEQ ID NO: 48, or comprises an amino acid sequence that has at least 90% identity to SEQ ID NO: 48 and is capable of forming an inter-polypeptide disulfide bond and trimerizing the recombinant polypeptides.

Embodiment 21: The protein according to any one of Embodiments 1 to 19, wherein the C-terminal propeptide comprises SEQ ID NO: 49, or comprises an amino acid sequence having at least 90% identity to SEQ ID NO: 49 and is capable of forming an inter-polypeptide disulfide bond and trimerizing the recombinant polypeptides.

Embodiment 22: The protein according to any one of Embodiments 1 to 19, wherein the C-terminal propeptide comprises SEQ ID NO: 50, or comprises an amino acid sequence that has at least 90% identity to SEQ ID NO: 50 and is capable of forming an inter-polypeptide disulfide bond and trimerizing the recombinant polypeptides.

Embodiment 23: The protein according to any one of Embodiments 1 to 19, wherein the C-terminal propeptide comprises SEQ ID NO: 51, or comprises an amino acid sequence that has at least 90% identity to SEQ ID NO: 51 and is capable of forming an inter-polypeptide disulfide bond and trimerizing the recombinant polypeptides.

Embodiment 24: The protein according to any one of Embodiments 1 to 19, wherein the C-terminal propeptide comprises SEQ ID NO: 52, or comprises an amino acid sequence that has at least 90% identity to SEQ ID NO: 52 and is capable of forming an inter-polypeptide disulfide bond and trimerizing the recombinant polypeptides.

Embodiment 25: The protein according to any one of Embodiments 1 to 19, wherein the C-terminal propeptide comprises SEQ ID NO: 53, or comprises an amino acid sequence that has at least 90% identity to SEQ ID NO: 53 and is capable of forming an inter-polypeptide disulfide bond and trimerizing the recombinant polypeptides.

Embodiment 26: The protein according to any one of Embodiments 1 to 19, wherein the C-terminal propeptide comprises SEQ ID NO: 54, or comprises an amino acid sequence that has at least 90% identity to SEQ ID NO: 54 and is capable of forming an inter-polypeptide disulfide bond and trimerizing the recombinant polypeptides.

Embodiment 27: The protein according to any one of Embodiments 1 to 19, wherein the C-terminal propeptide comprises any one of SEQ ID NOs: 55-59, or comprises an amino acid sequence that has at least 90% identity to any one of SEQ ID NOs: 55-59 and is capable of forming an inter-polypeptide disulfide bond and trimerizing the recombinant polypeptides.

Embodiment 28: The protein according to any one of Embodiments 1 to 19, wherein the C-terminal propeptide comprises SEQ ID NO: 60, or comprises an amino acid sequence that has at least 90% identity to SEQ ID NO: 60 and is capable of forming an inter-polypeptide disulfide bond and trimerizing the recombinant polypeptides.

Embodiment 29: The protein according to any one of Embodiments 1 to 19, wherein the C-terminal propeptide comprises any one of SEQ ID NOs: 61-63, or comprises an amino acid sequence that has at least 90% identity to any one of SEQ ID NOs: 61-63 and is capable of forming an inter-polypeptide disulfide bond and trimerizing the recombinant polypeptides.

Embodiment 30: The protein of any one of Embodiments 1 to 29, wherein the C-terminal propeptide comprises an amino acid sequence comprising a Gly-X-Y repeat sequence linked to the N-terminus of any one of SEQ ID NOs: 48-63, wherein X and Y are independently any amino acid, optionally proline or hydroxyproline, or comprises an amino acid sequence that has at least 90% identity thereto and is capable of forming an inter-polypeptide disulfide bond and trimerizing the recombinant polypeptides.

Embodiment 31: The protein according to any one of embodiments 1 to 30, wherein the F protein peptide in each recombinant polypeptide is in a prefusion conformation or a post-fusion conformation, and optionally, the protein comprises a rosette-like oligomer comprising a crutch-shaped rod-like F protein peptide trimer.

Embodiment 32: The protein according to any one of embodiments 1 to 31, wherein the F protein peptide in each recombinant polypeptide comprises any one of SEQ ID NOs: 72-79 or comprises an amino acid sequence that has at least 80% identity to any one of SEQ ID NOs: 72-79.

Embodiment 33: The protein of any one of Embodiments 1 to 31, wherein the recombinant polypeptide comprises any one of SEQ ID NOs: 76-79 or comprises an amino acid sequence that has at least 80% identity to any one of SEQ ID NOs: 76-79.

Embodiment 34: An immunogen comprising a protein according to any one of Embodiments 1 to 33.

Embodiment 35: A protein nanoparticle comprising a protein according to any one of Embodiments 1 to 33 directly or indirectly linked to a nanoparticle.

Embodiment 36: A virus-like particle (VLP) comprising a protein according to any one of Embodiments 1 to 33.

Embodiment 37: An isolated nucleic acid encoding one, two, three, or more recombinant polypeptides of a protein according to any one of embodiments 1 to 33.

Embodiment 38. The isolated nucleic acid of Embodiment 37, wherein the polypeptide encoding the F protein peptide is fused in-frame with a polypeptide encoding the C-terminal propeptide of collagen.

Embodiment 39: The isolated nucleic acid of Embodiment 37 or 38, the isolated nucleic acid being operably linked to a promoter.

Embodiment 40. The isolated nucleic acid according to any one of embodiments 37 to 39, the isolated nucleic acid being a DNA molecule.

Embodiment 41. The isolated nucleic acid according to any one of embodiments 37 to 39, the isolated nucleic acid being an RNA molecule, optionally an mRNA molecule such as a nucleoside modified mRNA, a non-amplified mRNA, a self-amplifying mRNA, or a trans-amplifying mRNA.

Embodiment 42: A vector comprising the isolated nucleic acid according to any one of embodiments 37 to 41.

Embodiment 43: The vector according to Embodiment 42, the vector being a viral vector.

Embodiment 44: A virus, pseudovirus or cell comprising a vector according to Embodiment 42 or 43, optionally, the virus or cell has a recombinant genome.

Embodiment 45: an immunogenic composition comprising any one or more of a protein, an immunogen, a protein nanoparticle, a VLP, an isolated nucleic acid, a vector, a virus, a pseudovirus, or a cell according to any one of Embodiments 1 to 44, and a pharmaceutically acceptable carrier.

Embodiment 46: a vaccine comprising the immunogenic composition according to Embodiment 45 and optionally an adjuvant, wherein the vaccine is optionally a subunit vaccine, and/or optionally, the vaccine is a prophylactic and/or therapeutic vaccine.

Embodiment 47: The vaccine of Embodiment 46, wherein the vaccine comprises a plurality of different adjuvants.

Embodiment 48: A method of producing a protein, the method comprising: expressing, in a host cell, an isolated nucleic acid or vector according to any one of embodiments 37 to 43 to produce a protein according to any one of embodiments 1 to 33; and purifying the protein.

Embodiment 49: A protein produced according to the method of Embodiment 48.

Embodiment 50: A method of producing an immune response to an F protein peptide of RSV or a fragment or epitope thereof in a subject, the method comprising administering to the subject an effective amount of any of a protein, immunogen, protein nanoparticle, VLP, isolated nucleic acid, vector, virus, pseudovirus, cell, immunogenic composition or vaccine according to any one of embodiments 1 to 47 and 49 to produce the immune response.

Embodiment 51: The method according to Embodiment 50, wherein the method is for treating or preventing RSV infection.

Embodiment 52: The method according to Embodiment 50 or 51, wherein generating the immune response inhibits or reduces replication of RSV in the subject.

Embodiment 53: The method according to any one of embodiments 50 to 52, wherein the immune response comprises a cell-mediated response and/or a humoral response, optionally comprising producing one or more neutralizing antibodies, such as polyclonal antibodies or monoclonal antibodies.

Embodiment 54: The method according to any one of embodiments 50 to 53, wherein the immune response is directed to an F protein peptide of RSV or a fragment or epitope thereof, but not to the C-terminal propeptide.

Embodiment 55: The method according to any one of embodiments 50 to 54, wherein the administering does not cause antibody-dependent enhancement (ADE) due to prior exposure of the subject to one or more RSV.

Embodiment 56: The method according to any of embodiments 50-55, wherein the administering does not cause antibody-dependent enhancement (ADE) when the subject is subsequently exposed to one or more RSV.

Embodiment 57: The method according to any one of embodiments 50 to 56, further comprising a prime and/or a boosting step.

Embodiment 58: The method according to any one of Embodiments 50 to 57, wherein the administration step is performed by local, transdermal, subcutaneous, intradermal, oral, intranasal (e.g. intranasal spray), endotracheal, sublingual, buccal, rectal, vaginal, inhalation, intravenous (e.g. intravenous injection), intra-arterial, intramuscular (e.g. intramuscular injection), intracardiac, intraosseous, intraperitoneal, transmucosal, intravitreal, subretinal, intra-articular, periarticular, topical or epidermal administration.

Embodiment 59: The method according to any one of embodiments 50 to 58, wherein the effective amount is administered as a single dose or in a series of doses separated by one or more intervals.

Embodiment 60: The method according to any one of embodiments 50 to 59, wherein the effective amount is administered without an adjuvant.

Embodiment 61: The method according to any one of embodiments 50 to 59, wherein the effective amount is administered with an adjuvant.

Embodiment 62: A method comprising administering to a subject an effective amount of a protein according to any one of Embodiments 1 to 33 to produce a neutralizing antibody or neutralizing antiserum against RSV in the subject.

Embodiment 63: The method according to Embodiment 62, wherein the subject is a mammal, optionally a human or non-human primate.

Embodiment 64: The method according to Embodiment 62 or 63, further comprising isolating the neutralizing antibody or neutralizing antiserum from the subject.

Embodiment 65: The method according to Embodiment 64, further comprising administering to a human subject an effective amount of isolated neutralizing antibody or neutralizing antiserum by passive immunization to prevent or treat RSV infection.

Embodiment 66: The method according to any one of embodiments 62 to 65, wherein the neutralizing antibody or neutralizing antiserum for RSV comprises a polyclonal antibody against the RSV F protein peptide or fragment or epitope thereof, optionally, the neutralizing antibody or neutralizing antiserum is free or substantially free of antibodies directed to the C-terminal propeptide of collagen.

Embodiment 67: The method according to any one of embodiments 62 to 65, wherein the neutralizing antibody comprises a monoclonal antibody against the RSV F protein peptide or a fragment or epitope thereof, optionally, the neutralizing antibody is free or substantially free of antibodies directed to the C-terminal propeptide of collagen.

Embodiment 68: The protein, immunogen, protein nanoparticle, VLP, isolated nucleic acid, vector, virus, pseudovirus, cell, immunogenic composition or vaccine according to any one of embodiments 1 to 47 and 49, for inducing an immune response to RSV in a subject, and/or for treating or preventing RSV infection.

Embodiment 69: Use of a protein, immunogen, protein nanoparticle, VLP, isolated nucleic acid, vector, virus, pseudovirus, cell, immunogenic composition or vaccine according to any one of embodiments 1 to 47 and 49 for inducing an immune response to RSV in a subject, and/or for treating or preventing RSV infection.

Embodiment 70: Use of the protein, immunogen, protein nanoparticle, VLP, isolated nucleic acid, vector, virus, pseudovirus, cell, immunogenic composition or vaccine according to any one of embodiments 1 to 47 and 49 in the manufacture of a medicament or prophylactic agent for inducing an immune response to RSV in a subject, and/or for treating or preventing RSV infection.

Embodiment 71: A method of analyzing a sample, the method comprising: contacting a sample with a protein according to any one of embodiments 1 to 33, and detecting binding between the protein and an analyte capable of specifically binding to an RSV F protein peptide or a fragment or epitope thereof.

Embodiment 72: The method of embodiment 71, wherein the analyte is an antibody, receptor, or cell that binds to the F protein peptide or fragment or epitope thereof.

Embodiment 73: The method of Embodiment 71 or 72, wherein the binding indicates the presence of the analyte in the sample, and/or the subject from which the sample is derived being infected with RSV.

Embodiment 74: A kit comprising a protein according to any one of Embodiments 1 to 33 and a substrate, pad or vial containing or immobilizing the protein, optionally, the kit is an ELISA or lateral flow assay kit.

### EXAMPLES

The following examples are included for illustrative purposes only and are not intended to limit the scope of the invention.

### Example 1: Recombinant Polypeptide Comprising RSV F Protein Peptide

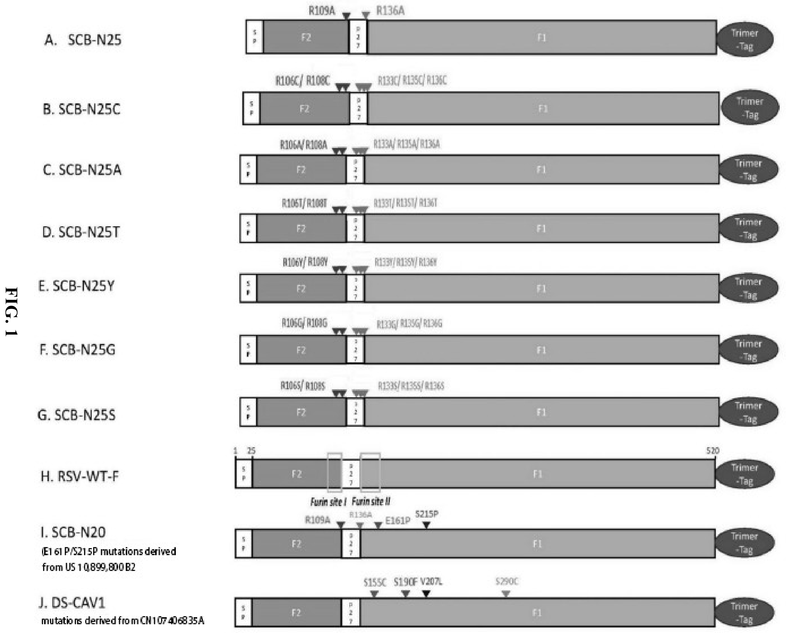

The RSV F glycoprotein construct is derived from RSV A2 strain (accession number AAC55970). The coding sequence corresponding to residues 1-520 of the F protein peptide was codon-optimized, synthesized and subcloned into the mammalian expression vector pTPRIMER-T0D, and the vector encodes the Hind III and Bgl II sites of the human α1 collagen C-propeptide. **FIG. 1** shows a schematic diagram of an exemplary recombinant polypeptide. A: SCB-N25 fusion protein; B: SCB-N25C; C: SCB-N25A; D: SCB-N25T; E: SCB-N25Y; F: SCB-N25G; G: SCB-N25S; H: RSV-WT-F.
SCB-N25: SEQ ID NO: 8
SCB-N20: SEQ ID NO: 12
SCB-N25C: SEQ ID NO: 64
SCB-N25A: SEQ ID NO: 80
SCB-N25T: SEQ ID NO: 81
SCB-N25Y: SEQ ID NO: 82
SCB-N25G: SEQ ID NO: 83
SCB-N25S: SEQ ID NO: 84
RSV-WT-F: SEQ ID NO: 2

### Example 2: Expression of Prefusion F Protein of Recombinant Polypeptide Comprising RSV F Protein Peptide

On day 3, the expression level of prefusion F protein in HEK-293T cell supernatant was determined by measuring the optical density using an ELISA kit (**FIG. 2**). HEK-293T cells were transiently transfected with plasmids carrying the variants indicated on the horizontal (x) axis. After three days, the culture supernatant was harvested and centrifuged to remove cells and cell debris.

293T cells stably passaged in DMEM (10% FBS) were diluted to 0.75 million/mL. 0.5 mL of cells and 1.5 mL of DMEM were seeded into a six-well plate and incubated overnight at 37°C with CO₂. 300 µL of DMEM (-FBS) was added to a 1.5 mL centrifuge tube, followed by 9 µL of FuGene 6. The mixture was left to stand at room temperature for 5 min. 100 µL of the DMEM-FuGene 6 mixture was aliquoted into three separate 1.5 mL centrifuge tubes, and 1 µg of recombinant plasmid was added to each. The mixture was left to stand at room temperature for 20 min. 1 mL of culture medium was removed from each well of the 293T culture plate, and the plasmid-FuGene 6 mixture was added to each well, mixed well, and incubated in an incubator. Three days later, cell supernatants were collected for detecting protein expression. Subsequently, the centrifuged supernatants were analyzed at OD280 using Synagis antibody and AM22 antibody to determine OD values. A: Comparison of high optical density (OD) values among variants; B: Comparison of intermediate OD values among variants. N25C showed higher pre-fusion F protein expression level than other variants. SCB-N20 as disclosed in US 10,899,800 B2 containing the E161P/S215P mutation served as a control.

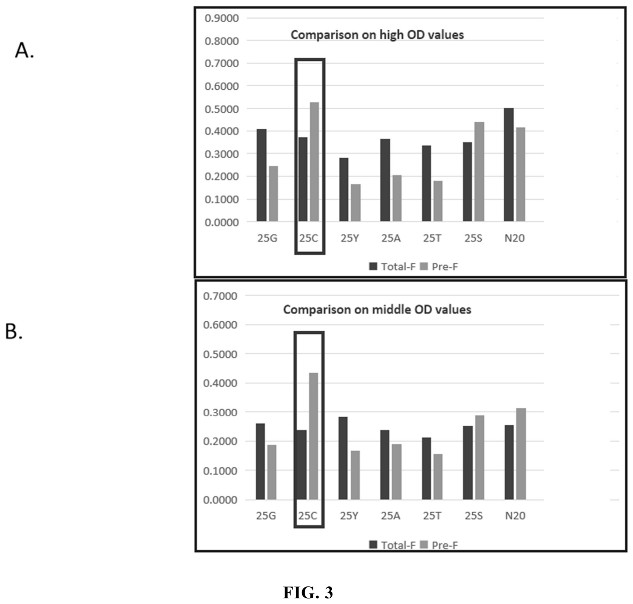

On day 3, the expression level of pre-fusion F protein in CHO cell supernatant was determined by measuring optical density using an ELISA kit (**FIG. 3**). A: Comparison of high optical density (OD) values among variants; B: Comparison of intermediate OD values among variants. N25C showed high expression level of pre-fusion F protein among variants.

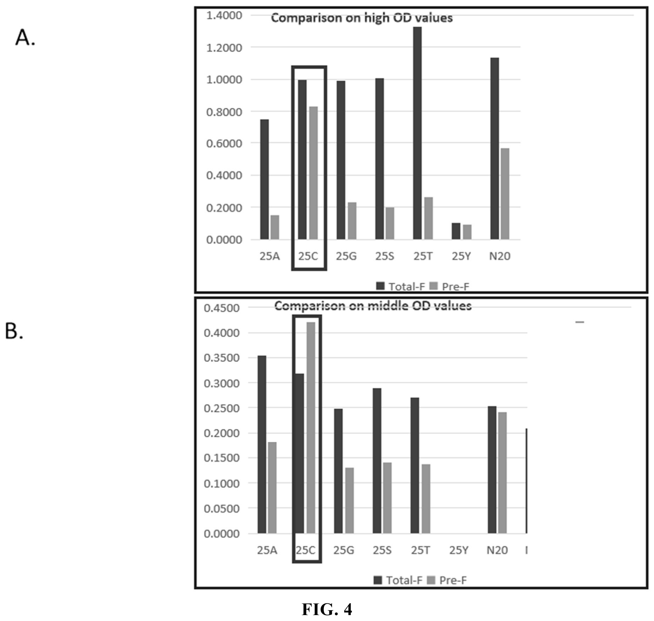

On day 7, the expression level of pre-fusion F protein in CHO cell supernatant was determined by measuring optical density using an ELISA kit (FIG. 4). A: Comparison of high OD values among variants; B: Comparison of intermediate OD values among variants. N25C showed high expression levels of pre-fusion F protein among the variants.

### Example 3: Production of Recombinant Polypeptides Comprising RSV F Protein Peptides

Provided herein were secreted forms of recombinant polypeptides comprising RSV F protein peptides as candidate vaccines.

The RSV F glycoprotein construct was derived from the RSV A2 strain (accession number AAC55970). The coding sequence for residues 1-520 of the F protein peptide was codon-optimized, synthesized, and subcloned into the Hind *III* and Bgl *II* sites of a mammalian expression vector that encodes the human α1 collagen C-propeptide. FIG. 1 shows a schematic diagram of an exemplary recombinant polypeptide.

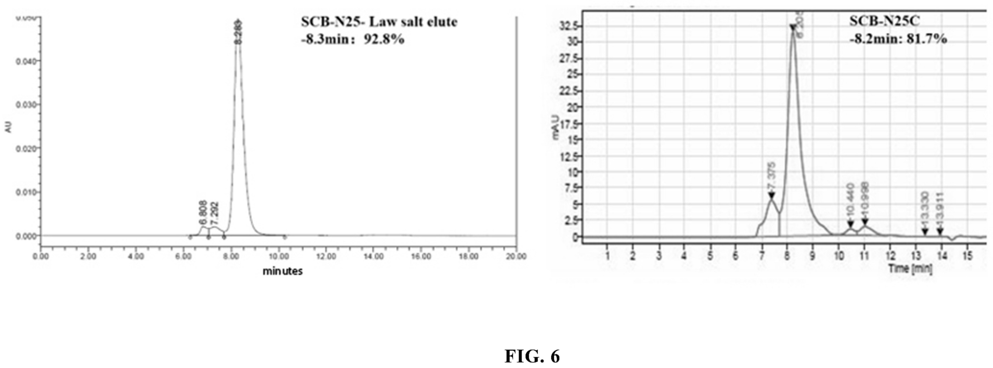

The recombinant plasmid was transfected into GH-CHO (*dfhr-*) cells, and gene amplification was gradually performed with increasing concentrations of MTX (Sigma) under selection in hypoxanthine-thymidine (HT)-free medium (Invitrogen) so as to achieve high-titer expression of the fusion protein in serum-free culture using CD007-4 TM1 medium (Jianshun Biosciences). The exemplary recombinant polypeptide was preliminarily purified by affinity binding to Endo180 and eluted using a salt gradient, and then further purified on a Superdex 200 gel filtration column (GE Healthcare). The purity of the exemplary recombinant polypeptide containing the RSV F peptide was determined by size-exclusion chromatography (SEC-HPLC) according to the manufacturer's instructions (Sepax Technologies) **(****FIG. 6****).** The main peak area of the SCB-N25 fusion protein was 92.8%, and the main peak area of the SCB-N25C protein was 81.7%.

During the serum-free fed-batch culture process, it was found that the production titer of the disulfide bond-linked fusion peptides (e.g., trimers) reached as high as about 0.15 g/L **(****FIG. 1B****).** The conditioned medium containing the trimerized recombinant polypeptides was first purified by affinity binding to Fc-tagged collagen receptor uPARAP/Endo180 (a member of the mannose receptor family) which had been pre-captured onto a protein A chromatography column (Thomas et al., (2005) J. Biol. Chem. 280, 22596-22605), and then purified by gel filtration chromatography. SEC-HPLC analysis indicated that the purity of the exemplary recombinant polypeptide trimers was about 95% **(****FIG. 6****).**

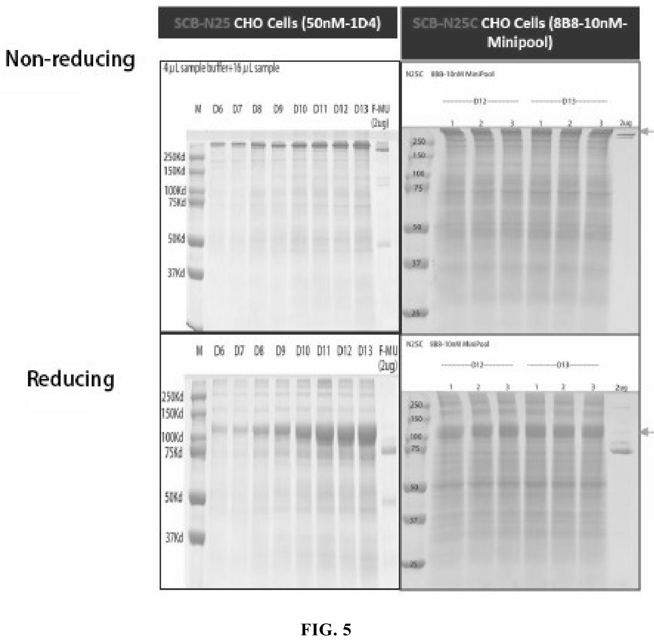

**FIG. 5** shows the expression levels of exemplary fusion peptides derived from serum-free fed-batch cell cultures determined by 8% SDS-PAGE analysis of the peptides. SCB-N25C: the cell-free conditioned medium from Day 1 to Day 13 was separated under non-reducing and reducing conditions, followed by Coomassie blue staining, with a loading amount of 26 µL; SCB-N25: the cell-free conditioned medium from Day 1 to Day 13 was separated under non-reducing and reducing conditions, followed by Coomassie blue staining, with a loading amount of 26 µL. The theoretical molecular weight of the monomer of the fusion protein was 91 kD, and that of the trimer was 273 kD.

**FIG. 7** **illustrates the affinity kinetic assay.** Panels A and B of **FIG. 7** show the binding studies performed by bio-layer interferometry between Synagis monoclonal antibody and an exemplary fusion peptide comprising the RSV F protein peptide.

The affinity analysis instrument used for the experiment was the Fortebio Octet molecular interaction analyzer, and five Protein A probes were selected; before use, the probes were soaked in PBS.

Reagent preparation: Regeneration solution (0.01 M glycine solution): 0.03 g of glycine was accurately weighed, dissolved in water to 40 mL, mixed uniformly, and the pH was adjusted to approximately 1.5 with 6 M hydrochloric acid, and was stored at room temperature.

Sample loading sequence: a clean 96-well test plate was taken, and samples were added in the following order, 200 µL per well.

| 1 | 2 | 3 | 4 | 5 | 6 | 7 | 8 | 9 | 10 | 11 | 12 |
|---|---|---|---|---|---|---|---|---|---|---|---|
| B | L1 | B | S1 | | B | L2 | B | S1 | | R | N |
| B | L1 | B | S2 | | B | L2 | B | S2 | | R | N |
| B | L1 | B | S3 | | B | L2 | B | S3 | | R | N |
| B | L1 | B | S4 | | B | L2 | B | S4 | | R | N |
| B | L1 | B | S5 | | B | L2 | B | S5 | | R | N |

| | | | | | | | | | | | |
|---|---|---|---|---|---|---|---|---|---|---|---|
| **Notes: B:** PBS; L1: 10 µg/mL Synagis; L2: 10 µg/mL D25; S1 = 40 µg/mL protein sample; S2 = 20 µg/mL protein sample; S3 = 10 µg/mL protein sample; S4 = 5 µg/mL protein sample; S5 = PBS; R: Regeneration buffer (0.01M glycine solution); N: PBS. | | | | | | | | | | | |

After the above reagents and samples were added, the data acquisition software was opened, and the information of the samples and the reagents used was entered before running the program. The experiment was carried out in two groups, the first group measuring the affinity between the protein sample to be tested and the Synagis antibody; the second group measuring the affinity between the protein sample to be tested and the D25 antibody. After the run was completed, the Octet data analysis software was used to obtain the Kon, Kdis, and KD results.

The binding affinity performance of Synagis with the purified exemplary SCB-N25 and SCB-N25C recombinant polypeptides was shown in **FIG. 7** and in the labels in FIG. 7. **FIGs. 7C** and **7D** show affinity kinetic studies conducted by the binding between D25 and the exemplary fusion peptides comprising the RSV F protein peptide. The corresponding KD, Kon, Kdis from the antibody D25 affinity experiment were shown in the labels in **FIG. 7****.** SCB-N25C exhibited high affinity to both palivizumab and antibody D25.

### Example 4: Functional Characterization of Recombinant Polypeptides Comprising RSV F Protein Peptides

To evaluate the immunogenicity and protective efficacy of the exemplary recombinant polypeptides produced as described in Example 1, randomly grouped BALB/c mice were intramuscularly immunized twice on Day 0 and Day 21 with one of three doses (1, 6, and 30 µg) of the exemplary fusion polypeptides containing different adjuvants. Another group immunized with PBS served as the control group.

**FIGs. 8A-8D** show the experimental results of immunization with the exemplary fusion peptides comprising the RSV F protein peptide.

**FIG. 8A** shows a schematic diagram of the immunization experimental method: mice were immunized on Day 0 and Day 21, and serum was collected on Day 0 and Day 35. SPF-grade female BALB/c mice aged 6-8 weeks were randomly divided into 16 groups, with 10 mice per group; each group of mice was intramuscularly injected with 50 µL of PBS or antigen, and a booster immunization was administered 3 weeks later. Two weeks after the second immunization, blood was collected from the orbital vein and centrifuged to obtain serum, and spleens from four mice per group were taken for the ELISpot assay.

**FIG. 8B** shows the serum anti-F IgG ELISA titers against the purified exemplary fusion peptides comprising the RSV F protein peptide and the adjuvants Alum, Alum + CpG 1018, or CAS-1. Serum from animals immunized with the hRSV candidate vaccine was mixed with hRSV A2 wild-type virus, and Hep2 cells were added to the serum-virus mixture for incubation. RSV A2 virus was able to infect Hep2 cells, and after incubation for 3-4 days (depending on the cytopathic effect (CPE), with the virus control wells reaching 60% or higher CPE), the newly proliferated virus could be captured by biotin-labeled Synagis antibody, followed by color development using SA-HRP secondary antibody. The absorbance value at OD450 nm indirectly reflected the extent of viral proliferation. If the serum sample contained neutralizing antibodies capable of neutralizing hRSV virus, the neutralized virus could no longer infect cells, thereby affecting the absorbance value. A lower OD450 nm value indicated a higher titer of neutralizing antibodies in the serum. The fusion protein formulated with Alum + CpG 1018 as adjuvant showed higher titers than the corresponding fusion proteins formulated with Alum or CAS-1. SCB-N25C exhibited higher titers than the other antigens.

**FIG. 8C** shows the competitive IgG titers of D25 and palivizumab that provided 50% inhibition of the binding of D25 and palivizumab to heat-inactivated RSV (HI-RSV) particles, as determined by dilution of serum samples. The values are presented as log₂ and mean ± SEM. DCA: D25 was added as the coating antibody to a high-binding ELISA 96-well plate and was coated overnight at 2-8°C, followed by the addition of gradient-diluted serum samples and an SCB-N20-biotin (N20-biotin) antigen-labeled mixture (1:1). The DCA in the immune serum competed with D25 for binding to the Site zero region on the F protein of the N20-biotin antigen label. A secondary antibody (SA-HRP) was subsequently added to recognize N20-biotin, and at each step the unbound portions were removed by washing. Finally, TMB substrate was added for color development, and the reaction was terminated with H₂SO₄. A lower OD450 nm value indicated a higher titer of competitive antibody DCA in the serum. PCA: palivizumab (Synagis) was added as the coating antibody to a high-binding ELISA 96-well plate and was coated overnight at 2-8°C, followed by the addition of gradient-diluted serum samples and an SCB-N20-biotin (N20-biotin) antigen-labeled mixture (1:1). The PCA in the immune serum competed with palivizumab for binding to the Site II region on the F protein of the N20-biotin antigen label. A secondary antibody (SA-HRP) was subsequently added to recognize N20-biotin, and at each step the unbound portions were removed by washing. Finally, TMB substrate was added for color development, and the reaction was terminated with H₂SO₄. A lower OD450 nm value indicated a higher titer of competitive antibody PCA in the serum. All antigens formulated with Alum exhibited lower antibody (DCA and PCA) responses than those formulated with the other two adjuvant formulations. When combined with the Alum + CpG 1018 adjuvant, DS-CAV1-Trimer and N20 both showed high DCA titers and high PCA titers, with no significant difference between the two. N25 performed the worst. For the four candidate antigens combined with the CAS-1 adjuvant, none showed effective DCA titers. N20 and N25 showed higher PCA titers, while DS-CAV1-Trimer exhibited the lowest PCA titer.

**FIG. 8D** shows the results of the ELISpot assay. After vaccination, the splenocytes of mice secreted cytokines locally upon stimulation, and these cytokines were captured by specific monoclonal antibodies. The captured cytokines bound to a biotin-labeled secondary antibody, which subsequently bound to alkaline phosphatase-labeled streptavidin. After incubation with BCIP/NBT substrate, blue-purple spots appeared on the PVDF plate, indicating that the cells secreted specific cytokines. The results were obtained by analyzing the spots using an ELISpot enzyme-linked spot analysis system. The N25C antigen combined with the Alum adjuvant produced a strong Th1-type cellular immune response. When antigens were combined with the Alum + CpG 1018 adjuvant, low Th2-type cellular immune responses were generated. When antigens were combined with the CAS-1 adjuvant, high Th1-type and Th2-type cellular immune responses were generated.

Serum was evaluated by enzyme-linked immunosorbent assay (ELISA). Briefly, 96-well plates were coated overnight at 4°C with 2 µg/mL of purified exemplary fusion peptide (in PBS) and blocked with 1 mg/mL BSA. The plates were washed with PBST and subsequently incubated at room temperature for 2 hours with serial two-fold dilutions of sera (1:64 to 1:262,144). Bound antibodies were detected at room temperature for 1 hour using HRP-conjugated goat anti-mouse IgG (SouthernBiotech). The enzymatic reaction was developed with TMB (Thermo) and terminated by adding 2 M HCl, and the absorbance at 450 nm was recorded. Sera from PBS-immunized mice at the same dilution served as the negative control group, and the antibody titer was defined as the serum dilution that resulted in a ratio of OD RSV F trimer to OD PBS of 2.0.

RSV microneutralization assays were performed using HeLa cells and the RSV A2 strain. Sera were heat-inactivated at 56°C for 30 minutes and serially diluted in serum-free DMEM (50 µL/well) in 96-well cell culture plates. An equal volume of virus (1,000 pfu/mL, prepared in serum-free DMEM) was added to the plates, and the serum/virus mixture was incubated at 37°C for 1 hour. One hundred microliters of DMEM supplemented with 10% FBS containing approximately 5×10⁴ HeLa cells were added to the plates and were incubated at 37°C until the positive-control (virus-only) wells showed 100% CPE. The plates were washed with PBST and were fixed with PBS containing 80% pre-chilled acetone for 10 minutes. Appropriate palivizumab was added to the wells, the wells were blocked with 1 mg/mL BSA for 1 hour, and incubated at room temperature for 2 hours. After three washes, HRP-conjugated goat anti-human IgG (SouthernBiotech) was added, the enzymatic reaction was developed, and the OD at 450 nm was recorded. The dilution causing 50% inhibition of CPE formation was determined to be the neutralizing antibody titer.

Since antigenic site II was exposed on the exemplary recombinant polypeptide, palivizumab and D25 monoclonal antibody competitive ELISAs were performed to determine whether the antibodies induced by the exemplary recombinant polypeptide were directed against this site.

Competitive ELISAs with palivizumab and D25 monoclonal antibodies were performed using 96-well ELISA plates coated with 5×10⁶ pfu/mL heat-inactivated RSV (HI-RSV, in 50 mM carbonate-bicarbonate buffer, pH 9.2) and were incubated overnight at 4°C. Uncoated surfaces were blocked with 1 mg/mL BSA. Two-fold serial dilutions of serum mixtures (1:32 to 1:4,096) together with appropriate amounts of palivizumab and D25 monoclonal antibodies were added to the wells and were incubated at room temperature for 2 hours. Bound palivizumab was detected using HRP-conjugated goat anti-human IgG (SouthernBiotech) and TMB substrate. Wells containing sera from PBS-immunized mice served as non-competitive positive controls, and the percentage inhibition was calculated as ((OD PBS - OD RSV F trimer)/OD PBS) × 100%. The competitive binding titer was expressed as the dilution causing 50% inhibition.

The present invention is not intended to be limited in scope to the particular disclosed embodiments, which are provided for purposes such as illustrating various aspects of the invention. Various modifications of the compositions and methods will be apparent from the description and teachings herein. Such variations may be made without departing from the true scope and spirit of the present disclosure and are intended to fall within the scope of the present disclosure.

### SEQUENCE

| **SEQ ID NO.** | **SEQUENCE** | **Description** |
|---|---|---|
| 1 | QNITEEFYQSTCSAVSKGYLSALRTGWYTSVITIELSNIKENKCNG TDAKVKLIKQELDKYKNAVTELQLLMQSTPATNNRARRELPRFM NYTLNNAKKTNVTLSKKRKRRFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide: 806 aa |
| 2 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGKRSNGLPGPIGPPGPRGRTGDAGPVGPP GPPGPPGPPGPPSAGFDFSFLPQPPQEKAHDGGRYYRANDANVVR DRDLEVDTTLKSLSQQIENIRSPEGSRKNPARTCRDLKMCHSDWK SGEYWIDPNQGCNLDAIKVFCNMETGETCVYPTQPSVAQKNWYI SKNPKDKRHVWFGESMTDGFQFEYGGQGSDPADVAIQLTFLRLM STEASQNITYHCKNSVAYMDQQTGNLKKALLLQGSNEIEIRAEGN SRFTYSVTVDGCTSHTGAWGKTVIEYKTTKTSRLPIIDVAPLDVG APDQEFGFDVGPVCFL | RSV fusion glycoprotein F trimeric fusion polypeptide with signal peptide, 831 aa |
| 3 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRRFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide, 806 aa (R109A mutant) |
| 4 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGKRSNGLPGPIGPPGPRGRTGDAGPVGPP GPPGPPGPPGPPSAGFDFSFLPQPPQEKAHDGGRYYRANDANVVR DRDLEVDTTLKSLSQQIENIRSPEGSRKNPARTCRDLKMCHSDWK SGEYWIDPNQGCNLDAIKVFCNMETGETCVYPTQPSVAQKNWYI SKNPKDKRHVWFGESMTDGFQFEYGGQGSDPADVAIQLTFLRLM STEASQNITYHCKNSVAYMDQQTGNLKKALLLQGSNEIEIRAEGN SRFTYSVTVDGCTSHTGAWGKTVIEYKTTKTSRLPIIDVAPLDVG APDQEFGFDVGPVCFL | RSV fusion glycoprotein F trimeric fusion polypeptide with signal peptide, 831 aa (R109A mutant) |
| 5 | QNITEEFYQSTCSAVSKGYLSALRTGWYTSVITIELSNIKENKCNG TDAKVKLIKQELDKYKNAVTELQLLMQSTPATNNRARRELPRFM NYTLNNAKKTNVTLSKKRKRAFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide, 806 aa (R136A mutant) |
| 6 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRA FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGKRSNGLPGPIGPPGPRGRTGDAGPVGPP GPPGPPGPPGPPSAGFDFSFLPQPPQEKAHDGGRYYRANDANVVR DRDLEVDTTLKSLSQQIENIRSPEGSRKNPARTCRDLKMCHSDWK SGEYWIDPNQGCNLDAIKVFCNMETGETCVYPTQPSVAQKNWYI SKNPKDKRHVWFGESMTDGFQFEYGGQGSDPADVAIQLTFLRLM STEASQNITYHCKNSVAYMDQQTGNLKKALLLQGSNEIEIRAEGN SRFTYSVTVDGCTSHTGAWGKTVIEYKTTKTSRLPIIDVAPLDVG APDQEFGFDVGPVCFL | RSV fusion glycoprotein F trimeric fusion polypeptide with signal peptide, 831 aa (R136A mutant) |
| 7 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRAFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide, 806 aa (R109A/R136A mutant) |
| 8 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRA FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGKRSNGLPGPIGPPGPRGRTGDAGPVGPP GPPGPPGPPGPPSAGFDFSFLPQPPQEKAHDGGRYYRANDANVVR DRDLEVDTTLKSLSQQIENIRSPEGSRKNPARTCRDLKMCHSDWK SGEYWIDPNQGCNLDAIKVFCNMETGETCVYPTQPSVAQKNWYI SKNPKDKRHVWFGESMTDGFQFEYGGQGSDPADVAIQLTFLRLM STEASQNITYHCKNSVAYMDQQTGNLKKALLLQGSNEIEIRAEGN SRFTYSVTVDGCTSHTGAWGKTVIEYKTTKTSRLPIIDVAPLDVG APDQEFGFDVGPVCFL | RSV fusion glycoprotein F trimeric fusion polypeptide with signal peptide, 831 aa (R109A/R136A mutant), |
| 9 | QNITEEFYQSTCSAVSKGYLSALRTGWYTSVITIELSNIKENKCNG TDAKVKLIKQELDKYKNAVTELQLLMQSTPATNNRARRELPRFM NYTLNNAKKTNVTLSKKRKRRFLGFLLGVGSAIASGVAVSKVLH LPGEVNKIKSALLSTNKAVVSLSNGVSVLTSKVLDLKNYIDKQLL PIVNKQSCSIPNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide, 806 aa (E161P/S215P mutant) |
| 10 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRR FLGFLLGVGSAIASGVAVSKVLHLPGEVNKIKSALLSTNKAVVSLS NGVSVLTSKVLDLKNYIDKQLLPIVNKQSCSIPNIATVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEVNLCNVDIFNPKYDCKIMTSKTDVSSSVITSLG AIVSCYGKTKCTASNKNRGIIKTFSNGCDYVSNKGVDTVSVGNTL YYVNKQEGKSLYVKGEPIINFYDPLVFPSDEFDASISQVNEKINQS LAFIRKSDELLHNVNAGKRSNGLPGPIGPPGPRGRTGDAGPVGPP GPPGPPGPPGPPSAGFDFSFLPQPPQEKAHDGGRYYRANDANVVR DRDLEVDTTLKSLSQQIENIRSPEGSRKNPARTCRDLKMCHSDWK SGEYWIDPNQGCNLDAIKVFCNMETGETCVYPTQPSVAQKNWYI SKNPKDKRHVWFGESMTDGFQFEYGGQGSDPADVAIQLTFLRLM STEASQNITYHCKNSVAYMDQQTGNLKKALLLQGSNEIEIRAEGN SRFTYSVTVDGCTSHTGAWGKTVIEYKTTKTSRLPIIDVAPLDVG APDQEFGFDVGPVCFL | RSV fusion glycoprotein F trimeric fusion polypeptide with signal peptide, 831 aa (E161P/S215P mutant) |
| 11 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRAFLGFLLGVGSAIASGVAVSKVLH LPGEVNKIKSALLSTNKAVVSLSNGVSVLTSKVLDLKNYIDKQLL PIVNKQSCSIPNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide, 806 aa (R109A/R136A/E161P/S215 P mutant) |
| 12 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRA FLGFLLGVGSAIASGVAVSKVLHLPGEVNKIKSALLSTNKAVVSLS NGVSVLTSKVLDLKNYIDKQLLPIVNKQSCSIPNIATVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEVNLCNVDIFNPKYDCKIMTSKTDVSSSVITSLG AIVSCYGKTKCTASNKNRGIIKTFSNGCDYVSNKGVDTVSVGNTL YYVNKQEGKSLYVKGEPIINFYDPLVFPSDEFDASISQVNEKINQS LAFIRKSDELLHNVNAGKRSNGLPGPIGPPGPRGRTGDAGPVGPP GPPGPPGPPGPPSAGFDFSFLPQPPQEKAHDGGRYYRANDANVVR DRDLEVDTTLKSLSQQIENIRSPEGSRKNPARTCRDLKMCHSDWK SGEYWIDPNQGCNLDAIKVFCNMETGETCVYPTQPSVAQKNWYI SKNPKDKRHVWFGESMTDGFQFEYGGQGSDPADVAIQLTFLRLM STEASQNITYHCKNSVAYMDQQTGNLKKALLLQGSNEIEIRAEGN SRFTYSVTVDGCTSHTGAWGKTVIEYKTTKTSRLPIIDVAPLDVG APDQEFGFDVGPVCFL | RSV fusion glycoprotein F trimeric fusion polypeptide with signal peptide, 831 aa (R109A/R136A/E161P/S215 P mutant), SCB-N20 |
| 13 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRAFLGFLLGVGSAIASGVAVSKVLH LPGEVNKIKSALLSTNKAVVSLSNGVSVLTSKVLDLKNYIDKQLL PIVNKQSCSIPNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSA NVVRDRDLEVDTTLKSLSQQIENIRSPEGSRKNPARTCRDLKMCH SDWKSGEYWIDPNQGCNLDAIKVFCNMETGETCVYPTQPSVAQK NWYISKNPKDKRHVWFGESMTDGFQFEYGGQGSDPADVAIQLTF LRLMSTEASQNITYHCKNSVAYMDQQTGNLKKALLLQGSNEIEIR AEGNSRFTYSVTVDGCTSHTGAWGKTVIEYKTTKTSRLPIIDVAPL DVGAPDQEFGFDVGPVCFL | RSV fusion glycoprotein F trimeric fusion polypeptide F-CICP without signal peptide (no glycine repeat sequence and BMP-1 site), 741 aa (R109A/R136A/E161P/S215 P mutant) |
| 14 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRA FLGFLLGVGSAIASGVAVSKVLHLPGEVNKIKSALLSTNKAVVSLS NGVSVLTSKVLDLKNYIDKQLLPIVNKQSCSIPNIATVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEVNLCNVDIFNPKYDCKIMTSKTDVSSSVITSLG AIVSCYGKTKCTASNKNRGIIKTFSNGCDYVSNKGVDTVSVGNTL YYVNKQEGKSLYVKGEPIINFYDPLVFPSDEFDASISQVNEKINQS LAFIRKSDELLHNVNAGKRSANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | RSV fusion glycoprotein F trimeric fusion polypeptide F-CICP with signal peptide (no glycine repeat sequence and BMP-1 site), 766 aa (R109A/R136A/E161P/S215 P mutant) |
| 15 | QNITEEFYQSTCSAVSKGYLSALRTGWYTSVITIELSNIKENKCNG TDAKVKLIKQELDKYKNAVTELQLLMQSTPPTNNRARRELPRFM NYTLNNAKKTNVTLSKKRKRRFLGFLLGVGSAIASGVAVSKVLH LEGEVNKIKSALLSTNKAVVSLSNGVSVLTSKVLDLKNYIDKQLL PIVNKQSCSISNIETVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEINLCNVDIFN PKYDCKIMTSKTDVSSSVITSLGAIVSCYGKTKCTASNKNRGIIKT FSNGCDYVSNKGMDTVSVGNTLYYVNKQEGKSLYVKGEPIINFY DPLVFPSDEFDASISQVNEKINQSLAFIRKSDELLHNVNAGKRSNG LPGPIGPPGPRGRTGDAGPVGPPGPPGPPGPPGPPSAGFDFSFLPQP PQEKAHDGGRYYRANDANVVRDRDLEVDTTLKSLSQQIENIRSPE GSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F trimeric fusion polypeptide without signal peptide, 806 aa (P102, E218, I379, M447) |
| 16 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPPTNNRARRELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIETVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEINLCNVDIFNPKYDCKIMTSKTDVSSSVITSLGA IVSCYGKTKCTASNKNRGIIKTFSNGCDYVSNKGMDTVSVGNTLY YVNKQEGKSLYVKGEPIINFYDPLVFPSDEFDASISQVNEKINQSL AFIRKSDELLHNVNAGKRSNGLPGPIGPPGPRGRTGDAGPVGPPGP PGPPGPPGPPSAGFDFSFLPQPPQEKAHDGGRYYRANDANVVRDR DLEVDTTLKSLSQQIENIRSPEGSRKNPARTCRDLKMCHSDWKSG EYWIDPNQGCNLDAIKVFCNMETGETCVYPTQPSVAQKNWYISK NPKDKRHVWFGESMTDGFQFEYGGQGSDPADVAIQLTFLRLMST EASQNITYHCKNSVAYMDQQTGNLKKALLLQGSNEIEIRAEGNSR FTYSVTVDGCTSHTGAWGKTVIEYKTTKTSRLPIIDVAPLDVGAP DQEFGFDVGPVCFL | RSV fusion glycoprotein F trimeric fusion polypeptide with signal peptide, 831 aa (P102, E218, I379, M447) |
| 17 | QNITEEFYQSTCSAVSKGYLSALRTGWYTSVITIELSNIKENKCNG TDAKVKLIKQELDKYKNAVTELQLLMQSTPATNNRARRELPRFM NYTLNNAKKTNVTLSKKRKRRFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGK | RSV fusion glycoprotein F extracellular domain without signal peptide |
| 18 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGK | RSV fusion glycoprotein F extracellular domain with signal peptide |
| 19 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRRFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGK | RSV fusion glycoprotein F extracellular domain without signal peptide (R109A mutant) |
| 20 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVOIVROOSYSIMSIIKEEVLAYVVOLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGK | RSV fusion glycoprotein F extracellular domain with signal peptide (R109A mutant) |
| 21 | QNITEEFYQSTCSAVSKGYLSALRTGWYTSVITIELSNIKENKCNG TDAKVKLIKQELDKYKNAVTELQLLMQSTPATNNRARRELPRFM NYTLNNAKKTNVTLSKKRKRAFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGKRSN GLPGPIGPPGPRGRTGDAGPVGPPGPPGPPGPPGPPSAGFDFSFLPQ PPQEKAHDGGRYYRANDANVVRDRDLEVDTTLKSLSQQIENIRSP EGSRKNPARTCRDLKMCHSDWKSGEYWIDPNQGCNLDAIKVFCN METGETCVYPTQPSVAQKNWYISKNPKDKRHVWFGESMTDGFQ FEYGGQGSDPADVAIQLTFLRLMSTEASQNITYHCKNSVAYMDQ QTGNLKKALLLQGSNEIEIRAEGNSRFTYSVTVDGCTSHTGAWGK TVIEYKTTKTSRLPIIDVAPLDVGAPDQEFGFDVGPVCFL | RSV fusion glycoprotein F extracellular domain without signal peptide (R136A mutant) |
| 22 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRA FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGK | RSV fusion glycoprotein F extracellular domain with signal peptide (R136A mutant) |
| 23 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRAFLGFLLGVGSAIASGVAVSKVLH LEGEVNKIKSALLSTNKAVVSLSNGVSVLTSKVLDLKNYIDKQLL PIVNKQSCSISNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGK | RSV fusion glycoprotein F extracellular domain without signal peptide (R109A/R136A mutant) |
| 24 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRA FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGK | RSV fusion glycoprotein F extracellular domain with signal peptide (R109A/R136A mutant) |
| 25 | QNITEEFYQSTCSAVSKGYLSALRTGWYTSVITIELSNIKENKCNG TDAKVKLIKQELDKYKNAVTELQLLMQSTPATNNRARRELPRFM NYTLNNAKKTNVTLSKKRKRRFLGFLLGVGSAIASGVAVSKVLH LPGEVNKIKSALLSTNKAVVSLSNGVSVLTSKVLDLKNYIDKQLL PIVNKQSCSIPNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGK | RSV fusion glycoprotein F extracellular domain without signal peptide (E161P/S215P mutant) |
| 26 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRR FLGFLLGVGSAIASGVAVSKVLHLPGEVNKIKSALLSTNKAVVSLS NGVSVLTSKVLDLKNYIDKQLLPIVNKQSCSIPNIATVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEVNLCNVDIFNPKYDCKIMTSKTDVSSSVITSLG AIVSCYGKTKCTASNKNRGIIKTFSNGCDYVSNKGVDTVSVGNTL YYVNKQEGKSLYVKGEPIINFYDPLVFPSDEFDASISQVNEKINQS LAFIRKSDELLHNVNAGK | RSV fusion glycoprotein F extracellular domain with signal peptide (E161P/S215P mutant) |
| 27 | QNITEEFYQSTCSAVSKGYLSALRTGWYTSVITIELSNIKENKCNG TDAKVKLIKQELDKYKNAVTELQLLMQSTPATNNRARAELPRFM NYTLNNAKKTNVTLSKKRKRAFLGFLLGVGSAIASGVAVSKVLH LPGEVNKIKSALLSTNKAVVSLSNGVSVLTSKVLDLKNYIDKQLL PIVNKQSCSIPNIATVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEVNLCNVDIF NPKYDCKIMTSKTDVSSSVITSLGAIVSCYGKTKCTASNKNRGIIK TFSNGCDYVSNKGVDTVSVGNTLYYVNKQEGKSLYVKGEPIINF YDPLVFPSDEFDASISQVNEKINQSLAFIRKSDELLHNVNAGK | RSV fusion glycoprotein F extracellular domain without signal peptide (R109A/R136A/E161P/S215 P mutant) |
| 28 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARAELPRFMNYTLNNAKKTNVTLSKKRKRA FLGFLLGVGSAIASGVAVSKVLHLPGEVNKIKSALLSTNKAVVSLS NGVSVLTSKVLDLKNYIDKQLLPIVNKQSCSIPNIATVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEVNLCNVDIFNPKYDCKIMTSKTDVSSSVITSLG AIVSCYGKTKCTASNKNRGIIKTFSNGCDYVSNKGVDTVSVGNTL YYVNKQEGKSLYVKGEPIINFYDPLVFPSDEFDASISQVNEKINQS LAFIRKSDELLHNVNAGK | RSV fusion glycoprotein F extracellular domain with signal peptide (R109A/R136A/E161P/S215 P mutant) |
| 29 | QNITEEFYQSTCSAVSKGYLSALRTGWYTSVITIELSNIKENKCNG TDAKVKLIKQELDKYKNAVTELQLLMQSTPPTNNRARRELPRFM NYTLNNAKKTNVTLSKKRKRRFLGFLLGVGSAIASGVAVSKVLH LEGEVNKIKSALLSTNKAVVSLSNGVSVLTSKVLDLKNYIDKQLL PIVNKQSCSISNIETVIEFQQKNNRLLEITREFSVNAGVTTPVSTYM LTNSELLSLINDMPITNDQKKLMSNNVQIVRQQSYSIMSIIKEEVL AYVVQLPLYGVIDTPCWKLHTSPLCTTNTKEGSNICLTRTDRGWY CDNAGSVSFFPQAETCKVQSNRVFCDTMNSLTLPSEINLCNVDIFN PKYDCKIMTSKTDVSSSVITSLGAIVSCYGKTKCTASNKNRGIIKT FSNGCDYVSNKGMDTVSVGNTLYYVNKQEGKSLYVKGEPIINFY DPLVFPSDEFDASISQVNEKINQSLAFIRKSDELLHNVNAGK | An RSV fusion glycoprotein F extracellular domain without signal peptide (P102, E218, I379, M447) |
| 30 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPPTNNRARRELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIETVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEINLCNVDIFNPKYDCKIMTSKTDVSSSVITSLGA IVSCYGKTKCTASNKNRGIIKTFSNGCDYVSNKGMDTVSVGNTLY YVNKQEGKSLYVKGEPIINFYDPLVFPSDEFDASISQVNEKINQSL AFIRKSDELLHNVNAGK | An RSV fusion glycoprotein F extracellular domain with signal peptide (P102, E218, I379, M447) |
| 31 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPPTNNRARRELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIETVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEINLCNVDIFNPKYDCKIMTSKTDVSSSVITSLGA IVSCYGKTKCTASNKNRGIIKTFSNGCDYVSNKGMDTVSVGNTLY YVNKQEGKSLYVKGEPIINFYDPLVFPSDEFDASISQVNEKINQSL AFIRKSDELLHNVNAGKSTTNIMITTIIIVIIVILLSLIAVGLLLYCKA RSTPVTLSKDQLSGINNIAFSN | RSV fusion glycoprotein F0, 574 aa (P102, E218, I379, M447) |
| 32 | MELLILKANAITTILTAVTFCFASGQNITEEFYQSTCSAVSKGYLSA LRTGWYTSVITIELSNIKENKCNGTDAKVKLIKQELDKYKNAVTE LQLLMQSTPATNNRARRELPRFMNYTLNNAKKTNVTLSKKRKRR FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGKSTTNIMITTIIIVIIVILLSLIAVGLLLYC KARSTPVTLSKDQLSGINNIAFSN | RSV fusion glycoprotein F0, 574 aa (A102, A218, V379, V447) |
| 33 | MELLILKANAITTILTAVTFCFASG | RSV fusion glycoprotein F signal peptide, 25 aa (1-25 of SEQ ID NO: 31 or 32) |
| 34 | QNITEEFYQSTCSAVSKGYLSALRTGWYTSVITIELSNIKENKCNG TDAKVKLIKQELDKYKNAVTELQLLMQSTPPTNNRARR | RSV fusion glycoprotein F2(26-109 of SEQ ID NO: 31) |
| 35 | QNITEEFYQSTCSAVSKGYLSALRTGWYTSVITIELSNIKENKCNG TDAKVKLIKQELDKYKNAVTELQLLMQSTPATNNRARR | RSV fusion glycoprotein F2(26-109 of SEQ ID NO: 32) |
| 36 | RARR | furin site I (106-109 of SEQ ID NO: 31 or 32) |
| 37 | RARA | furin site I mutant (R109A) |
| 38 | ELPRFMNYTLNNAKKTNVTLSKKRKRR | RSV fusion glycoprotein F pep27 (110-136 of SEQ ID NO: 31 or 32) |
| 39 | KKRKRR | furin site II (131-136 of seq id no: 31 or 32) |
| 40 | KKRKRA | furin site II mutant (R136A) |
| 41 | FLGFLLGVGSAIASGVAVS | RSV fusion glycoprotein F FP (137-155 of SEQ ID NO: 31 or 32) |
| 42 | FLGFLLGVGSAIASGVAVSKVLHLEGEVNKIKSALLSTNKAVVSL SNGVSVLTSKVLDLKNYIDKQLLPIVNKQSCSISNIETVIEFQQKNN RLLEITREFSVNAGVTTPVSTYMLTNSELLSLINDMPITNDQKKLM SNNVQIVRQQSYSIMSIIKEEVLAYVVQLPLYGVIDTPCWKLHTSP LCTTNTKEGSNICLTRTDRGWYCDNAGSVSFFPQAETCKVQSNRV FCDTMNSLTLPSEINLCNVDIFNPKYDCKIMTSKTDVSSSVITSLGA IVSCYGKTKCTASNKNRGIIKTFSNGCDYVSNKGMDTVSVGNTLY YVNKQEGKSLYVKGEPIINFYDPLVFPSDEFDASISQVNEKINQSL AFIRKSDELLHNVNAGKSTTN | RSV fusion glycoprotein F1(137-524 of SEQ ID NO: 31) |
| 43 | FLGFLLGVGSAIASGVAVSKVLHLEGEVNKIKSALLSTNKAVVSL SNGVSVLTSKVLDLKNYIDKQLLPIVNKQSCSISNIATVIEFQQKN NRLLEITREFSVNAGVTTPVSTYMLTNSELLSLINDMPITNDQKKL MSNNVQIVRQQSYSIMSIIKEEVLAYVVQLPLYGVIDTPCWKLHTS PLCTTNTKEGSNICLTRTDRGWYCDNAGSVSFFPQAETCKVQSNR VFCDTMNSLTLPSEVNLCNVDIFNPKYDCKIMTSKTDVSSSVITSL GAIVSCYGKTKCTASNKNRGIIKTFSNGCDYVSNKGVDTVSVGNT LYYVNKQEGKSLYVKGEPIINFYDPLVFPSDEFDASISQVNEKINQ SLAFIRKSDELLHNVNAGKSTTN | RSV fusion glycoprotein F1(137-524 of SEQ ID NO: 32) |
| 44 | IMITTIIIVIIVILLSLIAVGLLLYC | RSV fusion glycoprotein F transmembrane domain (SEQ ID NO: 31 or 32; 525-550) |
| 45 | KARSTPVTLSKDQLSGINNIAFSN | RSV fusion glycoprotein F cytoplasmic domain (551-574 of SEQ ID NO: 31 or 32) |
| 46 | KVLHLEGEVNKIKSALLSTNKAVVSLSNGVSVLTSKVLDLKNYID KQLLPIVNKQSCSISNIETVIEFQQKNNRLLEITREFSVNAGVTTPV STYMLTNSELLSLINDMPITNDQKKLMSNNVQIVRQQSYSIMSIIK EEVLAYVVQLPLYGVIDTPCWKLHTSPLCTTNTKEGSNICLTRTD RGWYCDNAGSVSFFPQAETCKVQSNRVFCDTMNSLTLPSEINLCN VDIFNPKYDCKIMTSKTDVSSSVITSLGAIVSCYGKTKCTASNKNR GIIKTFSNGCDYVSNKGMDTVSVGNTLYYVNKQEGKSLYVKGEPI INFYDPLVFPSDEFDASISQVNEKINQSLAFIRKSDELLHNVNAGK | RSV fusion glycoprotein F1 fragment (156-520 of SEQ ID NO: 31) |
| 47 | KVLHLEGEVNKIKSALLSTNKAVVSLSNGVSVLTSKVLDLKNYID KQLLPIVNKQSCSISNIATVIEFQQKNNRLLEITREFSVNAGVTTPV STYMLTNSELLSLINDMPITNDQKKLMSNNVQIVRQQSYSIMSIIK EEVLAYVVQLPLYGVIDTPCWKLHTSPLCTTNTKEGSNICLTRTD RGWYCDNAGSVSFFPQAETCKVQSNRVFCDTMNSLTLPSEVNLC NVDIFNPKYDCKIMTSKTDVSSSVITSLGAIVSCYGKTKCTASNKN RGIIKTFSNGCDYVSNKGVDTVSVGNTLYYVNKQEGKSLYVKGE PIINFYDPLVFPSDEFDASISQVNEKINQSLAFIRKSDELLHNVNAG K | RSV fusion glycoprotein F1 fragment (SEQ ID NO: 32; 156-520) |
| 48 | ANVVRDRDLEVDTTLKSLSQQIENIRSPEGSRKNPARTCRDLKMC HSDWKSGEYWIDPNQGCNLDAIKVFCNMETGETCVYPTQPSVAQ KNWYISKNPKDKRHVWFGESMTDGFQFEYGGQGSDPADVAIQLT FLRLMSTEASQNITYHCKNSVAYMDQQTGNLKKALLLQGSNEIEI RAEGNSRFTYSVTVDGCTSHTGAWGKTVIEYKTTKTSRLPIIDVAP LDVGAPDQEFGFDVGPVCFL | Trimerized peptide (type I), QT version |
| 49 | RSANVVRDRDLEVDTTLKSLSQQIENIRSPEGSRKNPARTCRDLK MCHSDWKSGEYWIDPNQGCNLDAIKVFCNMETGETCVYPTQPSV AQKNWYISKNPKDKRHVWFGESMTDGFQFEYGGQGSDPADVAI QLTFLRLMSTEASQNITYHCKNSVAYMDQQTGNLKKALLLQGSN EIEIRAEGNSRFTYSVTVDGCTSHTGAWGKTVIEYKTTKTSRLPIID VAPLDVGAPDQEFGFDVGPVCFL | Trimerized peptide (type I), QT version |
| 50 | NGLPGPIGPPGPRGRTGDAGPVGPPGPPGPPGPPGPPSAGFDFSFLP QPPQEKAHDGGRYYRANDANVVRDRDLEVDTTLKSLSQQIENIR SPEGSRKNPARTCRDLKMCHSDWKSGEYWIDPNQGCNLDAIKVF CNMETGETCVYPTQPSVAQKNWYISKNPKDKRHVWFGESMTDG FQFEYGGQGSDPADVAIQLTFLRLMSTEASQNITYHCKNSVAYM DQQTGNLKKALLLQGSNEIEIRAEGNSRFTYSVTVDGCTSHTGAW GKTVIEYKTTKTSRLPIIDVAPLDVGAPDQEFGFDVGPVCFL | Trimerized peptide (type I) having a Gly-X-Y repeat sequence and a D→N mutation of BMP-1 site, QT version |
| 51 | NGLPGPIGPPGPRGRTGDAGPVGPPGPPGPPGPPGPPSAGFDFSFLP QPPQEKAHDGGRYYRNDDANVVRDRDLEVDTTLKSLSQQIENIR SPEGSRKNPARTCRDLKMCHSDWKSGEYWIDPNQGCNLDAIKVF CNMETGETCVYPTQPSVAQKNWYISKNPKDKRHVWFGESMTDG FQFEYGGQGSDPADVAIQLTFLRLMSTEASQNITYHCKNSVAYM DQQTGNLKKALLLQGSNEIEIRAEGNSRFTYSVTVDGCTSHTGAW GKTVIEYKTTKTSRLPIIDVAPLDVGAPDQEFGFDVGPVCFL | Trimerized peptide (type I) having a Gly-X-Y repeat sequence and an A→N-mutation of BMP-1 site, QT version |
| 52 | RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | Trimerized peptide (type I) having a Gly-X-Y repeat sequence and a D→N mutation of BMP-1 site, QT version |
| 53 | GSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | Trimerized peptide (type I) having a Gly-X-Y repeat sequence and a D→N mutation of BMP-1 site, QT version |
| 54 | ANVVRDRDLEVDTTLKSLSQQIENIRSPEGSRKNPARTCRDLKMC HSDWKSGEYWIDPNQGCNLDAIKVFCNMETGETCVYPTQPSVAQ KNWYISKNPKDKRHVWFGESMTDGFQFEYGGQGSDPADVAIQLT FLRLMSTEASQNITYHCKNSVAYMDQQTGNLKKALLLKGSNEIEI RAEGNSRFTYSVTVDGCTSHTGAWGKTVIEYKTTKSSRLPIIDVAP LDVGAPDQEFGFDVGPVCFL | Trimerized peptide (type I), KS version |
| 55 | RSANVVRDRDLEVDTTLKSLSQQIENIRSPEGSRKNPARTCRDLK MCHSDWKSGEYWIDPNQGCNLDAIKVFCNMETGETCVYPTQPSV AQKNWYISKNPKDKRHVWFGESMTDGFQFEYGGQGSDPADVAI QLTFLRLMSTEASQNITYHCKNSVAYMDQQTGNLKKALLLKGSN EIEIRAEGNSRFTYSVTVDGCTSHTGAWGKTVIEYKTTKSSRLPIID VAPLDVGAPDQEFGFDVGPVCFL | Trimerized peptide (type I), KS version |
| 56 | NGLPGPIGPPGPRGRTGDAGPVGPPGPPGPPGPPGPPSAGFDFSFLP QPPQEKAHDGGRYYRANDANVVRDRDLEVDTTLKSLSQQIENIR SPEGSRKNPARTCRDLKMCHSDWKSGEYWIDPNQGCNLDAIKVF CNMETGETCVYPTQPSVAQKNWYISKNPKDKRHVWFGESMTDG FQFEYGGQGSDPADVAIQLTFLRLMSTEASQNITYHCKNSVAYM DQQTGNLKKALLLKGSNEIEIRAEGNSRFTYSVTVDGCTSHTGAW GKTVIEYKTTKSSRLPIIDVAPLDVGAPDQEFGFDVGPVCFL | Trimerized peptide (type I) having a Gly-X-Y repeat sequence and a D→N mutation of BMP-1 site, KS version |
| 57 | NGLPGPIGPPGPRGRTGDAGPVGPPGPPGPPGPPGPPSAGFDFSFLP QPPQEKAHDGGRYYRNDDANVVRDRDLEVDTTLKSLSQQIENIR SPEGSRKNPARTCRDLKMCHSDWKSGEYWIDPNQGCNLDAIKVF CNMETGETCVYPTQPSVAQKNWYISKNPKDKRHVWFGESMTDG FQFEYGGQGSDPADVAIQLTFLRLMSTEASQNITYHCKNSVAYM DQQTGNLKKALLLKGSNEIEIRAEGNSRFTYSVTVDGCTSHTGAW GKTVIEYKTTKSSRLPIIDVAPLDVGAPDQEFGFDVGPVCFL | Trimerized peptide (type I) having a Gly-X-Y repeat sequence and an A →N-mutation of BMP-1 site, KS version |
| 58 | RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLKGSNEIEIRAEGNSRFTYSVTVDGCTSHTGA WGKTVIEYKTTKSSRLPIIDVAPLDVGAPDQEFGFDVGPVCFL | Trimerized peptide (type I) having a Gly-X-Y repeat sequence and a D→N mutation of BMP-1 site, KS version |
| 59 | GSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLKGSNEIEIRAEGNSRFTYSVTVDGCTSHTGA WGKTVIEYKTTKSSRLPIIDVAPLDVGAPDQEFGFDVGPVCFL | Trimerized peptide (type I) having a Gly-X-Y repeat sequence and a D→N mutation of BMP-1 site, KS version |
| 60 | DEIMTSLKSVNGQIESLISPDGSRKNPARNCRDLKFCHPELKSGEY WVDPNQGCKLDAIKVFCNMETGETCISANPLNVPRKHWWTDSSA EKKHVWFGESMDGGFQFSYGNPELPEDVLDVQLAFLRLLSSRAS QNITYHCKNSIAYMDQASGNVKKALKLMGSNEGEFKAEGNSKFT YTVLEDGCTKHTGEWSKTVFEYRTRKAVRLPIVDIAPYDIGGPDQ EFGVDVGPVCF | Trimerized peptide (type III) |
| 61 | EPMDFKINTDEIMTSLKSVNGQIESLISPDGSRKNPARNCRDLKFC HPELKSGEYWVDPNQGCKLDAIKVFCNMETGETCISANPLNVPR KHWWTDSSAEKKHVWFGESMDGGFQFSYGNPELPEDVLDVQLA FLRLLSSRASQNITYHCKNSIAYMDQASGNVKKALKLMGSNEGEF KAEGNSKFTYTVLEDGCTKHTGEWSKTVFEYRTRKAVRLPIVDIA PYDIGGPDQEFGVDVGPVCFL | Trimerized peptide (type III) |
| 62 | SEPMDFKINTDEIMTSLKSVNGQIESLISPDGSRKNPARNCRDLKF CHPELKSGEYWVDPNQGCKLDAIKVFCNMETGETCISANPLNVPR KHWWTDSSAEKKHVWFGESMDGGFQFSYGNPELPEDVLDVQLA FLRLLSSRASQNITYHCKNSIAYMDQASGNVKKALKLMGSNEGEF KAEGNSKFTYTVLEDGCTKHTGEWSKTVFEYRTRKAVRLPIVDIA PYDIGGPDQEFGVDVGPVCFL | Trimerized peptide (type III) |
| 63 | RSEPMDFKINTDEIMTSLKSVNGQIESLISPDGSRKNPARNCRDLK FCHPELKSGEYWVDPNQGCKLDAIKVFCNMETGETCISANPLNVP RKHWWTDSSAEKKHVWFGESMDGGFQFSYGNPELPEDVLDVQL AFLRLLSSRASQNITYHCKNSIAYMDQASGNVKKALKLMGSNEG EFKAEGNSKFTYTVLEDGCTKHTGEWSKTVFEYRTRKAVRLPIVD IAPYDIGGPDQEFGVDVGPVCFL | Trimerized peptide (type III) |
| 64 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNCACRE LPRFMNYTLN NAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide (SCB-N25C) (R106C, R108C, R133C, R135C, R136C) |
| 65 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNRACRE LPRFMNYTLNNAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide: R108C, R133C, R135C, R136C |
| 66 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNRARRE LPRFMNYTLN NAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide R133C, R135C, R136C |
| 67 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNRARRE LPRFMNYTLN NAKKTNVTLS KKRKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide R135C, R136C |
| 68 | QNITE EFYQSTCSAV SKGYLSALRT GWYTSVITIELSNIKENKCN GTDAKVKLIK QELDKYKNAV TELQLLMQST PATNNCACRE LPRFMNYTLN NAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide without signal peptide (SCB-N25C) (R106C, R108C, R133C, R135C, R136C) |
| 69 | QNITE EFYQSTCSAV SKGYLSALRT GWYTSVITIELSNIKENKCN GTDAKVKLIK QELDKYKNAV TELQLLMQST PATNNRACRE LPRFMNYTLNNAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide without signal peptide: R108C, R133C, R135C, R136C |
| 70 | QNITE EFYQSTCSAV SKGYLSALRT GWYTSVITIE LSNIKENKCN GTDAKVKLIK QELDKYKNAV TELQLLMQST PATNNRARRE LPRFMNYTLNNAKKTNVTLS KKCKCCFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDPLVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide without signal peptide: R133C, R135C, R136C |
| 71 | QNITE EFYQSTCSAV SKGYLSALRT GWYTSVITIE LSNIKENKCN GTDAKVKLIK QELDKYKNAV TELQLLMQST PATNNRARRE LPRFMNYTLNNAKKTNVTLS KKRKCCFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDPLVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide without signal peptide: R135C, R136C |
| 72 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNCACRE LPRFMNYTLN NAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL | hRSV-F antigen with signal peptide: R106C, R108C, R133C, R135C, R136C |
| 73 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNRACRE LPRFMNYTLNNAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL | hRSV-F antigen with signal peptide: R108C, R133C, R135C, R136C |
| 74 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNRARRE LPRFMNYTLN NAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDP LVFPSDEFDA SISQVNEKIN QSLAFIRKSD ELL | hRSV-F antigen with signal peptide: R133C, R135C, R136C |
| 75 | MELLILKANA ITTILTAVTF CFASGQNITE EFYQSTCSAV SKGYLSALRT GWYTSVITIELSNIKENKCN GTDAKVKLIK QELDKYKNAV TELQLLMQST PATNNRARRE LPRFMNYTLN NAKKTNVTLS KKRKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDP LVFPSDEFDA SISQVNEKIN QSLAFIRKSD ELL | hRSV-F antigen with signal peptide: R135C, R136C |
| 76 | QNITE EFYQSTCSAV SKGYLSALRT GWYTSVITIELSNIKENKCN GTDAKVKLIK QELDKYKNAV TELQLLMQST PATNNCACRE LPRFMNYTLN NAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL | hRSV-F antigen without signal peptide: R106C, R108C, R133C, R135C, R136C |
| 77 | QNITE EFYQSTCSAV SKGYLSALRT GWYTSVITIELSNIKENKCN GTDAKVKLIK QELDKYKNAV TELQLLMQST PATNNRACRE LPRFMNYTLNNAKKTNVTLS KKCKCCFLGF LLGVGSAIAS GVAVSKVLHL EGEVNKIKSA LLSTNKAVVSLSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYVVQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKTKCTASNKNRG IIKTFSNGCD YVSNKGVDTV SVGNTLYYVN KQEGKSLYVK GEPIINFYDPLVFPSDEFDA SISQVNEKIN QSLAFIRKSD ELL | hRSV-F antigen without signal peptide: R108C, R133C, R135C, R136C |
| 78 | QNITE EFYQSTCSAV SKGYLSALRT GWYTSVITIE LSNIKENKCN GTDAKVKLIK QELDKYKNAV TELQLLMQST PATNNRARRE LPRFMNYTLNNAKKTNVTLS KKCKCCFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDPLVFPSDEFDA SISQVNEKIN QSLAFIRKSD ELL | hRSV-F antigen without signal peptide: R133C, R135C, R136C |
| 79 | QNITE EFYQSTCSAV SKGYLSALRT GWYTSVITIE LSNIKENKCN GTDAKVKLIK QELDKYKNAV TELQLLMQST PATNNRARRE LPRFMNYTLNNAKKTNVTLS KKRKCCFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVNAGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKVQSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDPLVFPSDEFDA SISQVNEKIN QSLAFIRKSD ELL | hRSV-F antigen without signal peptide: R135C, R136C |
| 80 | MELLILKANA ITTILTAVTF CFASGQNITE EFYQSTCSAV SKGYLSALRT GWYTSVITIE LSNIKENKCN GTDAKVKLIK QELDKYKNAV TELQLLMQST PATNNAAARE LPRFMNYTLN NAKKTNVTLS KKAKAAFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide (SCB-N25A): R106A, R108A, R133A, R135A, R136A |
| 81 | MELLILKANA ITTILTAVTF CFASGQNITE EFYQSTCSAV SKGYLSALRT GWYTSVITIE LSNIKENKCN GTDAKVKLIK QELDKYKNAV TELQLLMQST PATNNTATRE LPRFMNYTLN NAKKTNVTLS KKTKTTFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide (SCB-N25T): R106T, R108T, R133T, R135T, R136T |
| 82 | MELLILKANA ITTILTAVTF CFASGQNITE EFYQSTCSAV SKGYLSALRT GWYTSVITIE LSNIKENKCN GTDAKVKLIK QELDKYKNAV TELQLLMQST PATNNYAYRE LPRFMNYTLN NAKKTNVTLS KKYKYYFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide (SCB-N25Y): R106Y, R108Y, R133Y, R135Y, R136Y |
| 83 | MELLILKANA ITTILTAVTF CFASGQNITE EFYQSTCSAV SKGYLSALRT GWYTSVITIE LSNIKENKCN GTDAKVKLIK QELDKYKNAV TELQLLMQST PATNNGAGRE LPRFMNYTLN NAKKTNVTLS KKGKGGFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | hRSV-F fusion polypeptide with signal peptide (SCB-N25G): R106G, R108G, R133G, R135G, R136G |
| 84 | MELLILKANA ITTILTAVTF CFASGQNITE EFYQSTCSAV | hRSV-F fusion polypeptide |
| | SKGYLSALRT GWYTSVITIE LSNIKENKCN GTDAKVKLIK QELDKYKNAV TELQLLMQST PATNNSASRE LPRFMNYTLN NAKKTNVTLS KKSKSSFLGF LLGVGSAIAS GVAVSKVLHL EGEVNKIKSA LLSTNKAVVS LSNGVSVLTS KVLDLKNYID KQLLPIVNKQ SCSISNIETV IEFQQKNNRL LEITREFSVN AGVTTPVSTY MLTNSELLSL INDMPITNDQ KKLMSNNVQI VRQQSYSIMS IIKEEVLAYV VQLPLYGVID TPCWKLHTSP LCTTNTKEGS NICLTRTDRG WYCDNAGSVS FFPQAETCKV QSNRVFCDTM NSLTLPSEVN LCNVDIFNPK YDCKIMTSKT DVSSSVITSL GAIVSCYGKT KCTASNKNRG IIKTFSNGCD YVSNKGVDTV SVGNTLYYVN KQEGKSLYVK GEPIINFYDP LVFPSDEFDA SISQVNEKIN QSLAFIRKSD ELL RSNGLPGPIGPPGPRGRTGDAGPVGPPGPPGPPGPPGPPSAGFDFSF LPQPPQEKAHDGGRYYRANDANVVRDRDLEVDTTLKSLSQQIENI RSPEGSRKNPARTCRDLKMCHSDWKSGEYWIDPNQGCNLDAIKV FCNMETGETCVYPTQPSVAQKNWYISKNPKDKRHVWFGESMTD GFQFEYGGQGSDPADVAIQLTFLRLMSTEASQNITYHCKNSVAY MDQQTGNLKKALLLQGSNEIEIRAEGNSRFTYSVTVDGCTSHTGA WGKTVIEYKTTKTSRLPIIDVAPLDVGAPDQEFGFDVGPVCFL | with signal peptide (SCB-N25S): R106S, R108S, R133S, R135S, R136S |

## Claims

1. Use of a recombinant subunit vaccine in the preparation of a medicament for preventing respiratory syncytial virus (RSV) infection in a mammal, wherein the recombinant subunit vaccine comprises a soluble RSV surface antigen, the soluble RSV surface antigen comprises an F1 peptide, or a fragment or epitope thereof, having one or more mutation sites, wherein the one or more mutation sites of the F1 peptide or fragment or epitope thereof is or are a sulfur bond-containing amino acid or a sulfur bond-containing amino acid derivative, and the soluble RSV surface antigen is linked to collagen via in-frame fusion to form a disulfide bond-linked trimeric fusion protein.

2. The use according to claim 1, wherein the RSV is of subtype A or subtype B.

3. The use according to claim 1 or 2, wherein the RSV surface antigen comprises an F2 peptide or a fragment or epitope thereof.

4. The use according to any one of claims 1 to 3, wherein the RSV surface antigen comprises a mutant F2 peptide or a fragment or epitope thereof.

5. The use according to any one of claims 1 to 4, wherein the one or more mutation sites of the F1 peptide or fragment or epitope thereof is or are R106C and/or R108C.

6. The use according to any one of claims 1 to 5, wherein the RSV surface antigen comprises a pep27 peptide or a fragment or epitope thereof.

7. The use according to claim 6, wherein the pep27 peptide or a fragment or epitope thereof comprises one or more mutation sites.

8. The use according to claim 6 or 7, wherein the one or more mutation sites contained in the pep27 peptide or fragment or epitope thereof is or are a sulfur bond-containing amino acid.

9. The use according to any one of claims 6-8, wherein the one or more mutation sites of the pep27 peptide or fragment or epitope thereof is or are independently selected from R133C, R135C, R136C, or any combination thereof.

10. The use according to any one of claims 1 to 9, wherein the fusion protein comprises a sequence selected from the group consisting of SEQ ID NOs: 64-79.

11. The use according to any one of claims 1 to 10, wherein the fusion protein comprises a sequence selected from the group consisting of SEQ ID NOs: 76-79, optionally SEQ ID NOs: 64, 68, 72 or 76.

12. The use according to any one of claims 1 to 11, wherein the recombinant subunit vaccine is administered by intramuscular injection.

13. The use according to any one of claims 1 to 11, wherein the recombinant subunit vaccine is administered by intranasal spray.

14. The use according to any one of claims 1 to 12, wherein the recombinant subunit vaccine is administered as a single dose or as a series of doses spaced at intervals of weeks or months.

15. The use according to any one of claims 1 to 13, wherein the recombinant subunit vaccine is administered without an adjuvant.

16. The use according to any one of claims 1 to 13, wherein the recombinant subunit vaccine is administered with one or more adjuvants.

17. The use according to any one of claims 1 to 16, wherein the recombinant subunit vaccine comprises a pharmaceutically acceptable excipient, and the excipient is one or more selected from a buffer, an osmotic regulator, a stabilizer, and a bacteriostatic agent.

18. Use of a soluble respiratory syncytial virus (RSV) surface antigen in the preparation of a reagent for use in a method of detecting an antibody against RSV from mammalian serum, wherein the method comprises a step of contacting the serum with a soluble RSV surface antigen, the soluble RSV surface antigen comprises an F1 peptide, or a fragment or epitope thereof, having one or more mutation sites, and the one or more mutation sites is or are a sulfur bond-containing amino acid, and the soluble RSV surface antigen is linked to collagen via in-frame fusion to form a disulfide bond-linked trimeric fusion protein.

19. Use of a recombinant subunit vaccine comprising a soluble surface antigen from a respiratory syncytial virus (RSV) in the preparation of a neutralizing antibody for treating a patient infected with said RSV virus by passive immunotherapy, wherein the preparation comprises immunizing a mammal, and purifying the resulting neutralizing antibody, the soluble RSV surface antigen comprises an F1 peptide, or a fragment or epitope thereof, having one or more mutation sites, and the one or more mutation sites is or are a sulfur bond-containing amino acid, and the soluble surface antigen is linked to collagen via in-frame fusion to form a disulfide bond-linked trimeric fusion protein.

20. The use according to claim 19, wherein the neutralizing antibody comprises a polyclonal antibody and/or a monoclonal antibody, wherein the neutralizing antibody is a monoclonal antibody against F protein or peptide.

21. One or more polynucleotides encoding a soluble RSV surface antigen, wherein the soluble RSV surface antigen comprises a sequence selected from the group consisting of SEQ ID NOs: 64-79.

22. One or more vectors comprising one or more polynucleotides according to claim 21.

23. A host cell comprising one or more polynucleotides of claim 21, or one or more vectors of claim 22.

24. The host cell according to claim 23, wherein the host cell is GH-CHO.

25. The vector according to claim 23, wherein the vector is pTPRIMER-TOD.
